(19) Europäisches Patentamt
European Patent Office
Office européen des brevets

(11) EP 2 825 661 B1

(12) EUROPÄISCHE PATENTSCHRIFT

(45) Veröffentlichungstag und Bekanntmachung des Hinweises auf die Patenterteilung:
**18.05.2016 Patentblatt 2016/20**

(21) Anmeldenummer: **13712707.2**

(22) Anmeldetag: **11.03.2013**

(51) Int Cl.:
***C12P 13/00*** $^{(2006.01)}$

(86) Internationale Anmeldenummer:
**PCT/EP2013/054817**

(87) Internationale Veröffentlichungsnummer:
**WO 2013/135601 (19.09.2013 Gazette 2013/38)**

# (54) VERFAHREN ZUR HERSTELLUNG VON TERPEN-NITRILEN AUS TERPEN-OXIMEN UNTER VERWENDUNG EINER ALDOXIM DEHYDRATASE

PROCESS FOR PREPARING TERPENE NITRILES FROM TERPENE OXIMES EMPLOYING AN ALDOXIME DEHYDRATASE

PROCÉDÉ DE PRODUCTION DE NITRILES TERPÉNIQUES À PARTIR D'OXIMES TERPÉNIQUES EN UTILISANT UNE ALDOXIME DESHYDRATASE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.03.2012 EP 12159265**

(43) Veröffentlichungstag der Anmeldung:
**21.01.2015 Patentblatt 2015/04**

(73) Patentinhaber: **BASF SE**
**67056 Ludwigshafen am Rhein (DE)**

(72) Erfinder:
- **PIATESI, Andrea**
  **68239 Mannheim (DE)**
- **SIEGEL, Wolfgang**
  **67117 Limburgerhof (DE)**
- **BALDENIUS, Kai-Uwe**
  **69115 Heidelberg (DE)**

(74) Vertreter: **BASF IP Association**
**BASF SE**
**ZRX-C6**
**67056 Ludwigshafen (DE)**

(56) Entgegenhaltungen:
**WO-A1-2010/044031**

- **KATO, Y. ET AL.: "Novel Heme-Containing Lyase, Phenylacetaldoxime Dehydratase from Bacillus sp. Strain OxB-1: Purification, Characterization, and Molecular Cloning of the Gene", BIOCHEMISTRY, Bd. 39, Nr. 4, 4. Januar 2000 (2000-01-04) , Seiten 800-809, XP002687062, in der Anmeldung erwähnt**
- **KATO, Y. & ASANO, Y.: "Purification and Characterization of Aldoxime Dehydratase of the Head Blight Fungus, Fusarium graminearum", BIOSCIENCE, BIOTECHNOLOGY, AND BIOCHEMISTRY, Bd. 69, Nr. 11, November 2005 (2005-11), Seiten 2254-2257, XP002687063,**
- **XIE, S.X. ET AL.: "A Gene Cluster Responsible for Alkylaldoxime Metabolism Coexisting with Nitrile Hydratase and Amidase in Rhodococcus globerulus A-4", BIOCHEMISTRY, Bd. 42, Nr. 41, 26. September 2003 (2003-09-26), Seiten 12056-12066, XP002687064,**
- **PEDRAS M.S.C. ET AL.: "Indolyl-3-acetaldoxime dehydratase from the phytopathogenic fungus Sclerotinia sclerotiorum: Purification, characterization, and substrate specificity", PHYTOCHEMISTRY, Bd. 71, Nr. 17-18, 29. Oktober 2010 (2010-10-29), Seiten 1952-1962, XP027491109,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

SEQUENCE LISTING

<110> BASF SE

<120> Verfahren zur biokatalytischen Herstellung von Nitrilen aus Oximen und darin einsetzbare Oxim-Dehydratasen

<130> M/52415

<160> 28

<170> PatentIn version 3.3

<210> 1
<211> 351
<212> PRT
<213> Bacillus sp. OxB-1

<400> 1

```
Met Lys Asn Met Pro Glu Asn His Asn Pro Gln Ala Asn Ala Trp Thr
1               5                   10                  15

Ala Glu Phe Pro Pro Glu Met Ser Tyr Val Val Phe Ala Gln Ile Gly
            20                  25                  30

Ile Gln Ser Lys Ser Leu Asp His Ala Ala Glu His Leu Gly Met Met
        35                  40                  45

Lys Lys Ser Phe Asp Leu Arg Thr Gly Pro Lys His Val Asp Arg Ala
    50                  55                  60

Leu His Gln Gly Ala Asp Gly Tyr Gln Asp Ser Ile Phe Leu Ala Tyr
65                  70                  75                  80

Trp Asp Glu Pro Glu Thr Phe Lys Ser Trp Val Ala Asp Pro Glu Val
                85                  90                  95

Gln Lys Trp Trp Ser Gly Lys Lys Ile Asp Glu Asn Ser Pro Ile Gly
            100                 105                 110

Tyr Trp Ser Glu Val Thr Thr Ile Pro Ile Asp His Phe Glu Thr Leu
        115                 120                 125

His Ser Gly Glu Asn Tyr Asp Asn Gly Val Ser His Phe Val Pro Ile
    130                 135                 140

Lys His Thr Glu Val His Glu Tyr Trp Gly Ala Met Arg Asp Arg Met
145                 150                 155                 160

Pro Val Ser Ala Ser Ser Asp Leu Glu Ser Pro Leu Gly Leu Gln Leu
                165                 170                 175
```

```
Pro Glu Pro Ile Val Arg Glu Ser Phe Gly Lys Arg Leu Lys Val Thr
            180                 185                 190

Ala Pro Asp Asn Ile Cys Leu Ile Arg Thr Ala Gln Asn Trp Ser Lys
        195                 200                 205

Cys Gly Ser Gly Glu Arg Glu Thr Tyr Ile Gly Leu Val Glu Pro Thr
    210                 215                 220

Leu Ile Lys Ala Asn Thr Phe Leu Arg Glu Asn Ala Ser Glu Thr Gly
225                 230                 235                 240

Cys Ile Ser Ser Lys Leu Val Tyr Glu Gln Thr His Asp Gly Glu Ile
                245                 250                 255

Val Asp Lys Ser Cys Val Ile Gly Tyr Tyr Leu Ser Met Gly His Leu
            260                 265                 270

Glu Arg Trp Thr His Asp His Pro Thr His Lys Ala Ile Tyr Gly Thr
        275                 280                 285

Phe Tyr Glu Met Leu Lys Arg His Asp Phe Lys Thr Glu Leu Ala Leu
    290                 295                 300

Trp His Glu Val Ser Val Leu Gln Ser Lys Asp Ile Glu Leu Ile Tyr
305                 310                 315                 320

Val Asn Cys His Pro Ser Thr Gly Phe Leu Pro Phe Phe Glu Val Thr
                325                 330                 335

Glu Ile Gln Glu Pro Leu Leu Lys Ser Pro Ser Val Arg Ile Gln
            340                 345                 350
```

<210> 2
<211> 1056
<212> DNA
<213> Bacillus sp. OxB-1

<400> 2

```
ttgaaaaata tgccggaaaa tcacaatcca caagcgaatg cctggactgc cgaatttcct      60
cctgaaatga gctatgtagt atttgcgcag attgggattc aaagcaagtc tttggatcac     120
gcagcggaac atttgggaat gatgaaaaag agtttcgatt tgcggacagg ccccaaacat     180
gtggatcgag ccttgcatca aggagccgat ggataccaag attccatctt tttagcctac     240
tgggatgagc ctgaaacatt taaatcatgg gttgcggatc ctgaagtaca aaagtggtgg     300
tcgggtaaaa aaatcgatga aaatagtcca atcgggtatt ggagtgaggt aacgaccatt     360
ccgattgatc actttgagac tcttcattcc ggagaaaatt acgataatgg ggtttcacac     420
tttgtaccga tcaagcatac agaagtccat gaatattggg gagcaatgcg cgaccgcatg     480
ccggtgtctg ccagtagtga tttggaaagc cccccttggcc ttcaattacc ggaacccatt   540
gtccgggagt ctttcggaaa acggctaaaa gtcacggcgc cggataatat ttgcttgatt     600
cgaaccgctc aaaattggtc taaatgtggt agcggggaaa gggaaacgta tataggacta     660
gtggaaccga ccctcataaa agcgaatacg tttcttcgtg aaaatgctag tgaaacaggc     720
tgtattagtt caaaattagt ctatgaacag acccatgacg gcgaaatagt agataaatca     780
tgtgtcatcg gatattatct ctccatgggg catcttgaac gctggacgca tgatcatcca     840
acacataaag cgatctacgg aacctttat gagatgttga aaaggcatga ttttaagacc     900
gaacttgctt tatggcacga ggtttcggtg cttcaatcca aagatatcga gcttatctat     960
gtcaactgcc atccgagtac tggatttctt ccattctttg aagtgacaga aattcaagag    1020
cctttactga aaagccctag cgtcaggatc cagtga                              1056
```

<210> 3
<211> 1056
<212> DNA
<213> artificial

<220>
<223> DNA sequence coding for seq ID NO:1, codon-optimized for *E.coli*

<400> 3

atgaaaaaca tgccagaaaa ccacaacccg caggctaacg cctggactgc agaattcccg 60

ccagagatgt cttacgttgt cttcgcgcag attggcatcc agtctaaaag cctggatcat 120

gcagccgaac atctgggcat gatgaaaaaa tctttcgacc tccgtactgg tccaaaacac 180

gttgatcgcg cactgcacca gggtgctgat ggctatcagg actctatctt cctggcctac 240

tgggacgaac cagaaacctt caaaagctgg gttgcagacc cagaagtgca gaaatggtgg 300

agcggtaaaa aaatcgatga aaacagcccg atcggctatt ggtctgaggt taccactatc 360

ccgattgacc acttcgagac cctgcactct ggtgagaact atgacaacgg cgtttcccac 420

ttcgtgccga tcaaacatac cgaagtgcac gaatactggg gtgctatgcg tgatcgtatg 480

ccggtgtctg cttcttccga tctggaaagc ccgctgggtc tccagctgcc agaaccgatc 540

gtacgtgaga gctttggtaa acgcctgaaa gttaccgctc cagacaacat ctgcctgatt 600

cgtaccgcac agaactggtc caaatgtggt tctggtgaac gcgaaaccta cattggcctg 660

gtcgaaccga ctctgatcaa agcgaacacc ttcctgcgtg aaaacgcgtc tgagaccggt 720

tgcattagct ctaaactggt ttacgagcag acccatgatg gcgaaatcgt agataaatcc 780

tgtgtaatcg gctactatct gtccatgggt catctggaac gctggactca cgaccaccca 840

actcacaaag cgatttacgg cacgttctac gaaatgctga aacgtcacga ctttaaaacg 900

gaactggcgc tgtggcacga agtaagcgtt ctgcagtcta aagacatcga gctgatctat 960

gtcaactgcc acccatccac gggttttctg ccgttctttg aagtgaccga aatccaggaa 1020

ccactgctga aatccccatc cgtgcgtatt cagtga 1056

<210> 4
<211> 366
<212> PRT
<213> Arthrobacter aurescens

<400> 4

Met Ala Pro Asp Pro Ser Arg Gln Asp Pro Ala Ile Glu Ser Ser Ile
1               5                   10                  15

Pro Arg His Phe Thr Val Lys Arg Thr Arg Pro Lys Arg Ala Gly Ala
            20                  25                  30

Gly Tyr Ala Pro Pro Tyr Pro Ser Tyr Ser Val Arg Phe Ala Glu Gly
        35                  40                  45

Val Ser Asn Leu Val Cys Ala Phe Leu Gly Val Gln Ser Arg Ala Pro
    50                  55                  60

Leu Ser His Glu Ala Lys Val Ala Ser Ala Gly Met Trp Glu Leu Cys
65                  70                  75                  80

Ala Asn Glu Asp Gly Pro Met Ser Arg Glu His Ala Val His Thr Asp
                85                  90                  95

Glu Gln Gly Phe Asp Asn Gln Val Ile Ile Ala Tyr Trp Asp Asp Val
            100                 105                 110

Asp Ala Tyr Gly Arg Trp Phe Lys Lys His Arg Asp Ala Leu Ile Gly
        115                 120                 125

Ala Gly Leu Glu Pro Ser Asp Tyr Gly Arg Trp Ile Glu Ala Val Thr
    130                 135                 140

Pro Glu Ala Arg Gly Phe Glu Thr Leu Tyr Ser Ser Asn Thr Phe Pro
145                 150                 155                 160

Glu Gly Ala Ala Arg Met Ala Thr Gly Gly Phe Thr Gly Glu Ile Gln
                165                 170                 175

Glu His Gly Tyr Trp Gly Ser Met Arg Asp Arg Leu Pro Ile Ala Gln
            180                 185                 190

Thr Asp Ala Leu Glu Pro Val Gly Asp Pro Val Val Pro Arg Asn Pro
195 200 205

Gly Ile Val Arg Val Glu Pro His Asp Asn Leu Ala Val Ile Arg Ser
210 215 220

Gly Gln Asp Trp Ser Leu Cys Asp Asp Ala Glu Arg Ala Ser Tyr Phe
225 230 235 240

Ser His Val Glu Pro Gln Leu Lys Ala Gly Met Asp Phe Leu Thr Thr
245 250 255

Glu Gly Ala Ser Ile Gly Cys Tyr Ala Asn Arg Tyr Met Thr Ser Ser
260 265 270

Asp Gly Asn Gly Asn Val Leu Glu Gln Ser Phe Gly Leu Ser Phe Trp
275 280 285

His Ser Leu Glu Asp Met Glu Arg Trp Ala Glu Ser His Pro Thr His
290 295 300

Val Ala Ile Phe Arg Ser Ala Met Thr Phe Leu Gln Ala Asn Ala Gly
305 310 315 320

Ala Arg Leu Arg Leu Ser His Glu Val Ala Val Val Ser Arg Asp Gln
325 330 335

Gln Tyr Tyr Glu Tyr Asn Asn Cys His Ala Gly Thr Gly Met Leu Gly
340 345 350

Ala Arg Arg Pro Leu Gly Thr Ala Thr Pro Gly Thr Arg Ile
355 360 365

<210> 5
<211> 352
<212> PRT
<213> Acinetobacter sp. Stamm ADP1

<400> 5

Met Glu Ser Ala Ile Asp Lys His Leu Lys Cys Pro Arg Thr Leu Ser
1 5 10 15

Arg Arg Ile His Asp Glu Tyr Glu Pro Pro Phe Ala Met Trp Val Ala
20 25 30

Arg Ala Asp Glu Ser Leu Gln Gln Val Val Met Ala Tyr Phe Gly Val
35 40 45

```
Gln Phe Lys Ser Glu Gln Lys Ala Ile Ala Leu Lys Ala Met Gln His
    50                  55                  60

Ile Val Gln Ser Phe Ser Leu Asp Asn Gly Pro Gln Asn His Asp Ile
65                  70                  75                  80

Thr His His Thr Asp Asn Gln Gly Tyr Glu Asn Tyr Ile Val Val Gly
                85                  90                  95

Tyr Trp Arg Asp Pro Gly Ala Tyr Cys Arg Trp Phe Arg Ser Thr Glu
            100                 105                 110

Val Ser His Trp Trp Asp Ser Asp Glu Arg Leu Asn Asp Gly Ile Gly
        115                 120                 125

Tyr Phe Arg Glu Ile Val Ile Pro Arg Ala Asp Gln Phe Glu Thr Leu
    130                 135                 140

Tyr Ala Phe Lys Glu Asp Leu Pro Gly Val Gly Ala Val Met Asp Asp
145                 150                 155                 160

Ile Ser Asp Asp Ile Gln Glu His Gly Tyr Trp Gly Ser Ala Arg Glu
                165                 170                 175

Arg Phe Pro Ile Ser Gln Thr Asp Arg Met Leu Ala Asn Gly Glu Leu
            180                 185                 190

His Ile Ile Ser Gly Asp Pro Glu Lys Gly Gly Arg Val Leu Val Gln
        195                 200                 205

Gly His Asp Asn Ile Thr Leu Ile Arg Ser Gly Gln Asp Trp Val Asn
    210                 215                 220

Ala Asp Glu Lys Glu Arg Glu Leu Tyr Phe Asn Glu Met Leu Pro Ser
225                 230                 235                 240

Leu Gln Ala Gly Met Asp Phe Leu Arg Asp Glu Gly Gln Ala Leu Gly
                245                 250                 255

Cys Tyr Ser Asn Arg Phe Val Arg Asn Val Asp Ile Asp Gly Asn Leu
            260                 265                 270

Leu Asp Ile Ala Tyr Asp Ile Gly Tyr Ile Ala Pro Trp Arg Ser Leu
        275                 280                 285

Asp Lys Leu Glu Arg Trp Ala Glu Ser His Pro Thr His Leu Arg Ile
    290                 295                 300
```

Phe Thr Thr Phe Phe Lys Val Val Thr Gly Leu Gln Asn Leu Arg Leu
305 310 315 320

Tyr His Glu Val Ser Val Ser Asp Ala Lys Asn Gln Val Phe Glu Tyr
325 330 335

Ile Asn Cys His Pro Gln Thr Gly Met Met Arg Asp Ala Gln Met Thr
340 345 350

<210> 6
<211> 353
<212> PRT
<213> Acidovorax avanae

<400> 6

Met Glu Ser Ala Ile Pro Pro Arg Leu Gln Cys Pro Arg Ser Leu Ser
1 5 10 15

Arg Arg Val Ala Asp Asp Tyr Gln Pro Pro Phe Pro Met Trp Val Ala
20 25 30

Arg Pro Gln Ala Asp Leu Gln Gln Val Val Met Ala Tyr Phe Gly Ile
35 40 45

Gln Tyr Gln Gly Glu Ala Gln Lys Pro Arg Ala Leu Ala Val Leu Arg
50 55 60

Glu Trp Val Ala Ala Phe Gly Ala Pro Asp Gly Pro Leu Arg His Asp
65 70 75 80

Leu Thr His His Ile Asp Ala Gln Gly Tyr Asp Asn Leu Ile Ala Val
85 90 95

Gly Tyr Trp Arg Asp Pro Glu Ala His Arg Arg Trp Met Gln Ala Pro
100 105 110

Ala Gln Ala Gly Trp Trp Asn Ala Pro Glu Arg Leu Gln Glu Gly Leu
115 120 125

Gly Tyr Phe Arg Glu Val Ser Ala Pro Arg Ala Glu Gln Phe Glu Thr
130 135 140

Leu Tyr Ala Phe Gln Asp Ala Leu Pro Gly Val Gly Gly Val Met Glu
145 150 155 160

Ser Thr Ser Gly Asp Ile Gln Glu His Gly Tyr Trp Gly Ser Met Arg
165 170 175

```
Asp Arg Phe Pro Ala Ser Gln Val Asp Arg Met Gln Ala Arg Gly Thr
            180                 185                 190

Leu Leu Ile Ala Glu Gly Asp Pro Ala His Gly Gly Arg Val Val Val
        195                 200                 205

Arg Gly His Asp Asn Ile Ala Leu Ile Arg Ser Gly Gln Asp Trp Met
    210                 215                 220

Glu Ala Glu Ala Glu Glu Arg Arg Leu Tyr Leu Glu Asp Ile Glu Pro
225                 230                 235                 240

Thr Leu His Ala Gly Met Asp Phe Leu Arg Asp Gln Gly Ala Ala Val
                245                 250                 255

Gly Cys Tyr Ser Asn Arg Tyr Val His Asn Ile Asp Leu Asp Gly Arg
            260                 265                 270

Arg Leu Asp Gln Ser Tyr Asn Ile Gly His Trp Arg Ser Val Asp Leu
        275                 280                 285

Leu Glu Arg Trp Ala Glu Ser His Pro Thr His Leu Arg Ile Phe Gly
    290                 295                 300

Thr Phe Phe Lys Val Ala Ala Gly Leu Ser Lys Leu Arg Leu Tyr His
305                 310                 315                 320

Glu Val Ser Val Ser Asp Ala Ala Ser Gln His Phe Glu Tyr Ile Gln
                325                 330                 335

Cys His Pro Ala Thr Gly Met Met Arg Asp Ala Arg Leu Gln Ala Pro
            340                 345                 350

Ala
```

<210> 7
<211> 343
<212> PRT
<213> Actinosynnema mirum

<400> 7

```
Met Ser Ser Leu Pro Leu Asp Asp Arg Ala Thr Ala His Arg Pro Glu
1               5                   10                  15

Gly Tyr Asp Pro Arg Gly Gly Pro Gly His Glu Val Arg Trp Ser Asp
            20                  25                  30
```

```
Asp Val Thr His Leu Val Val Ala Arg Phe Gly Val Gln Thr Asp Asp
        35                  40                  45

Ser Ala Ala Gly Val Lys Ala Ile Ala Arg Val Leu Glu Leu Ala Ala
    50                  55                  60

Gly Glu Ser Gly Pro Ala Leu Val Glu Arg Val Ser Asp Asp Asp Ser
65                  70                  75                  80

Glu Met Ala Val Cys Tyr Trp Pro Asp Pro Glu Ala His Arg Ala Trp
                85                  90                  95

Trp Ala Ser Gly Pro Val Arg Asp Trp Trp Ala Ser Leu Pro Val Asp
            100                 105                 110

Gly Pro Ile Gly His Trp His Glu Thr Ser Val Thr Pro Val Glu Gln
        115                 120                 125

Phe Glu Thr Leu Tyr Ser Ala Glu Phe Ala Ala Gly Pro Ser Arg Phe
    130                 135                 140

Ala Gly Thr Gly Pro Thr Asn Leu His Asp Tyr Asp Asn Ser Thr Leu
145                 150                 155                 160

Asp Arg Met Pro Ala Thr Ala His Arg Asp Leu Arg Gln Glu Arg Ala
                165                 170                 175

Glu Glu Pro Thr Thr Asp Leu Pro Pro Gly Glu Ser Pro Arg Gly Arg
            180                 185                 190

Arg Val Arg Leu Ala Glu Pro Ser Pro Ser Gly Leu Cys Trp Ile Arg
        195                 200                 205

Thr Ala Gln Glu Trp Ser Ile Ala Pro Asp Asp Gln Leu Ala Ser Tyr
    210                 215                 220

Arg Asp Gly Val Glu Pro Ala Tyr Arg Thr Ala Ile Ala His Leu Gln
225                 230                 235                 240

Asp Asn Pro His Asp Thr Gly Cys Leu Ser Ala Arg Leu Val Gly Asn
                245                 250                 255

Leu Asp Ala Asn Gly Ala Arg Ala Ala Gly Ala Glu Ala Val Val Trp
            260                 265                 270

Trp Arg Gly Ile Gly Asp Leu Leu Arg Trp Ala His Asp His Lys Thr
        275                 280                 285
```

His Gln Asp Ile Leu Asn Gly Phe Trp Glu His Val Ile Ala Lys Phe
290 295 300

Gly Pro Gly Thr Arg Val Arg Leu Trp His Glu Val His Val Leu Pro
305 310 315 320

Glu Gly Ala Leu Thr Ala Glu Tyr Val Asn Cys His Pro Gly Thr Gly
325 330 335

Leu Leu Gln Thr Trp Pro Gly
340

<210> 8
<211> 340
<212> PRT
<213> Bradyrhizobium sp. BTAi1

<400> 8

Met Glu Ser Ala Ile Pro Pro His Leu Ile Thr Thr Arg Cys Arg His
1 5 10 15

Arg Arg Val Asp Asp Asp Tyr Lys Pro Pro Tyr Pro Ser Phe Val Ala
20 25 30

Arg His Gly Ala Asp Val Ser Arg Val Val Met Ala Tyr Phe Gly Val
35 40 45

Gln Tyr Arg Ala Glu Thr Pro Ala Ala Ala Ser Thr Ala Asp Phe Met
50 55 60

Val Leu Val Ser Arg Ala Asp Gly Pro Ser His Trp Asp Leu Ala His
65 70 75 80

Tyr Val Asp Gln Ala Gly Phe Ala Asn Asp Val Phe Val Ala Tyr Trp
85 90 95

Asp Asp Val Val Arg Phe Asp Ser Trp Phe Glu Pro Ala Arg Ala Ala
100 105 110

Trp Thr Gly Pro Gly Ala Glu Gly Gly Gly Arg Phe Ile Glu Val Leu
115 120 125

Arg Pro Ala Val Glu Arg Tyr Glu Thr Leu Phe Ser Ser Leu Gly Arg
130 135 140

Pro Glu Gly Ile Ala Val Ile Ala Glu Gly Met Ser Gly Glu Val Leu
145 150 155 160

```
Glu His Ala Tyr Trp Gly Gly Met Arg Asp Arg Ile Pro Leu Ser Gln
                165                 170                 175

Thr Ser Glu Met Arg Ser Leu Gly Lys Pro Thr Leu Val Gln Asp Gly
            180                 185                 190

Pro Arg Leu Arg Val Ile Ala Gln Asp Asn Leu Cys Met Ile Arg Ser
        195                 200                 205

Gly Gln Asp Trp Ser Asp Thr Asp Ala Ala Glu Arg Arg Met Tyr Leu
    210                 215                 220

Asp Asp Val Glu Pro Val Leu Arg Glu Gly Met Asp Phe Leu Arg Asp
225                 230                 235                 240

Gln Gly Leu Ser Ile Gly Cys Tyr Ala Asn Arg Tyr Met Arg Leu Arg
                245                 250                 255

Gly Ala Asp Gly Ala Leu Thr Glu Lys Ser Tyr Gly Gln Ser Trp Trp
            260                 265                 270

Gln Ser Leu Ser Ala Leu Glu Arg Trp Ala Glu Ser His Pro Thr His
        275                 280                 285

Val Arg Ile Phe Gly Ala Ala Met Lys Tyr Leu Ser Ser Leu Gly Pro
    290                 295                 300

Ala Ala Arg Leu Arg Leu Tyr His Glu Val Thr Val Ala Ala Ala Asp
305                 310                 315                 320

Glu Gln Phe Phe Glu Tyr Arg Gly Cys His Ala Lys Thr Gly Met Leu
                325                 330                 335

Ala Ala Ala Gly
            340
```

<210> 9
<211> 350
<212> PRT
<213> Burkholderia ambifaria IOP40-10

<400> 9

```
Met Glu Ser Ala Ile Asp Lys His Leu Val Cys Pro Arg Thr Leu Ser
1               5                   10                  15

Arg Arg Val Ala Asp Asp Tyr Gln Pro Pro Phe Pro Met Tyr Val Ala
            20                  25                  30
```

```
Arg Ala Ser Glu Asp Leu Ser Gln Val Val Met Gly Tyr Phe Gly Val
        35                  40                  45

Gln Tyr Arg Gly Ala Asp Gln Arg Ser Ala Ala Leu Ala Ala Leu Arg
    50                  55                  60

Arg Ile Val Ala Asp Phe Asp Ala Pro Asp Gly Pro Gly Asn His Asp
65                  70                  75                  80

Leu Thr Gln His Thr Asp Asn Gln Gly Tyr Asp Asn Leu Ile Ala Val
                85                  90                  95

Gly Tyr Trp Arg Asp Pro Asp Ala Tyr Ala Arg Trp Ile Ala Ser Pro
            100                 105                 110

Ala Val Ala Glu Trp Trp Thr Ser Asp Ala Arg Leu Ala Asp Gly Ile
        115                 120                 125

Gly Tyr Phe Arg Glu Ile Val Ala Pro Arg Ala Glu Gln Phe Glu Thr
    130                 135                 140

Leu Tyr Ala Phe Thr Ala Asp Phe Pro Gly Val Gly Ala Ile Met Asp
145                 150                 155                 160

Gly Val Ser Gly Glu Ile Glu Glu His Gly Tyr Trp Gly Ser Met Arg
                165                 170                 175

Asp Arg Phe Pro Ile Ser Gln Thr Asp Trp Met His Ala Asp Gly Glu
            180                 185                 190

Leu Arg Ile Val Ala Gly Asp Pro Ala Arg Gly Gly Arg Val Val Val
        195                 200                 205

Leu Ala His Asp Asn Ile Ala Leu Ile Arg Ser Gly Gln Asp Trp Arg
    210                 215                 220

Ala Ala Gln Asp Asp Glu Arg Arg Leu Tyr Leu Asp Glu Ile Glu Pro
225                 230                 235                 240

Thr Leu Arg Ser Gly Met Glu Phe Leu Arg Asp Asn Gly Val Asp Val
                245                 250                 255

Gly Cys Tyr Ser Asn Arg Tyr Val Arg Ser Ile Asp Leu Asp Gly Asn
            260                 265                 270

Leu Leu Asp Glu Ser Tyr Asn Ile Gly His Trp Arg Ser Leu Asp Arg
```

```
            275                 280                 285

Leu Glu Arg Trp Ala Glu Ser His Pro Thr His Leu Arg Ile Phe Val
    290                 295                 300

Thr Phe Phe Arg Val Val Thr Gly Leu Ser Lys Leu Arg Leu Tyr His
305                 310                 315                 320

Glu Val Ser Val Phe Asp Ala Lys His Gln Val Tyr Glu Tyr Val Asn
                325                 330                 335

Cys His Pro Asn Thr Gly Met Met Arg Asp Ala Val Ala Arg
            340                 345                 350
```

<210> 10
<211> 350
<212> PRT
<213> Burkholderia ambifaria MC40-6

<400> 10

```
Met Glu Ser Ala Ile Asp Lys His Leu Val Cys Pro Arg Thr Leu Ser
1               5                   10                  15

Arg Arg Val Ala Asp Asp Tyr Gln Pro Pro Phe Pro Met Tyr Val Ala
            20                  25                  30

Arg Ala Ala Glu Asp Leu Ser Gln Val Val Met Gly Tyr Phe Gly Val
        35                  40                  45

Gln Tyr Arg Gly Ala Asp Lys Arg Ser Val Ala Leu Ala Ala Leu Arg
    50                  55                  60

Arg Ile Val Ala Asp Phe Asp Ala Pro Asp Gly Pro Gly Asn His Asp
65                  70                  75                  80

Leu Thr Gln His Thr Asp Asn Gln Gly Tyr Asp Asn Leu Ile Ala Val
                85                  90                  95

Gly Tyr Trp Arg Asp Pro Asp Ala Tyr Ala Arg Trp Ile Ala Ser Pro
            100                 105                 110

Ala Val Ala Glu Trp Trp Ala Ser Asp Ala Arg Leu Ala Asp Gly Ile
        115                 120                 125

Gly Tyr Phe Arg Glu Ile Val Ala Pro Arg Ala Glu Gln Phe Glu Thr
    130                 135                 140

Leu Tyr Ala Phe Thr Asn Asp Phe Pro Gly Val Gly Ser Ile Met Asp
```

```
145                 150                 155                 160

Gly Val Ser Gly Glu Ile Glu Glu His Gly Tyr Trp Gly Ser Met Arg
                165                 170                 175

Asp Arg Phe Pro Ile Ser Gln Thr Asp Trp Met His Ala Asp Gly Glu
            180                 185                 190

Leu Arg Ile Val Ala Gly Asp Pro Ala Arg Gly Gly Arg Val Val Val
        195                 200                 205

Leu Ala His Asp Asn Ile Ala Leu Ile Arg Ser Gly Gln Asp Trp Arg
    210                 215                 220

Ala Ala Glu Asp Asp Glu Arg Arg Leu Tyr Leu Asp Glu Ile Glu Pro
225                 230                 235                 240

Thr Leu Arg Ser Gly Met Glu Phe Leu Arg Asp Asn Gly Val Asp Val
                245                 250                 255

Gly Cys Tyr Ser Asn Arg Tyr Val Arg Ser Ile Asp Leu Asp Gly Asn
            260                 265                 270

Leu Leu Asp Glu Ser Tyr Asn Ile Gly His Trp Arg Ser Leu Asp Arg
        275                 280                 285

Leu Glu Arg Trp Ala Glu Ser His Pro Thr His Leu Arg Ile Phe Val
    290                 295                 300

Thr Phe Phe Arg Val Val Thr Gly Leu Ser Lys Leu Arg Leu Tyr His
305                 310                 315                 320

Glu Val Ser Val Phe Asp Ala Lys His Gln Val Tyr Glu Tyr Val Asn
                325                 330                 335

Cys His Pro Thr Thr Gly Met Met Arg Asp Ala Ala Ala Arg
            340                 345                 350
```

<210> 11
<211> 352
<212> PRT
<213> Burkholderia cenocepacia MC0-3

<400> 11

```
Met Glu Ser Ala Ile Asp Lys His Leu Ile Cys Pro Arg Thr Leu Ser
1               5                   10                  15

Arg Arg Val Ala Asp Asp Tyr Gln Pro Pro Phe Pro Met Tyr Val Ala
```

```
            20                  25                  30

Arg Ala Ala Glu Asp Leu Ser Gln Val Val Met Gly Tyr Phe Gly Val
        35                  40                  45

Gln Tyr Ser Gly Ala Asp Lys Arg Ala Ala Ala Met Ala Ala Leu Arg
    50                  55                  60

Arg Ile Val Ala Asp Phe Gly Gly Gln Asp Gly Pro Asn Asn Phe Asp
65                  70                  75                  80

Leu Thr Gln His Thr Asp Asp Glu Gly Tyr Glu Asn Leu Ile Ala Val
                85                  90                  95

Gly Tyr Trp Arg Asp Pro Ala Ala Tyr Ala Arg Trp Ile Ala Ser Pro
            100                 105                 110

Ala Leu Val Glu Trp Trp Ala Ser Asp Ala Arg Leu Ala Asp Gly Ile
        115                 120                 125

Gly Tyr Phe Arg Glu Ile Val Ala Pro Arg Ala Glu Gln Phe Glu Thr
    130                 135                 140

Leu Tyr Ala Phe Thr Ser Asp Phe Pro Gly Val Gly Ala Ile Met Asp
145                 150                 155                 160

Gly Val Ser Gly Glu Ile Glu Glu His Gly Tyr Trp Gly Ser Met Arg
                165                 170                 175

Asp Arg Phe Pro Ile Ser Gln Thr Asp Trp Met Asn Ala Asn Gly Glu
            180                 185                 190

Leu Arg Ile Val Asp Gly Asp Pro Ala Arg Gly Gly Arg Val Val Val
        195                 200                 205

Leu Ala His Asp Asn Ile Ala Leu Ile Arg Ser Gly Gln Asp Trp Arg
    210                 215                 220

Ala Ala Glu Ser Asp Glu Arg Arg Leu Tyr Leu Glu Glu Ile Glu Pro
225                 230                 235                 240

Thr Leu Arg Ser Gly Met Glu Phe Leu Arg Asp Asn Gly Lys Asp Val
                245                 250                 255

Gly Cys Tyr Ser Asn Arg Tyr Val Arg Ser Ile Asp Leu Asp Gly Asn
            260                 265                 270
```

Val Leu Asp Glu Ser Tyr Asn Ile Gly His Trp Arg Ser Leu Asp Arg
275 280 285

Leu Glu Arg Trp Ala Glu Ser His Pro Thr His Leu Arg Ile Phe Val
290 295 300

Thr Phe Phe Arg Val Val Thr Gly Leu Ser Lys Leu Arg Leu Tyr His
305 310 315 320

Glu Val Ser Val Phe Asp Ala Lys His Gln Val Tyr Glu Tyr Val Asn
325 330 335

Cys His Pro Arg Thr Gly Leu Met Arg Asp Ala Val Ala Ile Ala Arg
340 345 350

<210> 12
<211> 348
<212> PRT
<213> Pantoea sp. At-9b

<400> 12

Met Glu Ser Ala Ile Asp Thr His Leu Lys Cys Pro Arg Thr Leu Ser
1 5 10 15

Arg Arg Val His Asp Asp Tyr Gln Pro Pro Phe Pro Met Phe Ala Gly
20 25 30

Arg Ala Asp Ala Ser Leu Thr Gln Val Val Met Ala Tyr Leu Gly Val
35 40 45

Gln Phe Arg Glu Glu Gln Arg Ala Ala Ala Ile Thr Ala Met Gln His
50 55 60

Ile Val Arg Ser Phe Ser Leu Asp Asn Gly Pro Gly Asn His Asp Val
65 70 75 80

Thr Phe His Thr Asp Asn Gln Gly Phe Gly Asn Phe Ile Val Val Gly
85 90 95

Tyr Trp Arg Asp Pro Ala Ala Tyr Cys Arg Trp Leu His Gln Pro Ala
100 105 110

Ile Thr Gly Trp Trp Ser Ser Asp Asp Arg Leu Arg Asp Gly Leu Gly
115 120 125

Tyr Phe Arg Glu Ile Ile Ala Pro Arg Ala Glu Gln Phe Glu Thr Leu
130 135 140

```
Tyr Ala Phe Lys Glu Ala Leu Pro Gly Val Gly Ala Val Met Asp Asn
145                 150                 155                 160

Leu Ser Gly Glu Ile Gln Glu His Gly Tyr Trp Gly Ser Val Arg Asp
                165                 170                 175

Arg Ile Pro Ala Ser Gln Thr Asp Trp Leu Gln Pro Asp Gly Glu Leu
            180                 185                 190

Arg Ile Ile Ser Gly Asp Pro Ala Ala Gly Gly Arg Val Val Val Gln
        195                 200                 205

Gly His Asp Asn Ile Thr Leu Ile Arg Ser Gly Gln Asp Trp Met Asp
    210                 215                 220

Ala Asp Glu Gln Glu Arg Ala Leu Tyr Phe Thr Glu Met Leu Pro Pro
225                 230                 235                 240

Leu Gln Ala Gly Met Asp Phe Leu Arg Asp Glu Gly Gln Thr Leu Gly
                245                 250                 255

Cys Tyr Ser Asn Arg Phe Val Arg Asn Val Asp Ile Asp Gly Asn Val
            260                 265                 270

Leu Asp Ile Ala Tyr Asp Ile Gly Phe Trp Arg Ser Leu Asp Arg Leu
        275                 280                 285

Glu Arg Trp Ala Glu Ser His Pro Thr His Leu Arg Ile Phe Thr Thr
    290                 295                 300

Phe Phe Arg Val Val Ala Gly Leu Gln Lys Leu Arg Leu Tyr His Glu
305                 310                 315                 320

Val Ser Val Ser Asp Ala Arg Phe Gln Thr Phe Glu Tyr Ile Asn Cys
                325                 330                 335

His Pro Gln Thr Gly Met Leu Arg Asp Ala Val Arg
            340                 345
```

<210> 13
<211> 166
<212> PRT
<213> Pseudomonas fluorescens WH6

<400> 13

```
Met Lys Pro Thr Thr Glu Leu Gln Val Val Ala Gly Asp Pro Ala Lys
1               5                   10                  15
```

Gly Gly Arg Val Val Val Met Gly His Asp Asn Leu Thr Leu Ile Arg
20 25 30

Ser Gly Gln Asp Trp Ala Asp Ala Glu Ala Asp Glu Arg Ser Leu Tyr
35 40 45

Leu Asp Glu Ile Leu Pro Thr Leu Gln Asp Gly Met Asp Phe Leu Arg
50 55 60

Asp Asn Gly Gln Pro Leu Gly Cys Tyr Ser Asn Arg Phe Val Arg Asn
65 70 75 80

Ile Asp Leu Asp Gly Asn Phe Leu Asp Val Ser Tyr Asn Ile Gly His
85 90 95

Trp Arg Ser Val Glu Lys Leu Glu Arg Trp Ala Glu Ser His Pro Thr
100 105 110

His Leu Arg Ile Phe Val Thr Phe Phe Arg Val Ala Ala Gly Leu Lys
115 120 125

Lys Leu Arg Leu Tyr His Glu Val Ser Val Ser Asp Ala Lys Ser Gln
130 135 140

Leu Phe Glu Tyr Ile Asn Cys His Pro His Thr Gly Met Leu Arg Asp
145 150 155 160

Ala Gln Ala Ala Thr Ala
165

<210> 14
<211> 186
<212> PRT
<213> Pseudomonas fluorescens WH6

<400> 14

Met Glu Ser Ala Ile Asp Thr His Leu Lys Cys Pro Arg Thr Leu Ser
1 5 10 15

Arg Arg Val Pro Asp Glu Tyr Gln Pro Pro Phe Pro Met Trp Val Ala
20 25 30

Arg Ala Asp Glu Gln Leu Glu Gln Val Val Met Ala Tyr Leu Gly Val
35 40 45

Gln Tyr Arg Gly Glu Ala Gln Arg Glu Ala Ala Leu Gln Ala Met Arg
50 55 60

His Ile Val Gly Ser Phe Ser Leu Ala Asp Gly Pro Gln Thr His Asp
65 70 75 80

Leu Thr His His Thr Asp Ser Ser Gly Phe Asp Asn Leu Ile Val Val
85 90 95

Gly Tyr Trp Lys Asp Pro Gly Ala His Cys Arg Trp Leu Arg Ser Ala
100 105 110

Pro Val Asn Asp Trp Trp Ala Ser Gln Asp Arg Leu Ser Asp Gly Leu
115 120 125

Gly Tyr Phe Arg Glu Ile Ser Ala Pro Arg Ala Glu Gln Phe Glu Thr
130 135 140

Leu Tyr Ala Phe Gln Asp Asn Leu Pro Gly Val Gly Ala Val Met Asp
145 150 155 160

Ala Thr Ser Gly Glu Ile Glu Asn Thr Val Thr Gly Ala Arg Cys Ala
165 170 175

Thr Ala Ser Pro Ser Pro Arg Gln Thr Gly
180 185

<210> 15
<211> 352
<212> PRT
<213> Pseudomonas putida F1

<400> 15

Met Glu Ser Ala Ile Asp Lys His Leu Val Cys Pro Arg Thr Leu Ser
1 5 10 15

Arg Arg Val Pro Asp Asp Tyr Gln Pro Pro Phe Pro Met Trp Val Gly
20 25 30

Arg Ala Asp Glu Gln Leu Thr Gln Val Val Met Ala Tyr Leu Gly Val
35 40 45

Gln Tyr Arg Gly Asp Gly Gln Arg Glu Arg Ala Leu Gln Ala Met Arg
50 55 60

Glu Ile Leu Gly Ser Phe Ser Leu Thr Asp Gly Pro Leu Thr His Asp
65 70 75 80

Leu Thr His His Thr Asp Ser Ser Gly Tyr Asp Asn Leu Met Ile Val
85 90 95

Gly Tyr Trp Lys Asp Ala Gly Ala Tyr Cys Arg Trp Leu Arg Ser Pro
100 105 110

Glu Val Asp Gly Trp Trp Ser Ser Pro Gln Arg Leu Asn Asp Gly Leu
115 120 125

Gly Tyr Tyr Arg Glu Ile Thr Ala Pro Arg Ala Glu Gln Phe Glu Thr
130 135 140

Leu Tyr Ala Phe Gln Asn Asp Leu Pro Gly Val Gly Ala Ile Met Asp
145 150 155 160

Asn Thr Ser Gly Glu Ile Glu Glu His Gly Tyr Trp Gly Ser Met Arg
165 170 175

Asp Arg Phe Pro Val Ser Gln Thr Asp Trp Met Asn Pro Asn Gly Glu
180 185 190

Leu Arg Val Val Ala Gly Asp Pro Ala Lys Gly Gly Arg Val Val Val
195 200 205

Leu Gly His Asp Asn Ile Ala Leu Ile Arg Ser Gly Gln Asp Trp Ala
210 215 220

Thr Ala Glu Ala Ala Glu Arg Ser Leu Tyr Leu Asp Glu Ile Leu Pro
225 230 235 240

Thr Leu Gln Asp Gly Met Asp Phe Leu Arg Asp Asn Gly Gln Pro Leu
245 250 255

Gly Cys Tyr Ser Asn Arg Phe Val Arg Asn Ile Asp Ala Asp Gly Asn
260 265 270

Leu Leu Asp Met Ser Tyr Asn Ile Gly His Trp Arg Ser Leu Glu Lys
275 280 285

Leu Glu Arg Trp Ala Glu Ser His Pro Thr His Leu Arg Ile Phe Val
290 295 300

Thr Phe Phe Arg Val Ala Ala Gly Leu Glu Lys Leu Arg Leu Tyr His
305 310 315 320

Glu Val Ser Val Ser Asp Ala Ser Ser Gln Val Phe Glu Tyr Ile Asn
325 330 335

Cys His Pro His Thr Gly Met Leu Arg Asp Ala Lys Val Ser Ser Asn
340 345 350

<210> 16
<211> 352
<212> PRT
<213> Pseudomonas putida Stamm BIRD-1

<400> 16

```
Met Glu Ser Ala Ile Asp Lys His Leu Met Cys Pro Arg Thr Leu Ser
1               5                   10                  15

Arg Arg Val Pro Asp Asp Tyr Gln Pro Pro Phe Pro Met Trp Val Gly
            20                  25                  30

Arg Ala Asp Glu Gln Leu Thr Gln Val Val Met Ala Tyr Leu Gly Val
        35                  40                  45

Gln Tyr Arg Gly Asp Ser Gln Arg Glu Arg Ala Leu Gln Ala Met Arg
    50                  55                  60

Glu Ile Leu Gly Ser Phe Ser Leu Ser Asp Gly Pro Leu Thr His Asp
65                  70                  75                  80

Leu Thr His His Thr Asp Ser Ser Gly Tyr Asp Asn Leu Met Ile Val
                85                  90                  95

Gly Tyr Trp Lys Asp Thr Gly Ala Tyr Cys Arg Trp Ser Arg Ser Pro
            100                 105                 110

Glu Val Asp Gly Trp Trp Ser Ser Pro Gln Arg Leu Asn Asp Gly Leu
        115                 120                 125

Gly Tyr Tyr Arg Glu Ile Thr Ala Pro Arg Ala Glu Gln Phe Glu Thr
    130                 135                 140

Leu Tyr Ala Phe Gln Ser Asp Leu Pro Gly Val Gly Ala Ile Met Asp
145                 150                 155                 160

Asn Thr Ser Gly Glu Ile Glu Glu His Gly Tyr Trp Gly Ser Met Arg
                165                 170                 175

Asp Arg Phe Pro Val Ser Gln Thr Asp Trp Met Asn Pro Asn Gly Glu
            180                 185                 190

Leu Arg Val Val Ala Gly Asp Pro Ala Lys Gly Gly Arg Val Val Val
        195                 200                 205

Ile Gly His Asp Asn Ile Ala Leu Ile Arg Ser Gly Gln Asp Trp Ala
    210                 215                 220
```

Ala Ala Glu Ala Ala Glu Arg Ser Leu Tyr Leu Asp Glu Ile Leu Pro
225 230 235 240

Thr Leu Gln Asp Gly Met Asp Phe Leu Arg Asp Asn Gly Gln Pro Leu
245 250 255

Gly Cys Tyr Ser Asn Arg Phe Val Arg Asn Ile Asp Ala Asp Gly Asn
260 265 270

Val Leu Asp Met Ser Tyr Asn Ile Gly His Trp Arg Ser Leu Glu Lys
275 280 285

Leu Glu Arg Trp Ala Glu Ser His Pro Thr His Leu Arg Ile Phe Val
290 295 300

Thr Phe Phe Arg Val Ala Ala Gly Leu Glu Lys Leu Arg Leu Tyr His
305 310 315 320

Glu Val Ser Val Ser Asp Ala Ser Ser Gln Val Phe Glu Tyr Ile Asn
325 330 335

Cys His Pro His Thr Gly Met Leu Arg Asp Ala Lys Val Ser Ser Asn
340 345 350

<210> 17
<211> 332
<212> PRT
<213> Shewanella woodyi ATCC 51908

<400> 17

Met Met Asn Asn Met Pro Lys Asn Trp Thr Pro Pro Ala Pro Ala Trp
1 5 10 15

Thr Ser Leu Trp Lys Thr Asp Glu Glu Asn Leu Val Cys Gly Leu Phe
20 25 30

Ala Ile Gln Gly Gln His Ser Ala Pro Leu Asp Asp Trp Ala Lys Lys
35 40 45

Ala Phe Thr Gly Glu Phe Ser Pro Lys Leu Leu Glu Gln Gly Met Phe
50 55 60

Thr Asp Lys Ala Gly Ile Thr Asn Tyr Leu Tyr Ile Ala Tyr Trp Phe
65 70 75 80

Ala Ser Asp Tyr Lys Thr Trp Trp Gln Gln Ser Ala Ala Asn Ser Trp
85 90 95

```
Trp Ala Ser Pro Leu Leu Asp Glu Gly Asp Ile Ser Val Trp Arg Glu
            100                 105                 110

Val Phe Thr Met Pro His Gln Arg Phe Glu Thr Leu His Ser Ser Glu
        115                 120                 125

Asn Ala His Gly Ala Ala Arg Leu Ser Pro Ser Leu Glu Gly Pro Met
    130                 135                 140

Met Glu His Gly Tyr Ser Gly Ala Ala Arg Asp Arg Ile Pro Cys Ser
145                 150                 155                 160

Ser Ser Gln Asp Ile Lys Asn Asp Asn Ser Ile Trp Glu His Leu Gln
                165                 170                 175

Val Asn Val Glu Asn Lys Ser Asn Arg Ile Lys Leu Ser Pro Pro Lys
            180                 185                 190

Asn Met Cys Val Ile Arg Ser Gly Gln Asp Trp Thr His Cys Glu Glu
        195                 200                 205

Asp Glu Lys Glu Tyr Tyr Leu Thr Asn Val His Thr Val Leu Lys Lys
    210                 215                 220

Gly Met Asp Tyr Leu Ser Asn Asn Pro Val Lys Thr His Cys Ala Ser
225                 230                 235                 240

Met Arg Phe Ile Thr Lys Thr Asp Gly Asn Trp Cys Ser Val Glu Gln
                245                 250                 255

Thr Phe Gly Leu Gly Tyr Gly Asn Asp Ile Tyr Ala Phe Glu Asn Trp
            260                 265                 270

Ala Lys Ser His Pro Thr His Ile Ala Ile Phe Asp Arg Phe Met Gly
        275                 280                 285

Met Val Glu Lys Tyr Asn Val Asp Leu Lys Leu Gln Leu Trp His Glu
    290                 295                 300

Val Thr Leu Ile Pro Glu Gln Asp Cys Glu Phe Glu Tyr Ile Asn Cys
305                 310                 315                 320

His Gly Gln Thr Gly Leu Leu Cys Tyr Ile Asn Ile
                325                 330
```

<210> 18
<211> 343

<212> PRT
<213> Sphingomonas wittichii RW1

<400> 18

```
Met Asp Ser Ser Ile Thr Pro His Leu Ala Cys Pro Arg Ser Arg Pro
1               5                   10                  15

Arg Arg Ile Ala Asp Asp Tyr Ala Pro Pro Tyr Pro Ala Trp Ser Ala
            20                  25                  30

Arg Ile Asp Pro Ala Ile Gly Gln Val Val Met Ala Ser Tyr Gly Val
        35                  40                  45

Gln Gly Arg Glu Ser Gly Asp Val Ala Ala Ala Leu Ala His Val Arg
    50                  55                  60

Ala Leu Arg Ala Thr Leu Asp Ala Asp Val Arg His Val Asp Leu Ala
65                  70                  75                  80

Arg Tyr Val Asp Glu Ala Gly Tyr Asp Thr Leu Val Leu Gln Pro Tyr
                85                  90                  95

Trp Thr Asp Pro Glu Ala Phe Arg Arg Trp Glu Ala Arg Ala Asp Val
            100                 105                 110

Ala Ala Leu Leu Ala Ala Glu Thr Gly Leu Gly His Phe Arg Glu Ile
        115                 120                 125

Leu Thr Pro Thr Val Glu Arg Leu Glu Thr Leu Tyr Ser Thr Glu Asp
    130                 135                 140

Glu Met Glu Gly Leu Gly Arg Ala Leu Glu Arg Arg Ser Pro Pro Val
145                 150                 155                 160

Gln Glu His Ala Tyr Trp Gly Ser Ala Arg Asp Arg Phe Pro Ile Ala
                165                 170                 175

Gln Thr Asp Ala Leu Glu Pro Ala Gly Gln Leu Gly Phe Ala Ala Asp
            180                 185                 190

Gly Ala Gly Arg Val Thr Val Arg Gly His Asp Asn Ile Ala Ile Ile
        195                 200                 205

Arg Ser Gly Gln Asp Trp Gly Pro Thr Gly Gly Glu Glu Arg Arg Leu
    210                 215                 220

Tyr Leu Ala Glu Ile Glu Pro Val Leu Arg Ala Gly Met Asp Phe Leu
225                 230                 235                 240
```

```
Arg Asp Arg Gly Gly Glu Cys Gly Cys Tyr Leu Asn Arg Tyr Met Arg
                245                 250                 255

Ile Val Asp Met Asp Gly Ala Pro Gln Glu Lys Ser Phe Gly Trp Ser
            260                 265                 270

Tyr Trp Arg Ser Leu Gly Asp Met Glu Asn Trp Ser Glu Ala His Pro
        275                 280                 285

Ser His Leu Ala Ile Phe Gly Thr Phe Met Arg Ile Val Gln Gln Leu
    290                 295                 300

Asn Phe Asp Leu Lys Leu Arg Leu Trp His Glu Val Tyr Val Val Thr
305                 310                 315                 320

Pro Asp Gln Gln Leu Tyr Asp Tyr Arg Asn Cys His Pro Asp Thr Gly
                325                 330                 335

Phe Leu Lys Gln Met Pro Arg
            340
```

<210> 19
<211> 353
<212> PRT
<213> Variovorax paradoxus EPS

<400> 19

```
Met Glu Ser Ala Ile Ala Glu His Leu Lys Cys Pro Arg Thr Arg His
1               5                   10                  15

Arg Arg Val Glu Asp Asp Tyr Thr Pro Pro Tyr Pro Val Trp Ser Ala
            20                  25                  30

Arg Ala Pro Thr Ser Val Thr Gln Val Thr Met Gly Tyr Phe Gly Val
        35                  40                  45

Gln Ser Arg Gly Pro Glu Met Gln Gly Arg Ala Cys Ala Ala Leu Met
    50                  55                  60

Lys Ile Ala Arg Asp Phe Ala Leu Pro Asp Gly Pro Gly His His Asp
65                  70                  75                  80

Leu Ala His Tyr Val Asp Ala Asp Gly Phe Asp Asn Met Val Ala Ile
                85                  90                  95

Ala Tyr Trp His Asp Ala Ala Ala Phe Ala Arg Trp Ser Ala Thr Pro
            100                 105                 110
```

Ala Ile Asp Ala Trp Trp Arg Ser Asp Glu Arg Leu Asn Glu Gly Leu
115 120 125

Gly Tyr Phe Arg Glu Ile Ala Ser Pro Arg Val Glu His Phe Glu Thr
130 135 140

Met Phe Asn Thr Pro Asp Arg Phe Glu Gly Ile Gly Val Val Met Gly
145 150 155 160

Glu Leu Ser Gly Glu Leu Gln Glu His Gly Tyr Trp Gly Ser Met Arg
165 170 175

Asp Arg Ile Pro Leu Ser Gln Thr Asp Ala Leu Ser Pro Ser Gly Thr
180 185 190

Arg Ala Val Val Ala Gly Thr Pro Ala Pro Gly Gln Arg Val Arg Ile
195 200 205

Ala Gly His Glu Asn Ile Ala Met Ile Arg Ser Gly Gln Glu Trp Ala
210 215 220

Asp Thr Thr Gly Gln Glu Arg Thr Leu Tyr Leu Glu Asp Met Glu Pro
225 230 235 240

Val Leu Arg Glu Gly Met Asp Phe Leu Arg Asp Gln Gly Leu Gly Ile
245 250 255

Gly Cys Tyr Ser Asn Arg Tyr Met His His Leu Asp Ala Lys Gly Ala
260 265 270

Pro Leu Gln Lys Ser Phe Gly Leu Ser Tyr Trp Arg Ser Leu Ala Asp
275 280 285

Met Glu Arg Trp Ala Glu Ser His Pro Thr His Val Ala Ile Phe Gly
290 295 300

Ser Phe Met Arg Tyr Val Gln Ala Leu Asn Phe Gln Leu Gln Leu Arg
305 310 315 320

Val Tyr His Glu Val Ser Val Leu Lys Ala Asp Glu Gln Ser Tyr Glu
325 330 335

Tyr Ile Asn Cys Arg Ala Arg Ser Gly Leu Met Asn Gly Leu Ala Val
340 345 350

Thr

<210> 20
<211> 305
<212> PRT
<213> Variovorax paradoxus S110

<400> 20

Met Glu Ser Arg Asp Ala Asp Ala Arg Ala Leu Leu Ala Arg Val Ala
1               5                   10                  15

Asn Thr Phe Ala Gly Val Gly Gly Pro Ala Ser Leu Gly Arg Gly Val
            20                  25                  30

Ala Thr Gly Ala Gly Gly Glu Arg Ser Asp Ile Phe Tyr Ala Tyr Trp
        35                  40                  45

Asn Ser Ser Ala Glu Tyr Ala Ser Trp Leu Thr Thr Pro Arg Val Ala
    50                  55                  60

Ser Leu Trp Thr Asp Asp Ala Leu Leu Arg Gly Pro Ile Gly Leu Trp
65                  70                  75                  80

Arg Glu Ser Met Ile Ile Pro Val Glu Arg Asn Glu Thr Asn Tyr Ser
                85                  90                  95

Asn Asp Ala Ala Tyr Asp Gly Ile Ala Gln Ile Asp Lys Glu Met Arg
            100                 105                 110

Lys Thr Asp Val His Gly Tyr Trp Gly Ser Ala Arg Glu Arg Ile Pro
        115                 120                 125

Ala Ser Ala His Asp Thr Met Pro Ser Ala Ala Pro Asp Phe Met Gly
    130                 135                 140

Arg Pro Ala Ala Ser Met Glu Thr Leu Gly Arg Arg Phe Arg Ile Thr
145                 150                 155                 160

Leu Pro Gly Asn Thr Cys Val Ile Arg Ser Phe Gln Asp Trp Ser Gln
                165                 170                 175

Ala Gln Ala Ala Glu Val Asp Trp Tyr Leu Gly Asn Val Glu Pro Val
            180                 185                 190

Leu Arg Val Gly Leu Asp Tyr Leu Asn Gly Asn Arg Thr Glu Ala Lys
        195                 200                 205

Cys Tyr Gly Met Arg Tyr Ile Arg Glu Tyr Asp Ile Ser Gly Ala Ile

210 215 220

Asp Leu Asn Arg Thr Ser Thr Phe Gly Tyr Phe Glu Ser Leu Gln Thr
225 230 235 240

Leu Glu Arg Trp Thr His Thr His Pro Thr His Leu Asp Ile Phe Arg
245 250 255

Ala Ala Ile Ser Met Val Gln Arg Phe Gln Gly Glu Val Ala Val Lys
260 265 270

Leu Gly His Glu Val Ser Val Leu Pro Glu Gly Met Leu Ser Ala Glu
275 280 285

Tyr Val Asn Cys Ala Arg Ser Thr Gly Phe Leu Pro Trp Phe His Glu
290 295 300

Asp
305

<210> 21
<211> 353
<212> PRT
<213> Variovorax paradoxus S110

<400> 21

```
Met Glu Ser Ala Ile Ala Glu His Leu Lys Cys Pro Arg Thr Arg His
1               5                   10                  15

Arg Arg Val Glu Asp Asp Tyr Ala Pro Pro Tyr Pro Ala Trp Ser Ala
            20                  25                  30

Arg Ala Pro Gly Ala Val Arg Gln Val Val Met Gly Tyr Phe Gly Val
        35                  40                  45

Gln Ser Arg Gly Ala Gly Met Gln Gly Arg Ala Cys Ala Ala Leu Met
    50                  55                  60

Lys Ile Ala Ala Gly Phe Ala Leu Pro Asp Gly Pro Gly His His Asp
65                  70                  75                  80

Phe Ala His His Val Asp Ala Ala Gly Cys Asp Asn Met Val Ala Ile
                85                  90                  95

Ala Tyr Trp Asn Asp Pro Ala Ala His Ala Arg Trp Cys Thr Ala Pro
            100                 105                 110

Glu Val Asp Ala Trp Trp Arg Ser Asp Glu Arg Leu Ala Asp Gly Leu
```

115 120 125

Gly Tyr Phe Arg Glu Ile Val Ala Pro Arg Ala Glu His Phe Glu Thr
130 135 140

Met Phe Asn Thr Pro Asp Arg Leu Glu Gly Val Gly Val Val Met Gly
145 150 155 160

Gly Val Ser Gly Glu Leu Gln Glu His Gly Tyr Trp Gly Ser Met Arg
165 170 175

Asp Arg Ile Pro Leu Ser Gln Thr Asp Ala Met Ala Pro Ser Gly Thr
180 185 190

Arg Ala Val Ile Ala Gly Ala Pro Ala Pro Gly Gln Arg Val Arg Ile
195 200 205

Ala Gly His Glu Asn Ile Ala Met Ile Arg Ser Gly Gln Glu Trp Ala
210 215 220

Asp Thr Thr Gly Gln Glu Arg Ala Leu Tyr Leu Gly Glu Met Glu Pro
225 230 235 240

Val Leu Arg Glu Gly Met Asp Phe Leu Arg Asp Gln Gly Leu His Ile
245 250 255

Gly Cys Tyr Ser Asn Arg Tyr Met Gln His Leu Asp Ala Lys Gly Ala
260 265 270

Pro Leu Glu Lys Ser Phe Gly Leu Ser Phe Trp His Ser Leu Ala Asp
275 280 285

Met Glu Arg Trp Ala Glu Ser His Pro Thr His Val Ala Ile Phe Gly
290 295 300

Ser Phe Met Arg Tyr Val Gln Ala Leu Asn Phe Gln Leu Gln Leu Arg
305 310 315 320

Val Tyr His Glu Val Ser Val Leu Lys Ala Asp Glu Gln Ser Tyr Glu
325 330 335

Tyr Ile Asn Cys His Ala Gly Ser Gly Leu Met Asn Gly Leu Gly Glu
340 345 350

Val

<210> 22
<211> 347
<212> PRT
<213> Xanthobacter autotrophicus Py2

<400> 22

```
Met Val Phe His Val Glu Tyr Pro Arg Ile Val Pro Glu Arg Arg Pro
1               5                   10                  15

Pro Gly His Glu Pro Ala Ala Pro Arg Phe Ser Leu Arg Trp Glu Gln
            20                  25                  30

Pro Val Gly Leu Val Val Cys Ala Tyr Phe Gly Leu Gln Gly Gln Asp
        35                  40                  45

Leu Ala Trp Asp Glu Gln Lys Ala Phe Phe Asp Arg Leu Gln Thr Ser
    50                  55                  60

Phe Gly Thr Asp Gly Pro Val Ala His Glu Ile Met Arg Met His Asp
65                  70                  75                  80

Glu Thr Gly Ala Val Asn Ala Ile Leu Val Ala Tyr Trp Leu Asp Ala
                85                  90                  95

Thr Ala His Ala Arg Trp Glu Arg Asn Ser Pro Phe Met Ala Trp Phe
            100                 105                 110

Arg Asp Pro Ala Arg Leu Glu Gly Thr Arg Gly Val Trp Arg Glu Thr
        115                 120                 125

Met His Val Pro Tyr Asp Arg His Glu Thr Ile Tyr Ser Thr Pro Ser
    130                 135                 140

Tyr Val Ile Gly Leu Ala Arg Thr Pro Gly Ala Thr Arg Val Pro Ile
145                 150                 155                 160

Thr Thr Asn Gly Tyr Phe Gly Ala Met Arg Asp Arg Met Pro Val Ser
                165                 170                 175

Ala Ile Asp Thr Leu Glu Ser Pro Leu Gly Ala Met Pro Pro Arg Arg
            180                 185                 190

Ala Pro Asp Ser His Gly Arg Arg Leu Thr Ala Ala Phe Pro Leu Asn
        195                 200                 205

Leu Ile Ser Ile Arg Ser Gly Gln Tyr Trp Glu Gly Ala Gly Asn Glu
    210                 215                 220
```

Gln Thr Ala Asp Tyr Ile Asp Asn Leu Gln Pro Lys Leu Met Arg Gly
225 230 235 240

Met Ala His Leu Ser Ser His Pro Glu Gln Thr Gly Thr Leu Thr Leu
245 250 255

Arg Ile Met Thr Asn Leu Asp Ala Glu Gly Arg Pro Arg Ala Glu Thr
260 265 270

Ser Val His Gly Tyr Phe Leu Ser Met Ala His Leu Glu Glu Trp Ser
275 280 285

Arg Ser His Glu Thr His Leu Asp Ile Tyr Arg His Ala Ile Ala Met
290 295 300

Asn Arg Leu Tyr Lys Glu Lys Arg Glu Val Phe Thr Trp His Glu Val
305 310 315 320

Phe Ala Leu Leu Pro Gly Ala His Ala Glu Tyr Ala Asn Cys His Gly
325 330 335

Gly Thr Gly Leu Leu Pro Tyr Phe Ala Asp Ala
340 345

<210> 23
<211> 363
<212> PRT
<213> Gibberella zeae

<400> 23

Met Leu Arg Ser Arg Phe Pro Ala Ser His His Phe Thr Val Ser Val
1 5 10 15

Phe Gly Cys Gln Tyr His Ser Glu Ala Pro Ser Val Glu Lys Thr Glu
20 25 30

Leu Ile Gly Arg Phe Asp Lys Leu Ile Asp Ser Ala Ala Ile His Val
35 40 45

Glu His Leu Glu Gln Asn Asp Val Pro Ser Lys Ile Trp Met Ser Tyr
50 55 60

Trp Glu Ser Pro Gln Lys Phe Lys Gln Trp Trp Glu Lys Asp Asp Thr
65 70 75 80

Ala Ser Phe Trp Ala Ser Leu Pro Asp Asp Ala Gly Phe Trp Arg Glu
85 90 95

```
Thr Phe Ser Leu Pro Ala Thr Arg Ala Met Tyr Glu Gly Thr Gly Lys
            100                 105                 110

Asp Ala Tyr Gly Phe Gly His Cys Gly Ser Leu Ile Pro Leu Thr Thr
        115                 120                 125

Lys Thr Gly Tyr Trp Gly Ala Tyr Arg Ser Arg Met Thr Pro Asp Phe
    130                 135                 140

Glu Gly Asp Thr Phe Ser Ser Pro Ile Pro Thr Tyr Ala Asp Gln Ser
145                 150                 155                 160

Val Pro Ala Asp Lys Ile Arg Pro Gly Arg Val Arg Ile Thr Asp Phe
                165                 170                 175

Pro Asp Asn Leu Cys Met Val Val Glu Gly Gln His Tyr Ala Asp Met
            180                 185                 190

Gly Glu Arg Glu Arg Glu Tyr Trp Asn Glu Asn Phe Asp Gly Leu Thr
        195                 200                 205

Lys Gln Trp Val Thr Asn Val Val Thr Ala Gly His Glu Gln Gly Met
    210                 215                 220

Val Ile Ala Arg Ala Cys His Gly Phe Ala Gly Glu Lys Lys Leu Gly
225                 230                 235                 240

Ala Thr Asn Gly Pro Val Asn Gly Ile Phe Pro Gly Leu Asp Tyr Val
                245                 250                 255

His Gln Ala Gln Ile Leu Ile Trp Gln Asp Ile Ser Lys Met Glu His
            260                 265                 270

Ile Gly Arg Tyr Asp Gln Thr His Val Lys Leu Arg Arg Asp Phe Met
        275                 280                 285

Lys Ala Tyr Gly Pro Gly Gly Glu Met Glu Gly Gly Asp Leu Leu Leu
    290                 295                 300

Trp Val Asp Leu Gly Ile Leu Lys Lys Asp Glu Ile Asp Ala Glu Tyr
305                 310                 315                 320

Val Gly Cys Tyr Glu Ser Thr Gly Phe Leu Lys Leu Asp Lys Gly Gln
                325                 330                 335

Phe Phe Lys Val Glu Ser Thr Ala Gly Ser Lys Leu Pro Ser Phe Phe
            340                 345                 350
```

Asp Glu Pro Ile Glu Ser Lys Pro Ile Glu Trp
355 360

<210> 24
<211> 352
<212> PRT
<213> Pseudomonas chlororaphis

<400> 24

Met Glu Ser Ala Ile Asp Thr His Leu Lys Cys Pro Arg Thr Leu Ser
1 5 10 15

Arg Arg Val Pro Glu Glu Tyr Gln Pro Pro Phe Pro Met Trp Val Ala
20 25 30

Arg Ala Asp Glu Gln Leu Gln Gln Val Val Met Gly Tyr Leu Gly Val
35 40 45

Gln Tyr Arg Gly Glu Ala Gln Arg Glu Ala Ala Leu Gln Ala Met Arg
50 55 60

His Ile Val Ser Ser Phe Ser Leu Pro Asp Gly Pro Gln Thr His Asp
65 70 75 80

Leu Thr His His Thr Asp Ser Ser Gly Phe Asp Asn Leu Met Val Val
85 90 95

Gly Tyr Trp Lys Asp Pro Ala Ala His Cys Arg Trp Leu Arg Ser Ala
100 105 110

Glu Val Asn Asp Trp Trp Thr Ser Gln Asp Arg Leu Gly Glu Gly Leu
115 120 125

Gly Tyr Phe Arg Glu Ile Ser Ala Pro Arg Ala Glu Gln Phe Glu Thr
130 135 140

Leu Tyr Ala Phe Gln Asp Asn Leu Pro Gly Val Gly Ala Val Met Asp
145 150 155 160

Ser Thr Ser Gly Glu Ile Glu Glu His Gly Tyr Trp Gly Ser Met Arg
165 170 175

Asp Arg Phe Pro Ile Ser Gln Thr Asp Trp Met Lys Pro Thr Asn Glu
180 185 190

Leu Gln Val Val Ala Gly Asp Pro Ala Lys Gly Gly Arg Val Val Ile
195 200 205

Met Gly His Asp Asn Ile Ala Leu Ile Arg Ser Gly Gln Asp Trp Ala
210 215 220

Asp Ala Glu Ala Glu Glu Arg Ser Leu Tyr Leu Asp Glu Ile Leu Pro
225 230 235 240

Thr Leu Gln Asp Gly Met Asp Phe Leu Arg Asp Asn Gly Gln Pro Leu
245 250 255

Gly Cys Tyr Ser Asn Arg Phe Val Arg Asn Ile Asp Leu Asp Gly Asn
260 265 270

Phe Leu Asp Val Ser Tyr Asn Ile Gly His Trp Arg Ser Leu Glu Lys
275 280 285

Leu Glu Arg Trp Ala Glu Ser His Pro Thr His Leu Arg Ile Phe Val
290 295 300

Thr Phe Phe Arg Val Ala Ala Gly Leu Lys Lys Leu Arg Leu Tyr His
305 310 315 320

Glu Val Ser Val Ser Asp Ala Lys Ser Gln Val Phe Glu Tyr Ile Asn
325 330 335

Cys His Pro His Thr Gly Met Leu Arg Asp Ala Val Val Ala Pro Thr
340 345 350

<210> 25
<211> 352
<212> PRT
<213> Pseudomonas sp. K-9

<400> 25

Met Glu Ser Ala Ile Asp Thr His Leu Lys Cys Pro Arg Thr Leu Ser
1 5 10 15

Arg Arg Val Pro Asp Glu Tyr Gln Pro Pro Phe Ala Met Trp Met Ala
20 25 30

Arg Ala Asp Glu His Leu Glu Gln Val Val Met Ala Tyr Phe Gly Val
35 40 45

Gln Tyr Arg Gly Glu Ala Gln Arg Ala Ala Ala Leu Gln Ala Met Arg
50 55 60

His Ile Val Glu Ser Phe Ser Leu Ala Asp Gly Pro Gln Thr His Asp
65 70 75 80

```
Leu Thr His His Thr Asp Asn Ser Gly Phe Asp Asn Leu Ile Val Val
                85                  90                  95

Gly Tyr Trp Lys Asp Pro Ala Ala His Cys Arg Trp Leu Arg Ser Ala
            100                 105                 110

Pro Val Asn Ala Trp Trp Ala Ser Glu Asp Arg Leu Asn Asp Gly Leu
        115                 120                 125

Gly Tyr Phe Arg Glu Ile Ser Ala Pro Arg Ala Glu Gln Phe Glu Thr
    130                 135                 140

Leu Tyr Ala Phe Gln Asp Asn Leu Pro Gly Val Gly Ala Val Met Asp
145                 150                 155                 160

Arg Ile Ser Gly Glu Ile Glu Glu His Gly Tyr Trp Gly Ser Met Arg
                165                 170                 175

Asp Arg Phe Pro Ile Ser Gln Thr Asp Trp Met Lys Pro Thr Ser Glu
            180                 185                 190

Leu Gln Val Ile Ala Gly Asp Pro Ala Lys Gly Gly Arg Val Val Val
        195                 200                 205

Leu Gly His Gly Asn Leu Thr Leu Ile Arg Ser Gly Gln Asp Trp Ala
    210                 215                 220

Asp Ala Glu Ala Glu Glu Arg Ser Leu Tyr Leu Asp Glu Ile Leu Pro
225                 230                 235                 240

Thr Leu Gln Asp Gly Met Asp Phe Leu Arg Asp Asn Gly Gln Pro Leu
                245                 250                 255

Gly Cys Tyr Ser Asn Arg Phe Val Arg Asn Ile Asp Leu Asp Gly Asn
            260                 265                 270

Phe Leu Asp Val Ser Tyr Asn Ile Gly His Trp Arg Ser Val Glu Lys
        275                 280                 285

Leu Glu Arg Trp Thr Glu Ser His Pro Thr His Leu Arg Ile Phe Val
    290                 295                 300

Thr Phe Phe Arg Val Ala Ala Gly Leu Lys Lys Leu Arg Leu Tyr His
305                 310                 315                 320

Glu Val Ser Val Ser Asp Ala Lys Ser Gln Ile Phe Gly Tyr Ile Asn
                325                 330                 335
```

Cys His Pro Gln Thr Gly Met Leu Arg Asp Ala Gln Val Ser Pro Ala
340 345 350

<210> 26
<211> 353
<212> PRT
<213> Rhodococcus erythropolis

<400> 26

Met Glu Ser Ala Ile Gly Glu His Leu Gln Cys Pro Arg Thr Leu Thr
1 5 10 15

Arg Arg Val Pro Asp Thr Tyr Thr Pro Pro Phe Pro Met Trp Val Gly
20 25 30

Arg Ala Asp Asp Ala Leu Gln Gln Val Val Met Gly Tyr Leu Gly Val
35 40 45

Gln Phe Arg Asp Glu Asp Gln Arg Pro Ala Ala Leu Gln Ala Met Arg
50 55 60

Asp Ile Val Ala Gly Phe Asp Leu Pro Asp Gly Pro Ala His His Asp
65 70 75 80

Leu Thr His His Ile Asp Asn Gln Gly Tyr Glu Asn Leu Ile Val Val
85 90 95

Gly Tyr Trp Lys Asp Val Ser Ser Gln His Arg Trp Ser Thr Ser Thr
100 105 110

Pro Ile Ala Ser Trp Trp Glu Ser Glu Asp Arg Leu Ser Asp Gly Leu
115 120 125

Gly Phe Phe Arg Glu Ile Val Ala Pro Arg Ala Glu Gln Phe Glu Thr
130 135 140

Leu Tyr Ala Phe Gln Glu Asp Leu Pro Gly Val Gly Ala Val Met Asp
145 150 155 160

Gly Ile Ser Gly Glu Ile Asn Glu His Gly Tyr Trp Gly Ser Met Arg
165 170 175

Glu Arg Phe Pro Ile Ser Gln Thr Asp Trp Met Gln Ala Ser Gly Glu
180 185 190

Leu Arg Val Ile Ala Gly Asp Pro Ala Val Gly Gly Arg Val Val Val
195 200 205

```
Arg Gly His Asp Asn Ile Ala Leu Ile Arg Ser Gly Gln Asp Trp Ala
    210                 215                 220

Asp Ala Glu Ala Asp Glu Arg Ser Leu Tyr Leu Asp Glu Ile Leu Pro
225                 230                 235                 240

Thr Leu Gln Ser Gly Met Asp Phe Leu Arg Asp Asn Gly Pro Ala Val
                245                 250                 255

Gly Cys Tyr Ser Asn Arg Phe Val Arg Asn Ile Asp Ile Asp Gly Asn
            260                 265                 270

Phe Leu Asp Leu Ser Tyr Asn Ile Gly His Trp Ala Ser Leu Asp Gln
        275                 280                 285

Leu Glu Arg Trp Ser Glu Ser His Pro Thr His Leu Arg Ile Phe Thr
    290                 295                 300

Thr Phe Phe Arg Val Ala Ala Gly Leu Ser Lys Leu Arg Leu Tyr His
305                 310                 315                 320

Glu Val Ser Val Phe Asp Ala Ala Asp Gln Leu Tyr Glu Tyr Ile Asn
                325                 330                 335

Cys His Pro Gly Thr Gly Met Leu Arg Asp Ala Val Thr Ile Ala Glu
            340                 345                 350

His
```

<210> 27
<211> 353
<212> PRT
<213> Rhodococcus globerulus

<400> 27

```
Met Glu Ser Ala Ile Gly Glu His Leu Gln Cys Pro Arg Thr Leu Thr
1               5                   10                  15

Arg Arg Val Pro Asp Thr Tyr Thr Pro Pro Phe Pro Met Trp Val Gly
            20                  25                  30

Arg Ala Asp Asp Thr Leu His Gln Val Val Met Gly Tyr Leu Gly Val
        35                  40                  45

Gln Phe Arg Gly Glu Asp Gln Arg Pro Ala Ala Leu Arg Ala Met Arg
    50                  55                  60
```

Asp Ile Val Ala Gly Phe Asp Leu Pro Asp Gly Pro Ala His His Asp
65 70 75 80

Leu Thr His His Ile Asp Asn Gln Gly Tyr Glu Asn Leu Ile Val Val
85 90 95

Gly Tyr Trp Lys Asp Val Ser Ser Gln His Arg Trp Ser Thr Ser Pro
100 105 110

Pro Val Ser Ser Trp Trp Glu Ser Glu Asp Arg Leu Ser Asp Gly Leu
115 120 125

Gly Phe Phe Arg Glu Ile Val Ala Pro Arg Ala Glu Gln Phe Glu Thr
130 135 140

Leu Tyr Ala Phe Gln Asp Asp Leu Pro Gly Val Gly Ala Val Met Asp
145 150 155 160

Gly Val Ser Gly Glu Ile Asn Glu His Gly Tyr Trp Gly Ser Met Arg
165 170 175

Glu Arg Phe Pro Ile Ser Gln Thr Asp Trp Met Gln Ala Ser Gly Glu
180 185 190

Leu Arg Val Val Ala Gly Asp Pro Ala Val Gly Gly Arg Val Val Val
195 200 205

Arg Gly His Asp Asn Ile Ala Leu Ile Arg Ser Gly Gln Asp Trp Ala
210 215 220

Asp Ala Glu Ala Asp Glu Arg Ser Leu Tyr Leu Asp Glu Ile Leu Pro
225 230 235 240

Thr Leu Gln Ser Gly Met Asp Phe Leu Arg Asp Asn Gly Pro Ala Val
245 250 255

Gly Cys Tyr Ser Asn Arg Phe Val Arg Asn Ile Asp Ile Asp Gly Asn
260 265 270

Phe Leu Asp Leu Ser Tyr Asn Ile Gly His Trp Ala Ser Leu Asp Gln
275 280 285

Leu Glu Arg Trp Ser Glu Ser His Pro Thr His Leu Arg Ile Phe Thr
290 295 300

Thr Phe Phe Arg Val Ala Glu Gly Leu Ser Lys Leu Arg Leu Tyr His

305 310 315 320

Glu Val Ser Val Phe Asp Ala Ala Asp Gln Leu Tyr Glu Tyr Ile Asn
325 330 335

Cys His Pro Gly Thr Gly Met Leu Arg Asp Ala Val Ile Thr Ala Glu
340 345 350

His

<210> 28
<211> 497
<212> PRT
<213> Arabidopsis thaliana

<400> 28

```
Met Glu Met Ile Leu Ser Ile Ser Leu Cys Leu Thr Thr Leu Ile Thr
1               5                   10                  15

Leu Leu Leu Leu Arg Arg Phe Leu Lys Arg Thr Ala Thr Lys Val Asn
            20                  25                  30

Leu Pro Pro Ser Pro Trp Arg Leu Pro Val Ile Gly Asn Leu His Gln
        35                  40                  45

Leu Ser Leu His Pro His Arg Ser Leu Arg Ser Leu Ser Leu Arg Tyr
    50                  55                  60

Gly Pro Leu Met Leu Leu His Phe Gly Arg Val Pro Ile Leu Val Val
65                  70                  75                  80

Ser Ser Gly Glu Ala Ala Gln Glu Val Leu Lys Thr His Asp His Lys
                85                  90                  95

Phe Ala Asn Arg Pro Arg Ser Lys Ala Val His Gly Leu Met Asn Gly
            100                 105                 110

Gly Arg Asp Val Val Phe Ala Pro Tyr Gly Glu Tyr Trp Arg Gln Met
        115                 120                 125

Lys Ser Val Cys Ile Leu Asn Leu Leu Thr Asn Lys Met Val Glu Ser
    130                 135                 140

Phe Glu Lys Val Arg Glu Asp Glu Val Asn Ala Met Ile Glu Lys Leu
145                 150                 155                 160

Glu Lys Ala Ser Ser Ser Ser Ser Ser Glu Asn Leu Ser Glu Leu Phe
```

165 170 175

Ile Thr Leu Pro Ser Asp Val Thr Ser Arg Val Ala Leu Gly Arg Lys
180 185 190

His Ser Glu Asp Glu Thr Ala Arg Asp Leu Lys Lys Arg Val Arg Gln
195 200 205

Ile Met Glu Leu Leu Gly Glu Phe Pro Ile Gly Glu Tyr Val Pro Ile
210 215 220

Leu Ala Trp Ile Asp Gly Ile Arg Gly Phe Asn Asn Lys Ile Lys Glu
225 230 235 240

Val Ser Arg Gly Phe Ser Asp Leu Met Asp Lys Val Val Gln Glu His
245 250 255

Leu Glu Ala Ser Asn Asp Lys Ala Asp Phe Val Asp Ile Leu Leu Ser
260 265 270

Ile Glu Lys Asp Lys Asn Ser Gly Phe Gln Val Gln Arg Asn Asp Ile
275 280 285

Lys Phe Met Ile Leu Asp Met Phe Ile Gly Gly Thr Ser Thr Thr Ser
290 295 300

Thr Leu Leu Glu Trp Thr Met Thr Glu Leu Ile Arg Ser Pro Lys Ser
305 310 315 320

Met Lys Lys Leu Gln Asp Glu Ile Arg Ser Thr Ile Arg Pro His Gly
325 330 335

Ser Tyr Ile Lys Glu Lys Glu Val Glu Asn Met Lys Tyr Leu Lys Ala
340 345 350

Val Ile Lys Glu Val Leu Arg Leu His Pro Ser Leu Pro Met Ile Leu
355 360 365

Pro Arg Leu Leu Ser Glu Asp Val Lys Val Lys Gly Tyr Asn Ile Ala
370 375 380

Ala Gly Thr Glu Val Ile Ile Asn Ala Trp Ala Ile Gln Arg Asp Thr
385 390 395 400

Ala Ile Trp Gly Pro Asp Ala Glu Glu Phe Lys Pro Glu Arg His Leu
405 410 415

```
Asp Ser Gly Leu Asp Tyr His Gly Lys Asn Leu Asn Tyr Ile Pro Phe
                420                 425                 430

Gly Ser Gly Arg Arg Ile Cys Pro Gly Ile Asn Leu Ala Leu Gly Leu
            435                 440                 445

Ala Glu Val Thr Val Ala Asn Leu Val Gly Arg Phe Asp Trp Arg Val
        450                 455                 460

Glu Ala Gly Pro Asn Gly Asp Gln Pro Asp Leu Thr Glu Ala Ile Gly
465                 470                 475                 480

Ile Asp Val Cys Arg Lys Phe Pro Leu Ile Ala Phe Pro Ser Ser Val
                485                 490                 495

Val
```

## Beschreibung

Die vorliegende Erfindung betrifft neuartige Verfahren zur biokatalytischen Herstellung von Nitrilen aus Oximen unter Verwendung von Oxim-Dehydratasen, wie zur Herstellung von Citralnitril, Neralnitril, Geranialnitril oder Citronellylnitril aus Citraloxim, Neraloxim, Geranialoxim beziehungsweise Citronellaloxim. Dafür einsetzbare Oxim-Dehydratasen, Nukleotidsequenzen dafür sowie diese umfassende Expressionskonstrukte oder Mikroorganismen sind ebenfalls offenbart.

### Hintergrund der Erfindung

Terpene sind organische Verbindungen, die sich vom Isopren-Grundgerüst ableiten und in der Natur insbesondere als sekundäre Inhaltsstoffe von Organismen, insbesondere als sekundäre Pflanzenstoffe vorkommen. Terpene sind oftmals Geruchs- oder Geschmacksstoffe, und können beispielsweise aus ätherischen Ölen pflanzlichen Ursprungs gewonnen werden. Entsprechend werden Terpene und Terpenderivate als Inhaltstoffe für Produkte im Bereich der Kosmetik, der Körperpflege oder für Reinigungsprodukte verwendet, etwa als beigemischte Riech- oder Aromastoffe Parfüme, Körperlotionen, Duschgele, Seifen, Waschmittel oder Reinigungsmittel. Je nach verwendeter Verbindung können vielfältige Geruchsnoten erzeugt werden, etwa würzige, frische, citrusartige oder blumige Düfte der entsprechenden Produkte.

Die entsprechenden Ausgangsprodukte, die von der chemischen Industrie für die Beimischung zu entsprechenden Produkten angeboten werden, sind häufig Nitrile der Terpene oder Terpenderivate. Beispiele dafür sind Geranylnitril, welches einen intensiven frischen und citrusartigen Geruch aufweist, oder Citronellylnitril, welches rosenartig duftet.

Die entsprechenden Nitrile werden entweder als Naturprodukte isoliert oder aus geeigneten Vorstufen chemisch synthetisiert. So beschreibt die WO 2006/106141 der Anmelderin die Herstellung von gesättigten Nitrilen aus ungesättigten Nitrilen als Vorstufen. Die EP 0 491 127 beschreibt die Addition von ungesättigten Nitrilen an Hexenole unter Erhalt modifizierter Nitrile. In beiden Fällen werden Ausgangsverbindungen eingesetzt, welche bereits die Nitrilgruppe umfassen. Wünschenswert sind dementsprechend Verfahren, mit denen auch von Nitrilen abweichende Vorstufen verwendet werden können. Die DE 3139358 beschreibt ein Verfahren zur Erzeugung von Duftstoffen durch chemische Umsetzung von Oximen zu entsprechenden Nitrilen. Im Rahmen dieses Verfahrens werden für die erforderliche Dehydratisierung extreme Reaktionsbedingungen benötigt, insbesondere ätzende Dehydratisierungsreagenzien und Siedetemperaturen.

KATO, Y. und ASANO, Y beschreiben in Bioscience, Biotechnology, and Biochemistry, 2005, 69, 11, 2254-2257 ein Verfahren zur Herstellung von gesättigten $C_3$-$C_6$-Alkylnitrilen, wobei man die entsprechenden gesättigten $C_3$-$C_6$-Alkyloxime in Gegenwart der Phenylacetaldoxim Dehydratase aus *Gibberella zeae* umsetzt.

Xie, S.X. et al. beschreiben in, Biochemistry, 2003, 42, 41, 12056-12066 ein Verfahren zur Herstellung von gesättigten $C_3$-$C_6$-Alkylnitrilen, wobei man die entsprechenden gesättigten $C_3$-$C_6$-Alkyloxime in Gegenwart der aliphatischen Aldoxim Dehydratase aus Rhodococcus globerulus Stamm A-4 umsetzt.

Pedras M.S.C. et al. beschreiben in Phytochemistry, 2010, 71, 17-18, 1952-1962, die Indolacetaldoxim Dehydratase aus Sclerotinia sclerotiorum.

WO 2010/044031 A1 beschreibt ein Verfahren zur Herstellung des ungesättigten $C_{11}$-Alkylnitrils Methylcitronellalnitril aus Methylcitronellaldoxim durch chemische Synthese.

Es besteht somit ein Bedarf an alternativen Verfahren zur Herstellung von Nitrilen aus Oximen, insbesondere Verfahren, die unter sanfteren Reaktionsbedingungen verlaufen. Weiterhin besteht ein Bedarf an geeigneten Reaktionsmitteln zur Durchführung eines solchen Verfahrens.

### Kurzfassung der Erfindung

Obige erste Aufgabe wird erfindungsgemäß durch ein biokatalytisches Verfahren zur Herstellung von Nitrilen wobei man

a) ein Oxim, ausgewählt unter Citraloxim, Neraloxim, Geranialoxim, Citronellaloxim und teil- oder vollhydrierten Analoga davon, wobei die Verbindung in stereoisomerenreiner Form oder als Gemisch von Stereoisomeren vorliegt, in Gegenwart einer aliphatische Aldoxim Dehydratase (EC 4.99.1.5) oder einer Phenylacetaldoxim Dehydratase (PAOx) (EC 4.99.1.7) zum Nitril umsetzt, wobei das Nitril in stereoisomerenreiner Form oder als Gemisch von Stereoisomeren vorliegt, und
b) gegebenenfalls das Reaktionsprodukt anschließend weiter reinigt.

Obige zweite Aufgabe konnte durch Bereitstellung von hierin definierten Nukleinsäuresequenzen gelöst werden,

die für Proteine kodieren, die im Rahmen des erfindungsgemäßen Verfahrens einsetzbar sind.

Figurenbeschreibung

**Fig. 1** zeigt schematisch die Umsetzung von Citronellaloxim zu Citronellylnitril.
**Fig. 2A:** GC-Chromatogramm von E/Z-Citronellaloxim vor der biokatalytischen Umsetzung mit PAOx (RT: 7,816 & 8,018 min).
**Fig. 2B:** GC-Chromatogramm von E/Z-Citronellaloxim während der biokatalytischen Umsetzung mit PAOx (Zeit: 18 h). Citronellylnitril ist als Produkt der Umsetzung nachweisbar (RT: 6,765 min).
**Fig. 2C:** GC-Chromatogramm von Citronellylnitril, das während der biokatalytischen Umsetzung mit PAOx gebildet wurde (Zeit: 90 h). E/Z-Citronellaloxim wurde vollständig zum Nitril umgesetzt.

## Detaillierte Beschreibung der Erfindung

### A. Allgemeine Definitionen

"Aldoxim-Dehydratasen" im Sinne der vorliegenden Erfindung sind allgemein Enzyme bzw. Enzymmutanten, welche die Wasserabspaltung von Oximen der allgemeinen Formel $R^1R^2C{=}N\text{-}OH$ katalyiseren, wobei $R^1$ für einen beliebigen organischen Rest und $R^2$ für Wasserstoff steht. Der Begriff der Aldoxim-Dehydratasen umfasst weitere, genauer spezifizierte Aldoxim-Dehydratasen, wie etwa aliphatische Aldoxim-Dehydratase (EC 4.99.1.5, gemäß der EC-Nomenklatur des "Nomenciature Committee of the International Union of Biochemistry and Molecular Biology"), oder Phenylacetaldoxim-Dehydratase (PAOx) (EC 4.99.1.7).
Aufgrund der Reversibilität enzymatischer Reaktionen betrifft die vorliegende Erfindung die hierin beschriebenen enzymatischen Umsetzungen in beiden Umsetzungsrichtungen.
"Funktionale Mutanten" einer Aldoxim-Dehydratase umfassen die unten definierten "funktionalen Äquivalente" solcher Enzyme.
Der Begriff "biokatalytisches Verfahren" betrifft jegliches in Gegenwart von katalytischer Aktivität einer erfindungsgemäßen "Aldoxim-Dehydratase" bzw. eines Enzyms mit "Aldoxim-Dehydratase-Aktivität" durchgeführtes Verfahren, d.h. Verfahren in Gegenwart von rohem, oder gereinigtem, gelöstem, dispergiertem oder immobilisiertem Enzym, oder in Gegenwart ganzer mikrobieller Zellen, welche derartige Enzymaktivität aufweisen oder exprimieren. Biokatalytische Verfahren umfassen somit enzymatische als auch mikrobielle Verfahren.
Der Begriff "stereoisomerenrein" bedeutet, dass eines von mehreren möglichen Stereoisomeren einer im Rahmen der Erfindung genannten Verbindung mit wenigstens einem Asymmetriezentrum in hohem "Enatiomerenüberschuß" oder hoher "Enantiomerenreinheit", wie z.B. wenigstens 90%ee, insbesondere wenigstens 95 %ee, oder wenigstens 98 %ee, oder wenigstens 99 %ee vorliegt. Der ee% Wert wird nach folgender Formel berechnet:

$$ee\% = [X_A - X_B]/[X_A + X_B]*100,$$

worin $X_A$ und $X_B$ für den Molenbruch der Enantiomere A bzw B stehen.
Unter einer "Aldoxim-Dehydratase-Aktivität", die mit einem "Referenzsubstrat unter Standardbedingungen" bestimmt wurde, ist z.B. eine Enzymaktivität zu verstehen, welche die Bildung eines Nitril-Produkts aus einem Oxim-Substrat beschreibt. Standardbedingungen sind z.B. Substratkonzentrationen von 10 mM bis 0,2 M, insbesondere 15 bis 100mM, wie z.B. etwa 20 bis 25 mM; bei pH 4 bis 8, und bei Temperaturen von z.B. 15 bis 30 oder 20 bis 25 °C. Die Bestimmung kann dabei mit rekombinanten Aldoxim-Dehydratase-exprimierenden Zellen, aufgeschlossenen Aldoxim-Dehydratase-exprimierenden Zellen, Fraktionen davon oder angereichertem oder gereinigtem Aldoxim-Dehydratase-Enzym erfolgen. Insbesondere ist das Referenzsubstrat Citronellaloxim, oder ein Citronellaloxim-Racemat, wie auch in den Bespielen näher beschrieben. Ein spezifischer Standard-Assay zum Nachweis der Aldoxim-Dehydratase-Aktivität ist beispielsweise von Kato, Y. et al. in Biochemistry 2000, 39, 800-809 beschrieben. Danach enthält ein Standardansatz 50 μMol Kaliumphosphatpuffer, pH 7,0, 125 nMol FMN, 2,5 μMol Enzym. Die Reaktion wird nach 10 min durch Zugabe von 500 μL 0,5 M $H_3PO_4$ abgestoppt, der durch Zentrifugation (18.000 g, 10 min) gewonnene Überstand wird dann auf gebildetes Produkt analysiert.
"Terpene" sind Kohlenwasserstoffe die aus Isopreneinheiten (C5-Einheiten) aufgebaut sind, insbesondere nicht-cyclische Terpene, wie z.B. Squalen, der Kohlenstoffzahl durch 5 teilbar ist.
"Terpenoide" sind Substanzen, die von Terpenen, insbesondere nicht-cyclischen Terpenen, abgeleitet sind,

z.B. durch zusätzliche Einfügung von C-Atomen und/ oder Heteroatomen, wie z.B. Citronellal.

"Terpen-ähnliche" Verbindungen im Sinne der vorliegenden Erfindung umfassen insbesondere solche Verbindungen, welche unter die allgemeine Strukturformel (IV), wie im Folgenden definiert, fallen.

Generell mit umfasst sind erfindungsgemäß alle isomeren Formen der hierin beschriebenen Verbindungen, wie Konstitutionsisomere und insbesondere Stereoisomere und Gemische davon, wie z.B. optische Isomere oder geometrische Isomere, wie E- und Z-Isomere, sowie Kombinationen davon. Sind mehrere Asymmetriezentren in einem Molekül vorhanden, so umfasst die Erfindung sämtliche Kombinationen von unterschiedlichen Konformationen dieser Asymmetriezentren, wie z.B. Enantiomerenpaare.

Sofern keine abweichenden Angaben gemacht werden gelten hierin folgende allgemeine chemische Definitionen:

Aliphatische Kohlenwasserstoffreste umfassen Reste, die abgeleitet sind aus acyclischen oder cyclischen, gesättigten oder ungesättigten Kohlenstoffverbindungen mit Ausnahme von aromatischen Verbindungen. Aliphatische Kohlenwasserstoffreste umfassen insbesondere geradkettige oder verzweigte Alkylreste und geradkettige oder verzweigte Alkenylreste. Nicht-limitierende Beispiele sind nachfolgend aufgezählt:

Alkyl sowie alle Alkylteile in davon abgeleiteten Resten, wie z.B. Hydroxyalkyl: gesättigte, geradkettige oder einfach oder mehrfach, wie z.B. 1-, 2-, 3- oder 4-fach verzweigte Kohlenwasserstoffreste mit 1 bis 4, 1 bis 6, 1 bis 8, 1 bis 10 oder 4 bis 10 Kohlenstoffatomen, z. B.

- $C_1$-$C_{10}$-Alkyl oder $C_1$-$C_6$-Alkyl oder $C_4$-$C_{10}$-Alkyl:: wie Methyl, Ethyl, Propyl, 1-Methylethyl, Butyl, 1-Methylpropyl, 2-Methylpropyl, und 1,1-Dimethylethyl als beispielhafte Vertreter für $C_1$-$C_4$-Alkyl; sowie Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, wie insbesondere die aus Tabelle 4 entnehmbaren Bedeutungen von R, wie z.B. Hexyl
- Hydroxy-$C_1$-$C_{10}$-alkyl, wie z.B. Hydroxy-$C_1$-$C_6$-alkyl, oder Hydroxy-$C_1$-$C_4$-Alkyl, oder Hydroxy-$C_4$-$C_{10}$-Alkyl: wie z.B. Hydroxymethyl, 1-oder 2-Hydroxyethyl, 1-, 2- oder 3-Hydroxypropyl, 1-Hydroxymethylethyl, 1-, 2-, 3- oder 4-Hydroxybutyl, 1-Hydroxymethylpropyl und 2-Hydroxymethylpropyl. oder andere hydroxysubstituierte Analoga obiger Alkylreste, wie insbesondere die aus Tabelle 4 entnehmbaren Bedeutungen von R, wie z.B. 6-Hydroxy-6-methyl-hept-1-yl.
- Alkenyl steht für ein- oder mehrfach, insbesondere einfach oder zweifach ungesättigte, geradkettige oder einfach oder mehrfach, wie z.B. 1-, 2-, 3- oder 4-fach verzweigte Kohlenwasserstoffreste mit 2 bis 4, 2 bis 6, 2 bis 8, 2 bis 10, 4 bis 10, 2 bis 20 oder 4 bis 20 Kohlenstoffatomen und einer Doppelbindung in einer beliebigen Position, oder 2 nichtkumulierte, konjugierte oder insbesondere nicht-konjugierte Doppelbindungen, z. B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, wie insbesondere die aus Tabelle 4 entnehmbaren Bedeutungen von R, wie 2,6-Dimethyl-hepta-1,5-dien-1-yl, 2,6-Dimethyl-hept-5-en-1-yl, 2,6-Dimethyl-octa-1,5-dien-1-yl, Octa-1,5-dien-1-yl, 1,5-Dimethyl-hexa-4-en-1-yl, 1,5,9-Trimethyl-non-8-en-1-yi

"Oxo" steht z.B. für einen Rest, der zusammen mit dem C-Atom an das er gebunden ist eine Ketogruppe (C=O) bildet. Dementsprechend sind beispielsweise abgeleitete Reste vorgenannter Kohlenwasserstoffreste solche, die eine oder mehrere Ketogruppen tragen.

"Methylen" (=$CH_2$) steht z.B. für einen Rest, der zusammen mit dem C-Atom an das er gebunden ist einen

Vinylrest ($-CH=CH_2$) bildet.

B. Spezielle Ausgestaltungen der Erfindung

Die vorliegende Erfindung betrifft insbesondere folgende spezielle Ausführungsformen:

1. Biokatalytisches Verfahren zur Herstellung von Nitrilen
wobei man ein Oxim, ausgewählt unter Citraloxim, Neraloxim, Geranialoxim, Citronellaloxim und teil- oder vollhydrierten Analoga davon, wie etwa 6,7-Dihydrocitronellyloxim, wobei die Verbindung in stereoisomerenreiner Form oder als Gemisch von Stereoisomeren vorliegt,
in Gegenwart einer aliphatischen Aldoxim Dehydratase (E.C. 4.99.1.5) oder einer Phenylacetaldoxim Dehydratase (PAOx) (EC 4.99.1.7), wie beispielsweise eines Enzyms umfassend eine Aminosäuresequenz ausgewählt unter SEQ ID NO: 1 und 4 bis 27, oder eine zu einer dieser Sequenzen zu mindestens 60%, wie z.B. mindestens 65, 70, 75, 80, 85, 90, 91, 92, 93, 94, 95, 96, 97, 98, oder 99% identische Aminosäuresequenz aufweist; insbesondere einer Phenylacetaldoxim Dehydratase (PAOx) (EC 4.99.1.7), insbesondere einem Enzym unfassend SEQ ID NO:1 oder eine dazu zu mindestens 60%, wie z.B. mindestens 65, 70, 75, 80, 85, 90, 91, 92, 93, 94, 95, 96, 97, 98, oder 99% identische Aminosäuresequenz aufweist; zum Nitril umsetzt, wobei das Nitril in stereoisomerenreiner Form oder als Gemisch von Stereoisomeren vorliegt, und b) gegebenenfalls das Reaktionsprodukt anschließend weiter reinigt.

2. Verfahren nach Ausführungsform 1, wobei die PAOx ein Enzym aus *Rhodococcus* sp, *Gibberella zeae* oder insbesondere *Bacillus* sp. ist.

3. Verfahren nach einer der vorstehend genannten Ausführungsformen, wobei die PAOx eine Aminosäuresequenz gemäß SEQ ID NO: 1 oder eine dazu zu mindestens 60%, wie z.B. mindestens 65, 70, 75, 80, 85, 90, 91, 92, 93, 94, 95, 96, 97, 98, oder 99% identische Aminosäuresequenz aufweist; oder wobei bis zu 25%, wie z.B. bis zu 20, 15, 10, 9, 8, 7, 6, 5, 4, 3, 2 oder 1% der Aminosäurereste gegenüber SEQ ID NO:1 durch Addition, Deletion, Insertion, Substitution, Inversion oder einer Kombination davon verändert sind, und die noch mindestens 50%, wie z.B. mindestens 60, 65, 70, 75, 80, 85, 90 oder 95% der enzymatischen Aktivität von SEQ ID NO:1 aufweist.

4. Verfahren nach einer der vorstehend genannten Ausführungsformen, wobei man Citronellaloxim der Formel IIa

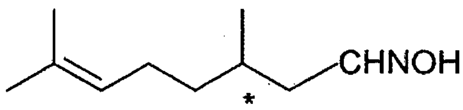
(IIa)

zu Citronellylnitril der Formel Ia

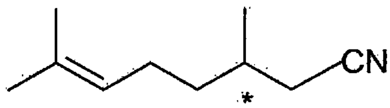
(Ia)

umsetzt, wobei man die Verbindung der Formel IIa, in stereoisomerenreiner Form oder als Stereoisomerengemisch, wie insbesondere als R/S- und/oder E/Z-Gemisch, insbesondere ein R/S- und E/Z-Gemisch einsetzt.

5. Verfahren nach einer der vorstehend genannten Ausführungsformen, wobei die Umsetzung enzymatisch, in Gegenwart von PAOx, einem PAOx enthaltenden Proteingemisch, oder in Gegenwart eines rekombinanten, PAOx funktional exprimierenden Mikroorganismus, eines davon abgeleiteten PAOx-haltigen Zellhomogenats oder einer PAOx-haltigen Fraktion davon erfolgt.

6. Verfahren nach einer der vorstehend genannten Ausführungsformen, wobei PAOx oder der PAOx funktional exprimierende Mikroorganismus im Reaktionsgemisch frei oder in immobilisierter Form vorliegt.

7. Verfahren nach einer der Ausführungsformen 5 und 6, wobei der rekombinante Mikroorganismus ein PAOx funktional exprimierender Bakterienstamm, insbesondere E. coli-Stamm, ist.

8. Verfahren nach Ausführungsform 7, wobei der rekombinante Mikroorganismus ein Expressionskonstrukt trägt, das die kodierende PAOx Nukleotidsequenz, gegebenenfalls angepasst an die Codonnutzung des Mikroorganismus, aufweist.

9. Verfahren nach einer der Ausführungsformen 5 bis 8, wobei der Mikroorganismus zusätzlich wenigstens ein die funktionale Expression (insbesondere korrekte Faltung) der PAOx unterstützendes Chaperon exprimiert.

10. Verfahren nach Ausführungsform 9, wobei der rekombinante Mikroorganismus ein Bakterienstamm ist, der zusätzlich zu PAOx ein oder mehrere Chaperone ausgewählt unter GroEL und-GroES exprimiert.

11. Verfahren nach einer der vorhergehenden Ausführungsformen, wobei man die Umsetzung in, insbesondere reinem Substrat, d.h. ohne weitere Zugabe von üblichen Zusätzen, wie Wasser oder Puffer, als Reaktionsmedium durchführt, wobei hierbei insbesondere ganze, das Dehydratase-Enzym exprimierende Zellen eingesetzt werden.

Darüber hinaus sind die folgendenden Nukleotidsequenzen, Expressionskonstrukte und rekombinante Mikroorganismen offenbart:

12. Nukleotidsequenz gemäß SEQ ID NO: 2 oder 3, kodierend für PAOx, wobei die Nukleotidsequenz angepasst ist an die Codonnutzung des Mikroorganismus Escherichia coli.

13. Expressionskonstrukt gemäß der Definition in einer der Ausführungsformen 8 bis 11 oder umfassend eine Nukleotidsequenz, wie unter 12. offenbart.

14. Rekombinanter Mikroorganismus gemäß der Definition in einer der vorstehend genannten Ausführungsformen 5 bis 11, oder umfassend eine Nukleotidsequenz, wie unter 12. offenbart, oder ein Expressionskonstrukt, wie unter 13. offenbart.

C. Weitere Ausgestaltungen der Erfindung

1. Proteine/Enzymmutanten

Die vorliegende Erfindung ist nicht auf die hierin konkret offenbarten Enzyme mit Aldoxim-Dehydratase-Aktivität, beschränkt, sondern erstreckt sich vielmehr auch auf funktionale Äquivalente davon.

"Funktionale Äquivalente" oder Analoga der konkret offenbarten Enzyme und Enzymmutanten sind im Rahmen der vorliegenden Erfindung davon verschiedene Polypeptide, welche weiterhin die gewünschte biologische Aktivität, Aldoxim-Dehydratase-Aktivität, besitzen.

So versteht man beispielsweise unter "funktionalen Äquivalenten" Enzyme und Mutanten, die in einem verwendeten Test auf "Aldoxim-Dehydratase-Aktivität" im Sinne der Erfindung (d.h. mit einem Referenzsubstrat unter Standardbedingungen) eine um mindestens 1%, insbesondere um mindestens etwa 5 bis 10 %, wie z.B. mindestens 10% oder mindestens 20 %, wie z.B. mindestens 50 % oder 75% oder 90 % höhere oder niedrigere Aktivität eines Enzyms, umfassend eine hierin konkret definierte Aminosäuresequenz (z.B. einer Mutante, abgeleitet von SEQ ID NO:1 oder abgeleitet von einer der Sequenzen gemäß SEQ ID NO: 4 bis 27) aufweisen.

Die Aktivitätsangaben für funktionale Äquivalente beziehen sich hierin, wenn nichts anderes angegeben ist, auf Aktivitätsbestimmungen, durchgeführt mittels eines Referenzsubstrates unter Standardbedingungen, wie hierin definiert.

Die "Aldoxim-Dehydratase-Aktivität" im Sinne der Erfindung kann mit Hilfe verschiedener bekannter Tests nachgewiesen werden. Ohne darauf beschränkt zu sein, sei ein Test unter Verwendung eines Referenzsubstrates, wie z. B. Citronellaloxim oder Citronellaloxim-Racemat, unter Standardbedingungen, wie oben beschrieben (vgl. auch Kato et al, Biochemistry, 2000, 39, 800-809; beschrieben für PAOx; und analog auf andere Aldoxim-Dehydratasen übertragbar bzw. anpassbar) und im experimentellen Teil erläutert, zu nennen.

Funktionale Äquivalente sind außerdem z.B. zwischen pH 4 bis 11 stabil und besitzen vorteilhaft ein pH-Optimum in einem Bereich von pH 5 bis 10, wie insbesondere 6,5 bis 9,5 oder 7 bis 8 oder etwa bei 7,5, sowie ein Temperaturoptimum im Bereich von 15°C bis 80°C oder 20°C bis 70°C, wie z.B. etwa 30 bis 60°C oder etwa 35 bis 45°C, wie etwa bei 40°C.

"Funktionale Äquivalente" umfassen die durch eine oder mehrere, wie z.B. 1 bis 50, 2 bis 30, 2 bis 15, 4 bis 12 oder 5 bis 10 "zusätzliche Mutationen", wie Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere

auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Derartige "zusätzliche Mutationen" können dabei an jeder beliebigen Position der jeweiligen Aminosäuresequenz erfolgen.

Nichtlimitierende Beispiele für geeignete Aminosäuresubstitutionen sind in folgender Tabelle zusammengefasst:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne die gewünschte biologische Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäß mit umfasste "funktionale Äquivalente" sind Homologe zu den konkret offenbarten Proteinen.

Diese besitzen wenigstens 60 %, vorzugsweise wenigstens 75% ins besondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der Proteine oder Polypeptide können durch Mutagenese erzeugt werden, z.B. durch Punktmutation, Verlängerung oder Verkürzung des Proteins.

Homologe der Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

## 2. Nukleinsäuren und Konstrukte

### 2.1 Nukleinsäuren

Offenbart sind auch Nukleinsäuresequenzen, die für ein wie oben beschriebenes Enzym bzw. eine oben beschriebenen Mutante davon mit Aldoxim-Dehydratase-Aktivität kodieren.

Ebenfalls offenbart sind Nukleinsäuren mit einem bestimmten Identitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |

(fortgesetzt)

| | |
|---|---|
| Hydrophilic residue gap | off |
| Transition weighting | 0 |

Pairwise alignment parameter:

| | |
|---|---|
| FAST algorithm | on |
| K-tuple size | 1 |
| Gap penalty | 3 |
| Window size | 5 |
| Number of best diagonals | 5 |

Alternativ dazu kann die Identität auch nach Chenna, Ramu, Sugawara, Hideaki, Koike, Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Intemetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 898-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Offenbart sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Offenbart sind sowohl isolierte Nukleinsäuremoleküle, welche für Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von vorliegend offenbarten kodierenden Nukleinsäuren verwendet werden können.

Die Nukleinsäuremoleküle können zudem untranslatierte Sequenzen vom 3'- und/oder 5'-Ende des kodierenden Genbereichs enthalten.

Offenbart sind außerdem die zu den konkret beschriebenen Nukleotidsequenzen komplementären Nukleinsäuremoleküle oder einen Abschnitt davon.

Die Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinander folgende Nukleotide eines Sense-Stranges einer vorliegend offenbarten Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder

anderen Chemikalien sein, wenn es chemisch synthetisiert wird.

Ein vorliegend offenbartes Nukleinsäuremolekül kann mittels molekularbiologischer Standard-Techniken und der bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die Oligonukleotide können ferner durch Standard-Syntheseverfahren, z.B. mit einem automatischen DNA-Synthesegerät, hergestellt werden.

Vorliegend offenbarte Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den vorliegend offenbarten Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der vorliegend offenbarten Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Harnes and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Offenbart sind auch Derivate der konkret offenbarten oder ableitbaren Nukleinsäuresequenzen.

So können weitere vorliegend offenbarte, für Aldoxim-Dehydratase kodierende Nukleinsäuresequenzen z.B. von SEQ ID NO:2 oder SEQ ID NO:3 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, wie etwa SEQ ID NO:3, die ausgehend von SEQ ID NO:2 hinsichtlich der Codon-Nutzung von *E. coli* optimiert ist, ebenso wie natürlich vorkommende Varianten, wie z.B. Spleißvarianten oder Allelvarianten, davon.

Offenbart sind ebenso durch konservative Nukleotidsubstutionen (d.h. die betreffende Aminosäure wird durch eine Aminosäure gleicher Lädung, Größe, Polarität und/öder Löslichkeit ersetzt) erhältliche Sequenzen.

Offenbart sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der offenbarten, für Aldoxim-Dehydratase kodierenden Nukleinsäuresequenzen abgeleitet von Sequenz SEQ ID NO: 3 oder von einer der kodierenden Sequenzen zu SEQ ID NO:1, sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der offenbarten Nukleinsäuresequenzen, beispielsweise pilzliche oder bakterielle Homologe, verkürzte Sequenzen, Einzelstrang-DNA oder RNA der kodierenden und nichtkodierenden DNA-Sequenz, zu verstehen.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertion, Inversion und/oder Deletion verändert sein, ohne dass aber die Funktionalität bzw. Wirksamkeit der Promotoren beeinträchtigt sind. Des Weiteren können die Promotoren durch Veränderung ihrer Sequenz in ihrer Wirksamkeit erhöht oder komplett durch wirksamere Promotoren auch artfremder Organismen ausgetauscht werden.

### 2.2 Generierung funktionaler Mutanten

Dem Fachmann sind darüber hinaus Verfahren zur Erzeugung funktionaler Mutanten der Enzyme bekannt.

Je nach verwendeter Technik kann der Fachmann völlig zufällige oder auch gezieltere Mutationen in Gene oder auch nicht kodierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der von diesen kodierten Protein sind dem Fachmann seit langem geläufig, wie beispielsweise

- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcárel R, Stunnenberg HG (1994) Nucleic Acids Res 22:541; Barik S (1995) Mol Biotechnoi 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739),
- die SeSaM-Methode (Sequence Saturation Method), bei der bevorzugte Austausche durch die Polymerase verhindert werden (Schenk et al., Biospektrum, Vol. 3, 2006, 277-279),
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey) oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der so genannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und

auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise kann stufenweise eine begrenzte Anzahl von Mutationen, wie z.B. 1, 2, 3, 4 oder 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die Ergebnisse liefern auch wichtige Informationen in Bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

Ebenfalls sind Informationen ableitbar bezüglich Aminosäure-Sequenzpositionen, in deren Bereich Mutationen durchgeführt werden können, die voraussichtlich wenig Einfuß auf die Enzymaktivität haben sollten, und als potentielle "silent mutations" bezeichnet werden können.

2.3 Konstrukte

Offenbart sind außerdem, insbesondere rekombinante, Expressionskonstrukte, enthaltend unter der genetischen Kontrolle regulativer Nukleinsäuresequenzen eine für ein Polypeptid kodierende Nukleinsäuresequenz; sowie, insbesondere rekombinante, Vektoren, umfassend wenigstens eines dieser Expressionskonstrukte.

Unter einer "Expressionseinheit" wird eine Nukleinsäure mit Expressionsaktivität verstanden, die einen Promotor, wie hierin definiert umfasst, und nach funktioneller Verknüpfung mit einer zu exprimierenden Nukleinsäure oder einem Gen die Expression, also die Transkription und die Translation dieser Nukleinsäure oder dieses Gens, reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator; derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierurigssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Vorliegend offenbarte Nukleinsäurekonstrukte umfassen insbesondere eine für eine Aldoxim-Dehydratase-Mutante kodierende Sequenz, insbesondere eine von der für eine PAOx gemäß SEQ ID NO:1 kodierenden Nukleinsäureequenz abgeleitete und an die Kodonnutzung von *E. coli* angepaßte SEQ ID NO: 3 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die vorliegend offenbarten Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt, enthalten sein.

Beispiele geeigneter Regutationssequenzen sind in Promotoren wie cos-, tac-, trp-, tet-, trp-tet-, Ipp-, lac-, Ipp-lac-, $lacI^{q-}$, T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha, AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, $pIN\text{-}III^{113}$-B1, Agt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen $pLGV23$, $pGHlac^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

In einer weiteren Ausgestaltungsform des Vektors kann der das Nukleinsäurekonstrukt oder die Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor, wie einem Plasmid, oder nur aus dem Nukleinsäurekonstrukt oder der Nukleinsäure bestehen.

Für eine optimale Expression heterologer Gene in Organismen ist es vorteilhaft, die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer vorliegend offenbarten Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im

Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 3. Mikroorganismen

Je nach Zusammenhang kann unter dem Begriff "Mikroorganismus" der Wildtyp-Mikroorganismus oder ein genetisch veränderter, rekombinanter Mikroorganismus oder beides verstanden werden.

Mit Hilfe der vorliegend offenbarten Vektoren sind rekombinante Mikroorganismen herstellbar, welche beispielsweise mit wenigstens einem vorliegend offenbarten Vektor transformiert sind und zur Produktion der Polypeptide eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die offenbarte Nukleinsäure oder den Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen enthalten dabei vorzugsweise mindestens eine der hierin offenbarten Nukleinsäuresequenzen, Nukleinsäurekonstrukte oder Vektoren, die für ein Enzym mit Phenylethanol Dehydrogenase-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

### 4. Rekombinante Herstellung von Enzymen

Offenbart sind weiterhin Verfahren zur rekombinanten Herstellung der Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) kultiviertwerden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen werden. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, T. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind

beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### 5. Enzymimmobilisierung

Die Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenol-formaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol.III, 991-1032, Wiley-VCH, Weinheim beschrieben. Weitere Informationen zu Biotransformationen und Bioreaktoren zur Durchführung erfindungsgemäßer Verfahren findet man z.B. auch in Rehm et al (Ed) Biotechology, 2nd Edn, Vol 3, Chapter 17, VCH, Weinheim.

### 6. Biokatalytische Herstellung von Nitrilen

Das erfindungsgemäße biokatalytische Verfahren zur Herstellung von Nitrilen aus entsprechenden Oximen wird durchgeführt in Gegenwart, einer aliphatischen Aldoxim Dehydratase (4.99.1.5) oder einer Phenylacetaldoxim Dehydratase (PAOx) (EC 4.99.1.7). Beispiele solcher Enzyme umfassen wenigstens eine Aminosäuresequenz gemäß SEQ ID NO: 1 oder 4 bis 27, oder eine dazu zu mindestens 60%, wie z.B. mindestens 65, 70, 75, 80, 85, 90, 91, 92, 93, 94, 95, 96, 97, 98, oder 99% identische Aminosäuresequenz; oder wobei bis zu 25%, wie z.B. bis zu 20, 15, 10, 9, 8, 7, 6, 5, 4, 3, 2 oder 1% der Aminosäurereste gegenüber einer der Sequenzen gemäß SEQ ID NO:1 oder 4 bis 27 durch Addition, Deletion, Insertion, Substitution, Inversion oder einer Kombination davon verändert sind, und die noch mindestens 50%, wie z.B. mindestens 60, 65, 70, 75, 80, 85, 90 oder 95% der enzymatischen Aktivität von SEQ ID NO:1 aufweist.

Insbesondere wird das erfindungsgemäße Verfahren in Gegenwart eines Enzyms durchgeführt, wobei das Enzym mit der Proteinsequenz gemäß SEQ ID NO:1 aufweist. Insbesondere ist das Enzym von einer Nukleinsäuresequenz gemäß SEQ ID NO:2 oder einem funktionalen Äquivalent davon, insbesondere einem funktionellen Äquivalent gemäß SEQ ID NO:3 kodiert, welches eine für die Expression in einem Mikroorganismus E. coli In kodonoptimierte Nukleinsäuresequenz darstellt. Dabei ist die Nukleinsäuresequenz insbesondere Bestandteil eines Genkonstrukts oder Vektors. Solche Genkonstrukte oder Vektoren werden ausführlich in der internationalen Anmeldung PCT/EP2010/057696 auf den Seiten 16 bis 20 beschrieben, worauf hier ausdrücklich Bezug genommen wird.

Die Wirtszelle, die ein Genkonstrukt oder einen Vektor enthält, worin die Nukleinsäuresequenz enthalten ist, die das Enzym mit der gewünschten Aktivität kodiert, bezeichnet man auch als transgenen Organismus. Die Herstellung solche transgenen Organismen ist prinzipiell bekannt und wird beispielsweise in internationalen Anmeldung PCT/EP2010/057696 auf Seite 20 diskutiert.

Als transgene Organismen werden bevorzugt Zellen aus der Gruppe bestehend aus Bakterien, Cyanobakterien, Pilzen und Hefen ausgewählt. Bevorzugt ist die Zelle ausgewählt aus Pilzen der Gattung Pichia oder Bakterien der Gattungen Escherichia, Corynebacterium, Ralstonia, Clostridium, Pseudomonas, Bacillus, Zymomonas, Rhodobacter, Streptomyces, Burkholderia, Lactobacillus oder Lactococcus. Besonders bevorzugt ist die Zelle ausgewählt aus Bakterien der Spezies Escherichia coli, Pseudomonas putida, Burkholderia glumae, Streptomyces lividans, Streptomyces coelicolor oder Zymomonas mobilis.

Bevorzugt ist ein erfindungsgemäßes Verfahren, das dadurch gekennzeichnet ist, dass das Enzym mit der Aktivität einer aliphatischen Aldoxim Dehydratase (EC 4.99.1.5) oder einer Phenylacetaldoxim Dehydratase (hier auch als PAOx bezeichnet) (EC 4.99.1.7) von einem Gen kodiert wird, das aus einem Mikroorganismus isoliert wurde, ausgewählt unter Zymomonas mobilis, Methylococcus capsulatus, Rhodopseudomonas palustris, Bradyrhizobium japonicum, Frankia spec, Streptomyces coelicolor, Acetobacter pasteurianus sowie Absidia sp., wie etwa A. corymbifera; Agrobacterium sp., wie etwa A.radiobacter; Alcaligenes sp., wie etwa A.faecalis; Arthrobacter sp., wie etwa A.crystallopoietes oder A. ramosus; Aspergillus sp., wie etwa A. amstelodami, A.celluosae; A.candidus oder A.pulverulentus; Aureobacterium sp., wie etwa A.testaceum; Aureobasidium sp., wie etwa A.pullulans; Bacillus sp., wie etwa B. coagulans, B. megaterium, B. sp. OxB-1 oder B.subtilis; Botryotinia sp., wie etwa Botryotinia fuckeliana; Brevibacterium sp., wie etwa B.butanicum; Cellulomonas sp., wie etwa C.fimi; Coprinus Sp., wie etwa C.phlyctidosporus; Corynebacterium sp, wie etwa C.paurometabolum oder C.rathavi; Cunninghamella sp., wie etwa C.echinulata var. Elegans; Flammulina sp., wie etwa F.sp. Stamm TPU 4675 oder F.velutipes; Flavobacterium sp., wie etwa F.aquatile, F.lutescens, F.rigense oder F. suaveolens; Fusarium sp., wie etwa F.culmorum, F.oxysporum f. sp. Nicotianae oder F. solani var. martii; Giberella sp., wie etwa Gibberella fujikuroi oder Gibberella zeae; Keratinomyces sp., wie etwa K.ajelloi; Klebsiella sp., wie etwa K.pneumoniae; Leptosphaeria sp., wie etwa Leptosphaeria maculans; Micrococcus sp.; wie etwa M.luteus oder M.ureae; Mortierella sp., wie etwa M.isabellina; M. ramanniana var. Angulispora oder M.sp. TPU 4801; Mucor sp., wie etwa M:fragilis; Neosartorya sp. wie etwa N.fisheri; Nocardia sp., wie etwa N.asteroides; Phycomyces sp., wie etwa P.nitens; Proteus sp., wie etwa P.vulgaris; Pseudomonas sp., wie etwa Pseudomonas chioraphis oder P.fluorescens; Pycnoporos sp., wie etwa P.coccineus; Rhizoctonia sp., wie etwa Rhizoctonia solani; Rhizopus, wie etwa R.nigricans oder R.oryzae; Rhodococcus sp., wie etwa R.erythropolis oder R.rhodochrous; Schizophyllum sp., wie etwa S. commune oder S. sp. Stamm TPU 4435; Sclerotinia sp., wie etwa Sclerotinia sclerotiorum; Talaromyces sp., wie etwa T. flavus; Serratia sp., wie etwa S. marcescens; Stenotrophomonas sp., wie etwa S. maltophilia; Xanthomonas sp., wie etwa X. flavus. Besonders bevorzugt sind die betreffenden Gene, insbesondere das Gen für PAOx, aus Rhodococcus sp., Gibberella zeae oder insbesondere Bacillus sp. isoliert.

Beispiele für aliphatische Acetaldoxim-Dehydratasen, die im Rahmen eines erfindungsgemäßen Verfahrens geeignet einsetzbar sind oder deren Sequenzen als Ausgangspunkt für die Erzeugung funktionaler Äquivalente geeignet sind, sind in der nachfolgenden Tabelle 1 gezeigt. Die Zugangsnummern beziehen sich auf die fachbekannten Datenbanken REFSEQ und UNIPROT, wobei der Fachmann auf die über Zugangsnummern identifizierten Sequenzeinträge über intemetbasierte Dienste ohne Weiteres auch auf anderen Datenbanken wie etwa GeneBank oder SwissProt, zugreifen zu kann.

Tabelle 1:

| SEQ ID NO: | Zugangsnummer | Beschreibung | EC-Nr. | Organismus |
|---|---|---|---|---|
| 4 | YP_947647 (NCIB) | Aldoxim-Dehydratase | 4.99.1.5 | Arthrobacter aurescens TC1 |
| 5 | Q6FBU3 | Aldoxim-Dehydratase; | 4.99.1.5 | Acinetobacter sp. Stamm ADP1 |

Beispiele für Phenylacetaldoxim-Dehydratasen, die im Rahmen eines erfindungsgemäßen Verfahrens geeignet einsetzbar sind oder deren Sequenzen als Ausgangspunkt für die Erzeugung funktionaler Äquivalente geeignet sind, sind in der nachfolgenden Tabelle 2 gezeigt:

Tabelle 2:

| **SEQ ID NO:** | **Zugangsnummer** | **Beschreibung** | **EC-Nr.** | **Organismus** |
|---|---|---|---|---|
| 6 | F0QD95_ACIAP | Phenylacetaldoxim-Dehydratase | 4.99.1.7 | Acidovorax avenae-Stamm ATCC 19860 DSM 7227 JCM 20985 NCPPB 1011 |
| 7 | YP_003097901 | Phenylacetaldoxim-Dehydratase | 4.99.1.7 | Actinosynnema mirum DSM 43827 |
| 8 | YP_001240697 | Vermutliche Phenylacetaldoxim-Dehydratase | 4.99.1.7 | Bradyrhizobium sp. BTAi1 |
| 9 | ZP_02891657 | Phenylacetaldoxim-Dehydratase | 4.99.1.7 | Burkholderia ambifaria IOP40-10 |
| 10 | YP_001816246 | Phenylacetaldoxim-Dehydratase [Burkholderia ambifaria MC40-6]. | 4.99.1.7 | Burkholderia ambifaria MC40-6 |

(fortgesetzt)

| SEQ ID NO: | Zugangsnummer | Beschreibung | EC-Nr. | Organismus |
|---|---|---|---|---|
| 11 | YP_001776877 | Phenylacetaldoxim-Dehydratase [Burkholderia cenocepacia MC0-3]. | 4.99.1.7 | Burkholderia cenocepacia MC0-3 |
| 12 | YP_004119277 | Phenylacetaldoxim-Dehydratase [Pantoea sp. At-9b]. | 4.99.1.7 | Pantoea sp. At-9b |
| 13 | ZP_07774756 | Phenylacetaldoxim-Dehydratase [Pseudomonas fluorescens WH6]. | 4.99.1.7 | Pseudomonas fluorescens WH6 |
| 14 | ZP_07774757 | Phenylacetaldoxim-Dehydratase [Pseudomonas | 4.99.1.7 | Pseudomonas fluorescens WH6 |
| | | fluorescens WH6]. | | |
| 15 | YP_001268042 | Phenylacetaldoxim-Dehydratase [Pseudomonas putida F1]. | 4.99.1.7 | Pseudomonas putida F1 |
| 16 | E4RFH2_PSEPB | Phenylacetaldoxim_ Dehydratase | 4.99.1.7 | Pseudomonas putida strain BIRD-1 |
| 17 | YP_001758607 | Phenylacetaldoxim-Dehydratase [Shewanella woodyi ATCC 51908]. | 4.99.1.7 | Shewanella woodyi ATCC 51908 |
| 18 | YP_001261493 | Phenylacetaldoxim-Dehydratase [Sphingomonas wittichii RW1]. | 4.99.1.7 | Sphingomonas wittichii RW1 |
| 19 | YP_004155682 | Phenylacetaldoxim-Dehydratase [Variovorax paradoxus EPS]. | 4.99.1.7 | Variovorax paradoxus EPS |
| 20 | YP_002942593 | Phenylacetaldoxim-Dehydratase [Variovorax paradoxus S110]. | 4.99.1.7 | Variovorax paradoxus S110 |
| 21 | YP_002944432 | Phenylacetaldoxim-Dehydratase [Variovorax paradoxus S110]. | 4.99.1.7 | Variovorax paradoxus S110 |
| 22 | YP_001416464 | Phenylacetaldoxim-Dehydratase [Xanthobacter autotrophicus Py2]. | 4.99.1.7 | Xanthobacter autotrophicus Py2 |

Ein Beispiel für eine Indolacetaldoxim-Dehydratase, deren Sequenz als Ausgangspunkt für die Erzeugung funktionaler Äquivalente geeignet ist, ist in der nachfolgenden Tabelle 3 gezeigt:

Tabelle 3:

| SEQ ID NO: | Zugangsnummer | Beschreibung | EC-Nr. | Organismus |
|---|---|---|---|---|
| 28 | 049342 | Indoleacetaldoxime-Dehydratase; EC 4.99.1.6; | 4.99.1.6 | Arabidopsis thaliana |

Gemäß einer weiteren Ausführungsform weist das Enzym eine Aminosäuresequenz auf, die abgeleitet ist von einer der in den Tabellen 1 bis 3 genannten Aldoxim-Dehydratasen als Ausgangssequenz und im Vergleich zu der betreffenden Ausgangssequenz ein mindestens zu 60%, wie etwa mindestens 75%, mindestens 80%, mindestens 90%, mindestens 95%, mindestens 96%, mindestens 97%, mindestens 98% oder mindestens 99% identischen Aminosäuresequenz aufweist. Eine besonders bevorzugte Ausführungsform des Verfahrens sieht als Enzym eine PAOx gemäß SEQ ID NO:1 vor (Kato, Y. et al., Biochemistry (2000), 39, 800-809 und Xie, S.-X. et al., Biosci. Biotechnol. Biochem. (2001), 65(12), 2666-2672) oder eine PAOx mit einer zu SEQ ID NO:1 mindestens zu 60%, wie etwa mindestens 75%, mindestens 80%, mindestens 90%, mindestens 95%, mindestens 96%, mindestens 97%, mindestens 98% oder mindestens 99% identischen Aminosäuresequenz.

Man setzt ein Oxim der Formel II

$$R\text{-}[\text{-}CH{=}N\text{-}OH]_n \qquad \textbf{(II)}$$

um, wobei n für 1 steht und das Oxim ausgewählt ist unter Verbindungen der nachfolgenden Tabelle 4, die sich durch unterschiedliche, mit der Oximgruppe verknüpfte Reste R unterscheiden:

Tabelle 4:

| | R-[C=N-OH]$_n$ (II) n = 1 | Name |
|---|---|---|
| 1 | 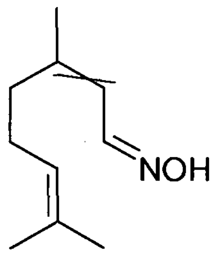 | Citraloxim (3,7-Dimethyl-octa-2,6-dienaloxim) |
| 2 | 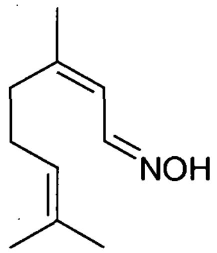 | Neraloxim |
| 3 | 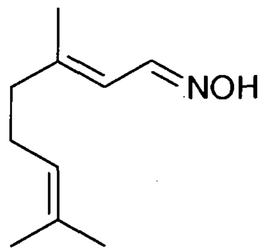 | Geranialoxim |
| 10 | 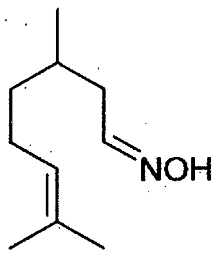 | Citronellaloxim |

Der Rest R kann weiterhin ausgewählt sein aus Analoga der Verbindungen 1 - 3 und 10 der Tabelle 4, wobei der Rest R hydriert ist. Das Oxim ist augewählt unter Verbindung 1 (Citraloxim), Verbindung 2 (Neraloxim), Verbindung 3 (Geranialoxim) und Verbindung 10 (Citronellaloxim) und teil- oder vollhydrierten Analoga davon. Ein Beispiel für eine Verbindung mit hydriertem Rest R ist die Verbindung 10 (Citronellaloxim) in hydrierter Form (also 6,7-Dihydrocitronellyloxim, welches im Rahmen des erfindungsgemäßen Verfahrens zu 6,7-Dihydrocitronellylnitril umgesetzt wird).

Bei dem offenbarten Verfahren können gleichzeitig zwei oder mehr dieser Oxime vorliegen, so dass ein Produktgemisch entsprechender Nitrile entsteht. Vorzugsweise wird jedoch genau ein Oxim eingesetzt.

Gemäß einer besonderen Ausführungsform des erfindungsgemäßen Verfahrens wird Citronellaloxim der Formel IIa

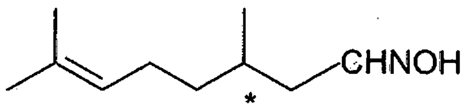

(IIa)

zu Citronellylnitril der Formel Ia

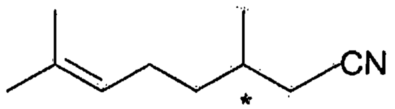

(Ia)

umgesetzt, wobei man die Verbindung der Formel IIa, in stereoisomerenreiner Form oder als Stereoisomerengemisch, wie insbesondere als R/S-E/Z-Gemisch einsetzt.

Eine erfindungsgemäße Umsetzung in Gegenwart einer aliphatischen Aldoxim-Dehydratase (EC 4.99.1.5) oder einer Phenylacetaldoxim-Dehydratase (PAOx) (EC 4.99.1.7) kann insbesondere folgendermaßen erfolgen als:

a) Umsetzung in Gegenwart der Aldoxim-Dehydratase
b) Umsetzung in Gegenwart eines die Aldoxim-Dehydratase enthaltenden Proteingemisches
c) Umsetzung in Gegenwart eines rekombinanten, die Aldoxim-Dehydratase funktional exprimierenden Mikroorganismus, eines davon abgeleiteten Aldoxim-Dehydratasehaltigen Zellhomogenats oder einer Aldoxim-Dehydratase-haltigen Fraktion davon.
Vorzugsweise handelt es sich bei der Aldoxim-Dehydratase um PAOx, gegebenenfalls zusammen mit weiteren Aldoxim-Dehydratasen. Bei Expression in rekombinanten Mikroorganismen werden überraschenderweise hohe Ausbeuten erreicht, beispielsweise Ausbeuten von mehr als 70% bis hin zu im Wesentlichen quantitativen Ausbeuten, bezogen auf das eingesetzte Substrat. Die Umsetzungen können dabei unter anderem auch bei niedrigen Temperaturen erfolgen, beispielsweise bei 15°C-60°C, bevorzugt 25°C - 45°C, wie bei Temperaturen von etwa 30°C.

Bei der Umsetzung in Gegenwart der Aldoxim-Dehydratase kann es sich beispielsweise um

i. freies, gegebenenfalls gereinigtes oder teilweise gereinigtes Polypeptid
ii. immobilisiertes Polypeptid, und/oder
iii. aus Zellen isoliertes Polypeptid

handeln. Bei einer Umsetzung gemäß der oben genannten Option c) in Gegenwart von Mikroorganismen, die eine Aldoxim-Dehydratase funktional exprimieren, handelt es sich um ganzen Zellen, die sich in jeder beliebigen Phase einer Zellkultur befinden können, beispielsweise ruhend oder wachsend, und mindestens ein Aldoxim-Dehydratase-Polypeptid enthalten.

In bevorzugten Ausführungsformen ist die Aldoxim-Dehydratase eine PAOx, beziehungsweise ein Gemisch aus Aldoxim-Dehydratase umfasst neben weiteren Aldoxim-Dehydratasen eine PAOx.

Im Rahmen eines erfindungsgemäßen Verfahrens kann das Enzym mit der Aldoxim-Dehydratase-Aktivität insbesondere von einem Mikroorganismus generiert werden, der das Enzym überproduziert. Der Mikroorganismus kann dabei insbesondere ausgewählt sein aus der Gruppe der Mikroorganismen bestehend aus den Gattungen Leptosphaeria, wie etwa Leptosphaeria maculans; Rhizoctonia, wie etwa Rhizoctonia solani; Sclerotinia, wie etwa Sclerotinia sclerotiorum; Botryotinia, wie etwa Botryotinia fuckeliana; Pseudomonas, wie etwa Pseudomonas chloraphis; Rhodococcus; Giberella, wie etwa Gibberella fujikuroi oder Gibberella zeae; Escherichia, Pichia, Aspergillus, Trichoderma oder Bacillus, wie Escherichia coli oder Pichia pastoris; sowie Saccharomycesw, wie Saccharomyces cerevisiae. Tabelle 6 enthält eine Liste mit weiteren geeigneten Mikroorganismen, wobei auch die Gattungen, die den dort genannen Arten zugrunde liegen, geeignete Beispiele darstellen. Alternativ kann eine Aldoxim-Dehydratase auch aus Pflanzen als überexprimierenden Organismen isoliert und im Sinne der oben angegebenen Optionen i) oder ii) im Rahmen eines erfindungsgemäßen Verfahrens verwendet werden. Als überexprimierende Pflanzen kommen beispielsweise Musa (insbesondere M. acuminata banana) und Arabidopsis (insbesondere A. thaliana) in Frage. Überexprimierende Mikroorganismen oder Pflanzen können dabei vom Fachmann auf fachbekannte Weise durch Screening auf überexprimierende Vertreter ermittelt werden. Alternativ kann Überexpression herbeibegeführt werden durch gentechnologische Verstärkung der Expression eines natürlicherweise in den Mikroorganismen oder Pflanzen vorkommenden Gens, welches eine Aldoxim-Dehydratase exprimiert, beispielsweise durch Einsetzen stärkerer Promotoren zur Steigerung der Transkription.

Gemäß einer besonderen Ausführungsform erfolgt eine Umsetzung des Oxims in Gegenwart eines Mikroorganismus, wobei der Mikroorganismus ein Bakterienstamm ist, der die Aldoxim-Dehydratase, vorzugsweise PAOx,

funktional exprimiert. Der Bakterienstamm kann besipielsweise ausgewählt sein unter Escherichia coli, Pseudomonas putida, Burkholderia glumae, Corynebacterium glutamicum, Bacillus subtilis oder Zymomonas mobilis. Gemäß besonderen Ausführungsformen ist der Bakterienstamm ein E. coli-Stamm. Der Mikroorganismus ist insbesondere ein rekombinanter Mikroorganismus, in den ein heterologes Gen der Oxim-Dehydratase, insbesondere ein Gen für PAOx eingebracht ist.

In einer besonderen Weiterbildung trägt der Mikroorganismus dabei ein Expressionskonstrukt, das eine für die Aldoxim-Dehydratase kodierende Nukleinsäurequenz aufweist. Die Nukleinsäuresequenz kann dabei teilweise oder vollständig an die Codonnutzung des Mikroorganismus angepasst sein. Eine vollständige Anpassung liegt vor, wenn alle Codons aller Aminosäuren so verändert sind, dass eine optimale Translation im vorgesehenen Mikroorganismus erfolgt, sofern nicht ohnehin bereits ein optimales Codon vorlag. Von einer teilweisen Anpassung ist auszugehen, wenn nicht die Codons aller Aminosäuren, sondern nur ausgewählter Aminosäuren optimiert sind, oder wenn nicht alle der innerhalb der Nukleinsäuresequenz vorkommenden Codons jeweils einer Aminosäure optimiert sind.

Gemäß einer weiteren bevorzugten Ausführungsform des erfindungsgemäßen Verfahrens exprimiert der Mikrorganismus wenigstens ein Chaperon, das die funktionale Expression der Aldoxim-Dehydratase, die insbesondere PAOx sein kann, unterstützt. Die funktionale Expression kann insbesondere dadurch unterstützt werden, dass das Chaperon die korrekte Faltung der exprimierten Oxim-Dehydratase unterstützt, insbesondere während der oder anschließend an die Translation. Durch die Expression von Chaperonen kann vorteilhaft der Anteil an funktionaler Aldoxim-Dehydratase im Mikroorganismus gesteigert und der Umsatz von Oxim zu Nitril gesteigert werden.

Im Rahmen des erfindungsgemäßen Verfahrens kann insbesondere vorgesehen sein, dass der Mikroorganismus ein oder mehrere Chaperone exprimiert, die ausgewählt sind unter GroEL und GroES. Dem Fachmann sind weitere Chaperone bekannt, die in fünf große Familien eingeteilt werden, nämlich die Hsp100/Clp-Familie, die Hsp90-Familie, die Hsp70-Familie (mit beispielsweise DnaK, DNAJ, Hsc62, Hsc56, and GrpE als Vertretern in E.coli), die Hsp60/GroEL-Familie (mit dem bereits erwähnten GroEL) und die Familie der kleinen Hitzeschockproteine (Hsp20). Chaperone können ebenfalls aus diesen Proteinfamilien ausgewählt werden. Ebenfalls können Kombinationen gebildet werden, beispielsweise aus dem Dank/DnaJ/GrpE-Komplex, dem GroEL/ES-Komplex und Trigger Factor, wie beispielsweise beschrieben in O'Donnell und Lis, MIT 7.88 Research Paper, 2006. Das oder die Chaperone können endogen in dem verwendeten Mikroorganismus exprimiert werden, oder teilweise oder ganz durch zusätzlich in den Mikroorganismus eingebrachte Nukleinsäuresequenzen exprimiert werden. Diese Nukleinsäuresequenzen können in das Genom des Mikroorganismus eingebracht sein, beispielsweise in ein Bakterienchromosom, oder extrachromosomal auf Expressionskonstrukten vorliegen. Erfolgt eine Expression einer Aldoxim-Dehydratase von einem extrachromosomalen Expressionskonstrukt, etwa weil ein heterologes Oxim-Dehydratase-Gen in einem Mikroorganismus exprimiert werden soll oder weil die Expression eines chromosomalen Gens durch die zusätzliche Expression eines extrachromosomalen Aldoxim-Dehydratase-Gens ergänzt werden soll, so können das Aldoxim-Dehydratase-Gen und das Chaperon-Gen auf einem gemeinsamen oder auf getrennten Expressionskonstrukten vorliegen.

Eine bevorzugte Ausführungsform des erfindungsgemäßen Verfahrens umfasst wenigstens die folgenden Schritte a), b) und d):

a) einen ein Enzym mit Aldoxim-Dehydratase-Aktivität produzierenden Mikroorganismus aus einer natürlichen Quelle zu isolieren oder rekombinant herzustellen,
b) diesen Mikroorganismus zu vermehren,
c) aus dem Mikroorganismus das Enzym mit Aldoxim-Dehydratase-Aktivität gegebenenfalls zu isolieren oder eine dieses Enzym enthaltende Proteinfraktion herzustellen, und
d) den Mikroorganismus gemäß Stufe b) oder das Enzym gemäß Stufe c) in ein Medium zu überführen, das das Oxim enthält.

In dem erfindungsgemäßen Verfahren wird Substrat, wie z.B. Citronellaloxim, mit dem Enzym, das die Aktivität der Aldoxim-Dehydratase besitzt, in einem Medium in Kontakt gebracht und/oder so inkubiert, dass eine Umsetzung des Substrats, wie z.B. von Citronellaloxim, zu Citronallalnitril, in Gegenwart des Enzyms erfolgt. Bevorzugt handelt es sich bei dem Medium um ein wässriges Reaktionsmedium.

Der pH-Wert des wässrigen Reaktionsmediums, in dem das erfindungsgemäße Verfahren bevorzugt durchgeführt wird, wird dabei vorteilhaft zwischen pH 4 und 12, bevorzugt zwischen pH 4,5 und 9, besonders bevorzugt zwischen pH 5 und 8 gehalten.

Bei den wässrigen Reaktionsmedien handelt es sich vorzugsweise um gepufferte Lösungen, die in der Regel einen pH-Wert von vorzugsweise von 5 bis 8, aufweisen. Als Puffer kann ein Citrat-, Phosphat-, TRIS- (Tris(hydroxymethyl)-aminomethan) oder MES-Puffer (2-(N-Morpholino)ethansulfonsäure) verwendet werden. Ferner kann das Reaktionsmedium noch weitere Zusätze enthalten, wie z.B. Detergentien (beispielsweise Taurodeoxycholat), FMN, Ionen, wie Metallionen (z.B. $Fe^{2+}$ und $Sn^{2+}$) oder Anionen wie $SO_3^{2-}$, sowie Natriumazid. Detaillierte Zusammensetzungen geeigneter Reaktionsgemische sind auch in folgenden Literaturquellen zu finden: Kato, Y. et al., Biochemistry (2000),

39, 800-809 und Xie, S.-X. et al., Biosci. Biotechnol. Biochem. (2001), 65(12), 2666-2672.

Das Substrat, wie z.B. Citronellaloxim, wird vorzugsweise in einer Konzentration von 2 - 200mM, besonders bevorzugt von 5 - 25mM in die enzymatische Umsetzung eingesetzt und kann kontinuierlich oder diskontinuierlich nachgeführt werden. Die Umsetzung kann aber auch mit reinem Aldoxim (z.B. Citronellaloxim) in Anwesenheit nur des Enzym-exprimierenden, wie der PAOx- exprimierenden Wirtsorganismus (z.B. E. coli) - wie im Beispiel 2 beschrieben - durchgeführt werden.

Die enzymatische Umsetzung von Oxim zu Nitril erfolgt in der Regel bei einer Reaktionstemperatur unterhalb der Deaktivierungstemperatur des eingesetzten Enzyms und oberhalb von -10°C. Bevorzugt wird das erfindungsgemäße Verfahren bei einer Temperatur zwischen 0°C und 95°C, besonders bevorzugt bei einer Temperatur zwischen 15°C und 60°C, insbesondere zwischen 20°C und 40°C, z.B. bei etwa 25°C bis 30°C durchgeführt.

Besonders bevorzugt ist ein erfindungsgemäßes Verfahren, wobei die Reaktion von Oxim zu Nitril bei einer Temperatur im Bereich von 20 bis 40 °C und/oder einem pH-Wert im Bereich von 4 bis 10 erfolgt, bevorzugt bei pH 8.

Neben diesen einphasigen wässrigen Systemen werden in einer weiteren Variante der Erfindung auch zweiphasige Systeme eingesetzt. Dabei werden neben einer wässrigen Phase als zweite Phase, organische, nicht-wassermischbare Reaktionsmedien verwendet. Dadurch akkumulieren die Reaktionsprodukte in der organischen Phase. Nach der Reaktion ist das Produkt, wie z.B. Citralnitril, Neralnitril, Geranialnitril oder Citronellalnitril, in der organischen Phase leicht von der wässrigen Phase, die den Biokatalysator enthält, abtrennbar.

Bevorzugt ist ein erfindungsgemäßes Verfahren, dadurch gekennzeichnet, dass die Herstellung der Nitrile Citralnitril, Neralnitril, Geranialnitril oder Citronellalnitril, in einphasigen wässrigen Systemen oder in zweiphasigen Systemen erfolgt.

Das Reaktionsprodukt, wie z.B. Citralnitril, Neralnitril, Geranialnitril oder Citronellalnitril, kann mit organischen Lösungsmitteln extrahiert werden und gegebenenfalls zur Aufreinigung destilliert werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Heptan, Methyl-tert-butylether, Diisopropylether, Tetrahydrofuran, Ethylacetat verwendet.

Die erfindungsgemäß eingesetzten Aldoxim-Dehydratasen können im erfindungsgemäßen Verfahren als freies oder immobilisiertes Enzym, wie oben bereits beschrieben, verwendet werden.

Für das erfindungsgemäße Verfahren können ruhende oder wachsende, freie oder immobilisierte Zellen verwendet werden, die für die Aldoxim-Dehydratase kodierenden Nukleinsäuren, Nukleinsäurekonstrukte oder Vektoren enthalten. Auch aufgeschlossene Zellen, wie Zelllysate oder Zellhomogenate, können verwendet werden. Unter aufgeschlossenen Zellen sind beispielsweise Zellen zu verstehen, die über eine Behandlung beispielsweise mit Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder teilweise gereinigte Enzyme können für das Verfahren verwendet werden.

Werden für das erfindungsgemäße Verfahren freie Organismen oder Enzyme verwendet, so werden diese vor der Extraktion zweckmäßigerweise abgetrennt, beispielsweise über eine Filtration oder Zentrifugation.

Das erfindungsgemäße Verfahren kann batchweise, semi-batchweise oder kontinuierlich betrieben werden.

## Experimenteller Teil

Sofern keine speziellen Angaben in den folgenden Beispielen gemacht werden, gelten die folgenden allgemeinen Angaben.

### A. Allgemeine Angaben

Sämtliche eingesetzte Materialien und Mikroorganismen sind im Handel oder in Stammsammlungen erhältliche Produkte.

Soweit nicht anderes angegeben wird, erfolgt die Klonierung und Expression von rekombinanten Proteinen nach Standardmethoden, wie z.B. in Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989 beschrieben.

**a) Bakterienstämme, Plasmide und Wachstumsbedingungen**

Sämtliche Experimente wurden mit *E. coli* durchgeführt. Für die Expression von PAOx wurde E. coli TG10, ein TG1-Derivat ohne Rhamnose-Isomerase, verwendet.

**b) Gaschromatographie**

**Messgerät:** Agilent 6890 N

**Säule:** Optima 5 Accent $_{Länge}$= 30m, $\varnothing_{innen}$ = 0,25mm, $\varnothing_{außen}$ = 0,4mm, Filmdicke 0,25 μm Fa. Macherey&Nagel # 725820.30
**Fluss:**1,0ml/min bei 13,5PSI (bei Ofentemp. 80°C)
**Split:** 1:50, Splitflow: 50ml/min, Septum purge 3ml/min (bei Ofentemp. 80°C)
**Trägergas:** Helium
**Injektor:** Split/Splitless mit Split Liner siltec-deactivated (Restec # 20713-214.5)
**Injektortemperatur:** 280°C
**Injektionsvolumen:** 1μl
**Detektor:** FID mit 300ml/min Luft, 30ml/min Wasserstoff und 30ml/min Make-Up-Gas (Helium)
**Detektortemperatur:** 320°C

**Temperaturprogramm:**

| | |
|---|---|
| Start: | 60°C |
| Verweildauer 1: | 0min |
| Temperaturrampe 1: | 15°C/min |
| Endtemperatur 1: | 320°C |
| Verweildauer 2: | 5min |
| Gesamtlaufzeit: | 22,3min |

**Auswertung:** Empower-2-Software nach Flächen-%

**B. Beispiele**

**Beispiel 1: Expression von PAOx in E.coli** (nicht erfinfungsgemäß)

Im vorliegenden Beispiel wurde die Kodonnutzung vom Gen der Phenlyacetaldoxim-Dehydratase (PAOx) aus *Bacillus* sp. Stamm OxB-1 für die Expression in *E.coli* optimiert. Die neue DNA-Sequenz wurde synthetisiert und in pDHE, einen Rhamnose-induzierbaren Expressionsvektor kloniert. Die GroEL/S-Chaperone, die für eine Produktion von löslicher PAOx erforderlich sind, wurden in IPTG-induzierbare pAgro- bzw. pHSG-Vektoren kloniert. Die Expression von PAOx erfolgte in *E. coli* TG10, ein TG1-Abkömmling ohne Rhamnose-Isomerase. TG10-Zellen, die pAgro und pHSG (TG10+) enthielten, wurden mit pDHE-PAOx transformiert und in 2xYT 5 h bei 37 °C in Gegenwart von Ampicillin (pDHE), Spectinomycin (pAgro) und Chloramphenicol (pHSG) kultiviert. 5 mL dieser Kultur wurden in 500 mL des gleichen Mediums transferiert, das 100 μM IPTG und 0.5 g/L Rhamnose enthielt. Die Induktion erfolgte bei 30 °C über einen Zeitraum von etwa 18 h. Die Zellen wurden durch Zentrifugation geerntet und als Katalysatoren bei der Umwandlung von Citronellaloxim in Citronellylnitril verwendet. Gegebenenfalls wurden die Zellen bis zur Verwendung bei -20°C gelagert.

**Beispiel 2: Umwandlung von Citronellaloxim in Citronellylnitril durch PAOx**

Die enzymatische Umwandlung von Citronellaloxim in das entsprechende Nitril wurde folgendermaßen durchgeführt: 50 mL racemisches R/S-E/Z Citronellaloxim wurden mit 12,5 g *E.coli* TG10 gemischt, die Phenylacetaldoxim-Dehydratase (PAOx) exprimierten. Das Reaktionsgemisch wurde unter Rühren bei 30°C inkubiert. Proben des Reaktionsgemisches (0,1 mL) wurden nach 18h und 90h entnommen und mit 0,5 mL n-Heptan gemischt. Die organische Phase wurde über Gaschromatographie (GC) analysisert. Die GC-Analyse erfolgte auf einem Agilent 6890 N (Säule: Optima 5 Accent, Macherey & Nagel; Fließgeschwindigkeit: 1 mL/min bei 13,5 PSI, Injectionstemp. 280°C; Detektionstemp. 320°C; Detektor: FID; Retentionszeit Ctronellaloxim: 7,816 min & 8,018 min; Retentionsezeit Citronellylnitril:

6,765 min). Nach 90 h war das racemische R/S-E/Z Citronellaloxim quantitativ in das entsprechende Nitril überführt worden. Die dabei erfolgte Reaktion ist in Fig. 1 gezeigt. Die entsprechenden Gaschromatogramme vor, während und nach der Umwandlung sind in Fig. 2A, Fig. 2B und Fig. 2C gezeigt. Ein grober zeitlicher Verlauf der Umsetzung, in welcher der prozentuale Anteil der Peakflächen der Gaschromatogramme angegeben ist, ist in Tabelle 5 gezeigt.

Tabelle 5

| Zeit [h] | Citronellaloxim | Citronellalnitril |
|---|---|---|
| 0 | 100,00 | 0,00 |
| 18 | 50,03 | 49,97 |
| 90 | 0,03 | 99,97 |

Tabelle 6 - Sequenzangaben:

| SEQ ID NO | Quelle | Art | Bemerkung | |
|---|---|---|---|---|
| 1 | Bacillus s. OxB-1 | AS | PAOx | |
| 2 | Bacillus s. OxB-1 | NS | PAOx | Wildtyp-DNA für SEQ ID NO:1 |
| 3 | künstlich | NS | PAOx | SEQ ID NO:1 kodonoptimiert für *E.coli* |
| 4 | Arthrobacter aurescens TC1 | AS | Aliphatische Aldoxim-Dehydratase | |
| 5 | Acinetobacter sp. Stamm ADP1 | AS | Aliphatische Aldoxim-Dehydratase | |
| 6 | Acidovorax avenae (ATCC 19860) | AS | Phenylacetaldoxim-Dehydratase | |
| 7 | Actinosynnema mirum (DSM 43827) | AS | Phenylacetaldoxim-Dehydratase | |
| 8 | Bradyrhizobium sp. BTAi1 | AS | Phenylacetaldoxim-Dehydratase | |
| 9 | Burkholderia ambifaria IOP40-10 | AS | Phenylacetaldoxim-Dehydratase | |
| 10 | Burkholderia ambifaria MC40-6 | AS | Phenylacetaldoxim-Dehydratase | |
| 11 | Burkholderia cenocepacia MC0-3 | AS | Phenylacetaldoxim-Dehydratase | |
| 12 | Pantoea sp. At-9b | AS | Phenylacetaldoxim-Dehyd ratase | |
| 13 | Pseudomonas fluorescens WH6 | AS | Phenylacetaldoxim-Dehydratase | |
| 14 | Pseudomonas fluorescens WH6 | AS | Phenylacetaldoxim-Dehydratase | |
| 15 | Pseudomonas putida F1 | AS | Phenylacetaldoxim-Dehydratase | |
| 16 | Pseudomonas putida Stamm BIRD-1 | AS | Phenylacetaldoxim-Dehydratase | |
| 17 | Shewanella woodyi ATCC 51908 | AS | Phenylacetaldoxim-Dehydratase | |
| 18 | Sphingomonas wittichii RW1 | AS | Phenylacetaldoxim-Dehyd ratase | |

(fortgesetzt)

| SEQ ID NO | Quelle | Art | Bemerkung | |
|---|---|---|---|---|
| 19 | Variovorax paradoxus EPS | AS | Phenylacetaldoxim-Dehyd ratase | |
| 20 | Variovorax paradoxus S110 | AS | Phenylacetaldoxim-Dehydratase | |
| 21 | Variovorax paradoxus S110 | AS | Phenylacetaldoxim-Dehyd ratase | |
| 22 | Xanthobacter autotrophicus Py2 | AS | Phenylacetaldoxim-Dehydratase | |
| 23 | Gibberella zeae | AS | Phenylacetaldoxim-Dehydratase | |
| 24 | Pseudomonas chlororaphis | AS | Aliphatische Aldoxim-Dehydratase | |
| 25 | Pseudomonas sp. K-9 | AS | Aliphatische Aldoxim-Dehydratase | |
| 26 | Rhodococcus erythropolis | AS | Aliphatische Aldoxim-Dehydratase | |
| 27 | Rhodococcus globerulus | AS | Aliphatische Aldoxim-Dehydratase | |
| 28 | Arabidopsis thaliana | AS | Indoleacetaldoxim_Dehydratase | |
| NS: Nukleinsäure; AS: Aminosäure | | | | |

Es folgt eine Auflistung brauchbarer Sequenzen gemäß Tabelle 6, die - mit Ausnahme der SEQ ID NO: 28 - direkt für ein erfindungsgemäßes Verfahren einsetzbar sind oder als Ausgangssequenzen, gegebenenfalls auch in Form kodierender Nukleinsäuresequenzen, für die Erzeugung funktionaler Äquivalente dienen, welche dann in einem erfindungsgemäßen Verfahren einsetzbar sind. Die Zugangsnummern verweisen auf fachbekannte Datenbanken, z.B. NCBI (National Center for Biotechnology Information), GeneBank, oder SwissProt.

SEQ ID NO: 1; Zugangsnummern: BAA90461; P82604 (UniProtKB/Swiss-Prot)

MKNMPENHNPQANAWTAEFPPEMSYVVFAQIGIQSKSLDHAAEHLGMMKKSFDLRTGP
KHVDRALHQGADGYQDSIFLAYWDEPETFKSWVADPEVQKWWSGKKIDENSPIGYWSEVTTI
PIDHFETLHSGENYDNGVSHFVPIKHTEVHEYWGAMRDRMPVSASSDLESPLGLQLPEPIVRE
SFGKRLKVTAPDNICLIRTAQNWSKCGSGERETYIGLVEPTLIKANTFLRENASETGCISSKLVY
EQTHDGEIVDKSCVIGYYLSMGHLERWTHDHPTHKAIYGTFYEMLKRHDFKTELALWHEVSVL
QSKDIELIYVNCHPSTGFLPFFEVTEIQEPLLKSPSVRIQ

SEQ ID NO: 2; Zugangsnummer: AB028892 (NCBI)

ttgaaaaatatgccggaaaatcacaatccacaagcgaatgcctggactgccgaatttcctcctgaaatgagctatgtagtattt
gcgcagattgggattcaaagcaagtctttggatcacgcagcggaacatttgggaatgatgaaaaagagtttcgatttgcggacaggc
cccaaacatgtggatcgagccttgcatcaaggagccgatggataccaagattccatctttttagcctactgggatgagcctgaaacattt
aaatcatgggttgcggatcctgaagtacaaaagtggtggtcgggtaaaaaaatcgatgaaaatagtccaatcgggtattggagtgag
gtaacgaccattccgattgatcactttgagactcttcattccggagaaaattacgataatggggtttcacactttgtaccgatcaagcatac
agaagtccatgaatattggggagcaatgcgcgaccgcatgccggtgtctgccagtagtgatttggaaagccccttggccttcaattac
cggaacccattgtccgggagtctttcggaaaacggctaaaagtcacggcgccggataatatttgcttgattcgaaccgctcaaaattgg
tctaaatgtggtagcggggaaagggaaacgtatataggactagtggaaccgaccctcataaaagcgaatacgtttcttcgtgaaaat
gctagtgaaacaggctgtattagttcaaaattagtctatgaacagacccatgacggcgaaatagtagataaatcatgtgtcatcggata
ttatctctccatggggcatcttgaacgctggacgcatgatcatccaacacataaagcgatctacggaacctttatgagatgttgaaaag
gcatgattttaagaccgaacttgctttatggcacgaggtttcggtgcttcaatccaaagatatcgagcttatctatgtcaactgccatccga
gtactggatttcttccattctttgaagtgacagaaattcaagagcctttactgaaaagccctagcgtcaggatccagtga

SEQ ID NO: 3;

atgaaaaacatgccagaaaaccacaacccgcaggctaacgcctggactgcagaattcccgccagagatgtcttacgttgtc
ttcgcgcagattggcatccagtctaaaagcctggatcatgcagccgaacatctgggcatgatgaaaaaatctttcgacctccgtactgg
tccaaaacacgttgatcgcgcactgcaccagggtgctgatggctatcaggactctatcttcctggcctactgggacgaaccagaaacc
ttcaaaagctgggttgcagacccagaagtgcagaaatggtggagcggtaaaaaaatcgatgaaaacagcccgatcggctattggtc
tgaggttaccactatcccgattgaccacttcgagaccctgcactctggtgagaactatgacaacggcgtttcccacttcgtgccgatcaa

acataccgaagtgcacgaatactggggtgctatgcgtgatcgtatgccggtgtctgcttcttccgatctggaaagcccgctgggtctcca
gctgccagaaccgatcgtacgtgagagctttggtaaacgcctgaaagttaccgctccagacaacatctgcctgattcgtaccgcacag
aactggtccaaatgtggttctggtgaacgcgaaacctacattggcctggtcgaaccgactctgatcaaagcgaacaccttcctgcgtg
aaaacgcgtctgagaccggttgcattagctctaaactggtttacgagcagacccatgatggcgaaatcgtagataaatcctgtgtaatc
ggctactatctgtccatgggtcatctggaacgctggactcacgaccacccaactcacaaagcgatttacggcacgttctacgaaatgct
gaaacgtcacgactttaaaacggaactggcgctgtggcacgaagtaagcgttctgcagtctaaagacatcgagctgatctatgtcaac
tgccacccatccacgggttttctgccgttctttgaagtgaccgaaatccaggaaccactgctgaaatccccatccgtgcgtattcagtga

SEQ ID NO: 4, Zugangsnummern: NCBI: YP_947647.1, GenBank: ABM07831.1

MAPDPSRQDPAIESSIPRHFTVKRTRPKRAGAGYAPPYPSYSVRFAEGVSNLVCAFLGV
QSRAPLSHEAKVASAGMWELCANEDGPMSREHAVHTDEQGFDNQVIIAYWDDVDAYGRWFK
KHRDALIGAGLEPSDYGRWIEAVTPEARGFETLYSSNTFPEGAARMATGGFTGEIQEHGYWGS
MRDRLPIAQTDALEPVGDPVVPRNPGIVRVEPHDNLAVIRSGQDWSLCDDAERASYFSHVEPQ
LKAGMDFLTTEGASIGCYANRYMTSSDGNGNVLEQSFGLSFWHSLEDMERWAESHPTHVAIF
RSAMTFLQANAGARLRLSHEVAVVSRDQQYYEYNNCHAGTGMLGARRPLGTATPGTRI

SEQ ID NO: 5; Zugangsnummer: NCBI: YP_046290.1; GenBank: CAG68468.1

MESAIDKHLKCPRTLSRRIHDEYEPPFAMWVARADESLQQVVMAYFGVQFKSEQKAIAL
KAMQHIVQSFSLDNGPQNHDITHHTDNQGYENYIVVGYWRDPGAYCRWFRSTEVSHWWDSD
ERLNDGIGYFREIVIPRADQFETLYAFKEDLPGVGAVMDDISDDIQEHGYWGSARERFPISQTD
RMLANGELHIISGDPEKGGRVLVQGHDNITLIRSGQDWVNADEKERELYFNEMLPSLQAGMDF
LRDEGQALGCYSNRFVRNVDIDGNLLDIAYDIGYWRSLDKLERWAESHPTHLRIFTTFFKVVTG
LQNLRLYHEVSVSDAKNQVFEYINCHPQTGMMRDAQMT

SEQ ID NO: 6; Zugangsnummer: F0QD95_ACIAP (UniProtKB/TrEMBL)

MESAIPPRLQCPRSLSRRVADDYQPPFPMWVARPQADLQQVVMAYFGIQYQGEAQKPR
ALAVLREWVAAFGAPDGPLRHDLTHHIDAQGYDNLIAVGYWRDPEAHRRWMQAPAQAGWW
NAPERLQEGLGYFREVSAPRAEQFETLYAFQDALPGVGGVMESTSGDIQEHGYWGSMRDRF
PASQVDRMQARGTLLIAEGDPAHGGRVVVRGHDNIALIRSGQDWMEAEAEERRLYLEDIEPTL
HAGMDFLRDQGAAVGCYSNRYVHNIDLDGRRLDQSYNIGHWRSVDLLERWAESHPTHLRIFG
TFFKVAAGLSKLRLYHEVSVSDAASQHFEYIQCHPATGMMRDARLQAPA

SEQ ID NO: 7, Zugangsnummer: YP_003097901.1 (NCBI)

MSSLPLDDRATAHRPEGYDPRGGPGHEVRWSDDVTHLVVARFGVQTDDSAAGVKAIAR
VLELAAGESGPALVERVSDDDSEMAVCYWPDPEAHRAWWASGPVRDWWASLPVDGPIGHW
HETSVTPVEQFETLYSAEFAAGPSRFAGTGPTNLHDYDNSTLDRMPATAHRDLRQERAEEPTT
DLPPGESPRGRRVRLAEPSPSGLCWIRTAQEWSIAPDDQLASYRDGVEPAYRTAIAHLQDNPH
DTGCLSARLVGNLDANGARAAGAEAVVWWRGIGDLLRWAHDHKTHQDILNGFWEHVIAKFGP
GTRVRLWHEVHVLPEGALTAEYVNCHPGTGLLQTWPG

SEQ ID NO: 8, Zugangsnummer: YP_001240697 (NCBI)

MESAIPPHLITTRCRHRRVDDDYKPPYPSFVARHGADVSRVVMAYFGVQYRAETPAAAS
TADFMVLVSRADGPSHWDLAHYVDQAGFANDVFVAYWDDVVRFDSWFEPARAAWTGPGAE
GGGRFIEVLRPAVERYETLFSSLGRPEGIAVIAEGMSGEVLEHAYWGGMRDRIPLSQTSEMRS
LGKPTLVQDGPRLRVIAQDNLCMIRSGQDWSDTDAAERRMYLDDVEPVLREGMDFLRDQGLS
IGCYANRYMRLRGADGALTEKSYGQSWWQSLSALERWAESHPTHVRIFGAAMKYLSSLGPAA
RLRLYHEVTVAAADEQFFEYRGCHAKTGMLAAAG

SEQ ID NO: 9, Zugangsnummer: ZP_02891657 (NCBI)

MESAIDKHLVCPRTLSRRVADDYQPPFPMYVARASEDLSQVVMGYFGVQYRGADQRSA ALAALRRIVADFDAPDGPGNHDLTQHTDNQGYDNLIAVGYWRDPDAYARWIASPAVAEWWTS DARLADGIGYFREIVAPRAEQFETLYAFTADFPGVGAIMDGVSGEIEEHGYWGSMRDRFPISQT DWMHADGELRIVAGDPARGGRVVVLAHDNIALIRSGQDWRAAQDDERRLYLDEIEPTLRSGM EFLRDNGVDVGCYSNRYVRSIDLDGNLLDESYNIGHWRSLDRLERWAESHPTHLRIFVTFFRV VTGLSKLRLYHEVSVFDAKHQVYEYVNCHPNTGMMRDAVAR

SEQ ID NO: 10; Zugangsnummern: ACB68693 (GenBank), YP_001816246 (NCBI)

MESAIDKHLVCPRTLSRRVADDYQPPFPMYVARAAEDLSQVVMGYFGVQYRGADKRSV ALAALRRIVADFDAPDGPGNHDLTQHTDNQGYDNLIAVGYWRDPDAYARWIASPAVAEWWAS DARLADGIGYFREIVAPRAEQFETLYAFTNDFPGVGSIMDGVSGEIEEHGYWGSMRDRFPISQT DWMHADGELRIVAGDPARGGRVVVLAHDNIALIRSGQDWRAAEDDERRLYLDEIEPTLRSGME FLRDNGVDVGCYSNRYVRSIDLDGNLLDESYNIGHWRSLDRLERWAESHPTHLRIFVTFFRVV TGLSKLRLYHEVSVFDAKHQVYEYVNCHPTTGMMRDAAAR

SEQ ID NO: 11, Zugangsnummer: YP_001776877 (NCBI)

MESAIDKHLICPRTLSRRVADDYQPPFPMYVARAAEDLSQVVMGYFGVQYSGADKRAAA MAALRRIVADFGGQDGPNNFDLTQHTDDEGYENLIAVGYWRDPAAYARWIASPALVEWWASD ARLADGIGYFREIVAPRAEQFETLYAFTSDFPGVGAIMDGVSGEIEEHGYWGSMRDRFPISQTD WMNANGELRIVDGDPARGGRVVVLAHDNIALIRSGQDWRAAESDERRLYLEEIEPTLRSGMEF

LRDNGKDVGCYSNRYVRSIDLDGNVLDESYNIGHWRSLDRLERWAESHPTHLRIFVTFFRVVT GLSKLRLYHEVSVFDAKHQVYEYVNCHPRTGLMRDAVAIAR

SEQ ID NO: 12, Zugangsnummer: YP_004119277 (NCBI)

MESAIDTHLKCPRTLSRRVHDDYQPPFPMFAGRADASLTQVVMAYLGVQFREEQRAAAI TAMQHIVRSFSLDNGPGNHDVTFHTDNQGFGNFIVVGYWRDPAAYCRWLHQPAITGWWSSD DRLRDGLGYFREIIAPRAEQFETLYAFKEALPGVGAVMDNLSGEIQEHGYWGSVRDRIPASQT DWLQPDGELRIISGDPAAGGRVVVQGHDNITLIRSGQDWMDADEQERALYFTEMLPPLQAGM DFLRDEGQTLGCYSNRFVRNVDIDGNVLDIAYDIGFWRSLDRLERWAESHPTHLRIFTTFFRVV AGLQKLRLYHEVSVSDARFQTFEYINCHPQTGMLRDAVR

SEQ ID NO: 13, Zugangsnummer: ZP_07774756 (NCBI)

MKPTTELQVVAGDPAKGGRVVVMGHDNLTLIRSGQDWADAEADERSLYLDEILPTLQDG MDFLRDNGQPLGCYSNRFVRNIDLDGNFLDVSYNIGHWRSVEKLERWAESHPTHLRIFVTFFR VAAGLKKLRLYHEVSVSDAKSQLFEYINCHPHTGMLRDAQAATA

SEQ ID NO: 14:, Zugangsnummer: ZP_07774757 (NCBI)

MESAIDTHLKCPRTLSRRVPDEYQPPFPMWVARADEQLEQVVMAYLGVQYRGEAQREA
ALQAMRHIVGSFSLADGPQTHDLTHHTDSSGFDNLIVVGYWKDPGAHCRWLRSAPVNDWWA
SQDRLSDGLGYFREISAPRAEQFETLYAFQDNLPGVGAVMDATSGEIENTVTGARCATASPSP
RQTG

SEQ ID NO: 15; Zugangsnummer: YP_001268042 (NCBI)

MESAIDKHLVCPRTLSRRVPDDYQPPFPMWVGRADEQLTQVVMAYLGVQYRGDGQRE
RALQAMREILGSFSLTDGPLTHDLTHHTDSSGYDNLMIVGYWKDAGAYCRWLRSPEVDGWW
SSPQRLNDGLGYYREITAPRAEQFETLYAFQNDLPGVGAIMDNTSGEIEEHGYWGSMRDRFPV
SQTDWMNPNGELRVVAGDPAKGGRVVVLGHDNIALIRSGQDWATAEAAERSLYLDEILPTLQD
GMDFLRDNGQPLGCYSNRFVRNIDADGNLLDMSYNIGHWRSLEKLERWAESHPTHLRIFVTFF
RVAAGLEKLRLYHEVSVSDASSQVFEYINCHPHTGMLRDAKVSSN

SEQ ID NO. 16, Zugangsnummer: E4RFH2_PSEPB (UniProtKB/TrEMBL)

MESAIDKHLMCPRTLSRRVPDDYQPPFPMWVGRADEQLTQVVMAYLGVQYRGDSQRE
RALQAMREILGSFSLSDGPLTHDLTHHTDSSGYDNLMIVGYWKDTGAYCRWSRSPEVDGWW
SSPQRLNDGLGYYREITAPRAEQFETLYAFQSDLPGVGAIMDNTSGEIEEHGYWGSMRDRFPV
SQTDWMNPNGELRVVAGDPAKGGRVVVIGHDNIALIRSGQDWAAAEAAERSLYLDEILPTLQD

GMDFLRDNGQPLGCYSNRFVRNIDADGNVLDMSYNIGHWRSLEKLERWAESHPTHLRIFVTFF
RVAAGLEKLRLYHEVSVSDASSQVFEYINCHPHTGMLRDAKVSSN

SEQ ID NO: 17, Zugangsnummer: YP_001758607 (NCBI)

MMNNMPKNWTPPAPAWTSLWKTDEENLVCGLFAIQGQHSAPLDDWAKKAFTGEFSPK
LLEQGMFTDKAGITNYLYIAYWFASDYKTWWQQSAANSWWASPLLDEGDISVWREVFTMPHQ
RFETLHSSENAHGAARLSPSLEGPMMEHGYSGAARDRIPCSSSQDIKNDNSIWEHLQVNVENK
SNRIKLSPPKNMCVIRSGQDWTHCEEDEKEYYLTNVHTVLKKGMDYLSNNPVKTHCASMRFIT
KTDGNWCSVEQTFGLGYGNDIYAFENWAKSHPTHIAIFDRFMGMVEKYNVDLKLQLWHEVTLI
PEQDCEFEYINCHGQTGLLCYINI

SEQ ID NO: 18, Zugangsnummer: YP_001261493 (NCBI)

MDSSITPHLACPRSRPRRIADDYAPPYPAWSARIDPAIGQVVMASYGVQGRESGDVAAA
LAHVRALRATLDADVRHVDLARYVDEAGYDTLVLQPYWTDPEAFRRWEARADVAALLAAETGL
GHFREILTPTVERLETLYSTEDEMEGLGRALERRSPPVQEHAYWGSARDRFPIAQTDALEPAG
QLGFAADGAGRVTVRGHDNIAIIRSGQDWGPTGGEERRLYLAEIEPVLRAGMDFLRDRGGEC
GCYLNRYMRIVDMDGAPQEKSFGWSYWRSLGDMENWSEAHPSHLAIFGTFMRIVQQLNFDLK
LRLWHEVYVVTPDQQLYDYRNCHPDTGFLKQMPR

SEQ ID NO: 19, Zugangsnummer: YP_004155682 (NCIB)

MESAIAEHLKCPRTRHRRVEDDYTPPYPVWSARAPTSVTQVTMGYFGVQSRGPEMQGRACAALMKIARDFALPDGPGHHDLAHYVDADGFDNMVAIAYWHDAAAFARWSATPAIDAWWRSDERLNEGLGYFREIASPRVEHFETMFNTPDRFEGIGVVMGELSGELQEHGYWGSMRDRIPLSQTDALSPSGTRAVVAGTPAPGQRVRIAGHENIAMIRSGQEWADTTGQERTLYLEDMEPVLREGMDFLRDQGLGIGCYSNRYMHHLDAKGAPLQKSFGLSYWRSLADMERWAESHPTHVAIFGSFMRYVQALNFQLQLRVYHEVSVLKADEQSYEYINCRARSGLMNGLAVT

SEQ ID NO: 20, Zugangsnummer YP_002942593 (NCBI)

MESRDADARALLARVANTFAGVGGPASLGRGVATGAGGERSDIFYAYWNSSAEYASWLTTPRVASLWTDDALLRGPIGLWRESMIIPVERNETNYSNDAAYDGIAQIDKEMRKTDVHGYWGSARERIPASAHDTMPSAAPDFMGRPAASMETLGRRFRITLPGNTCVIRSFQDWSQAQAAEVDWYLGNVEPVLRVGLDYLNGNRTEAKCYGMRYIREYDISGAIDLNRTSTFGYFESLQTLERWTHTHPTHLDIFRAAISMVQRFQGEVAVKLGHEVSVLPEGMLSAEYVNCARSTGFLPWFHED

SEQ ID NO: 21, Zugangsnummer: YP_002944432

MESAIAEHLKCPRTRHRRVEDDYAPPYPAWSARAPGAVRQVVMGYFGVQSRGAGMQGRACAALMKIAAGFALPDGPGHHDFAHHVDAAGCDNMVAIAYWNDPAAHARWCTAPEVDAWWRSDERLADGLGYFREIVAPRAEHFETMFNTPDRLEGVGVVMGGVSGELQEHGYWGSMRDRIPLSQTDAMAPSGTRAVIAGAPAPGQRVRIAGHENIAMIRSGQEWADTTGQERALYLGEMEPVLREGMDFLRDQGLHIGCYSNRYMQHLDAKGAPLEKSFGLSFWHSLADMERWAESHPTHVAIFGSFMRYVQALNFQLQLRVYHEVSVLKADEQSYEYINCHAGSGLMNGLGEV

SEQ ID NO: 22, Zugangsnummer: YP_001416464

MVFHVEYPRIVPERRPPGHEPAAPRFSLRWEQPVGLVVCAYFGLQGQDLAWDEQKAFFDRLQTSFGTDGPVAHEIMRMHDETGAVNAILVAYWLDATAHARWERNSPFMAWFRDPARLEGTRGVWRETMHVPYDRHETIYSTPSYVIGLARTPGATRVPITTNGYFGAMRDRMPVSAIDTLESPLGAMPPRRAPDSHGRRLTAAFPLNLISIRSGQYWEGAGNEQTADYIDNLQPKLMRGMAHLSSHPEQTGTLTLRIMTNLDAEGRPRAETSVHGYFLSMAHLEEWSRSHETHLDIYRHAIAMNRLYKEKREVFTWHEVFALLPGAHAEYANCHGGTGLLPYFADA

SEQ ID NO: 23; Zugangsnummer: Q2WG72 (NCBI, SwissProt)

MLRSRFPASHHFTVSVFGCQYHSEAPSVEKTELIGRFDKLIDSAAIHVEHLEQNDVPSKIWMSYWESPQKFKQWWEKDDTASFWASLPDDAGFWRETFSLPATRAMYEGTGKDAYGFGHCGSLIPLTTKTGYWGAYRSRMTPDFEGDTFSSPIPTYADQSVPADKIRPGRVRITDFPDNLCMVVEGQHYADMGEREREYWNENFDGLTKQWVTNVVTAGHEQGMVIARACHGFAGEKKLGATNGPVNGIFPGLDYVHQAQILIWQDISKMEHIGRYDQTHVKLRRDFMKAYGPGGEMEGGDLLLWVDLGILKKDEIDAEYVGCYESTGFLKLDKGQFFKVESTAGSKLPSFFDEPIESKPIEW

SEQ ID NO: 24, Zugangsnummer: Q7WSJ4 (NCBI, SwissProt)

MESAIDTHLKCPRTLSRRVPEEYQPPFPMWVARADEQLQQVVMGYLGVQYRGEAQRE AALQAMRHIVSSFSLPDGPQTHDLTHHTDSSGFDNLMVVGYWKDPAAHCRWLRSAEVNDWW TSQDRLGEGLGYFREISAPRAEQFETLYAFQDNLPGVGAVMDSTSGEIEEHGYWGSMRDRFPI SQTDWMKPTNELQVVAGDPAKGGRVVIMGHDNIALIRSGQDWADAEAEERSLYLDEILPTLQD GMDFLRDNGQPLGCYSNRFVRNIDLDGNFLDVSYNIGHWRSLEKLERWAESHPTHLRIFVTFF RVAAGLKKLRLYHEVSVSDAKSQVFEYINCHPHTGMLRDAVVAPT

SEQ ID NO: 25, Zugangsnummer: Q4W7T3 (NCBI, SwissProt)

MESAIDTHLKCPRTLSRRVPDEYQPPFAMWMARADEHLEQVVMAYFGVQYRGEAQRA AALQAMRHIVESFSLADGPQTHDLTHHTDNSGFDNLIVVGYWKDPAAHCRWLRSAPVNAWWA SEDRLNDGLGYFREISAPRAEQFETLYAFQDNLPGVGAVMDRISGEIEEHGYWGSMRDRFPIS

QTDWMKPTSELQVIAGDPAKGGRVVVLGHGNLTLIRSGQDWADAEAEERSLYLDEILPTLQDG MDFLRDNGQPLGCYSNRFVRNIDLDGNFLDVSYNIGHWRSVEKLERWTESHPTHLRIFVTFFR VAAGLKKLRLYHEVSVSDAKSQIFGYINCHPQTGMLRDAQVSPA

SEQ ID NO: 26, Zugangsnummer: Q76K71 (NCBI, SwissProt)

MESAIGEHLQCPRTLTRRVPDTYTPPFPMWVGRADDALQQVVMGYLGVQFRDEDQRP AALQAMRDIVAGFDLPDGPAHHDLTHHIDNQGYENLIVVGYWKDVSSQHRWSTSTPIASWWE SEDRLSDGLGFFREIVAPRAEQFETLYAFQEDLPGVGAVMDGISGEINEHGYWGSMRERFPIS QTDWMQASGELRVIAGDPAVGGRVVVRGHDNIALIRSGQDWADAEADERSLYLDEILPTLQSG MDFLRDNGPAVGCYSNRFVRNIDIDGNFLDLSYNIGHWASLDQLERWSESHPTHLRIFTTFFRV AAGLSKLRLYHEVSVFDAADQLYEYINCHPGTGMLRDAVTIAEH

SEQ ID NO: 27, Zugangsnummer: Q76EV4 (NCBI, SwissProt)

MESAIGEHLQCPRTLTRRVPDTYTPPFPMWVGRADDTLHQVVMGYLGVQFRGEDQRPA ALRAMRDIVAGFDLPDGPAHHDLTHHIDNQGYENLIVVGYWKDVSSQHRWSTSPPVSSWWES EDRLSDGLGFFREIVAPRAEQFETLYAFQDDLPGVGAVMDGVSGEINEHGYWGSMRERFPIS QTDWMQASGELRVVAGDPAVGGRVVVRGHDNIALIRSGQDWADAEADERSLYLDEILPTLQS GMDFLRDNGPAVGCYSNRFVRNIDIDGNFLDLSYNIGHWASLDQLERWSESHPTHLRIFTTFFR VAEGLSKLRLYHEVSVFDAADQLYEYINCHPGTGMLRDAVITAEH

SEQ ID NO: 28, Zugangsnummer: 049342 (NCBI, SwissProt)

MEMILSISLCLTTLITLLLLRRFLKRTATKVNLPPSPWRLPVIGNLHQLSLHPHRSLRSLSLR
YGPLMLLHFGRVPILVVSSGEAAQEVLKTHDHKFANRPRSKAVHGLMNGGRDVVFAPYGEYW
RQMKSVCILNLLTNKMVESFEKVREDEVNAMIEKLEKASSSSSSENLSELFITLPSDVTSRVALG
RKHSEDETARDLKKRVRQIMELLGEFPIGEYVPILAWIDGIRGFNNKIKEVSRGFSDLMDKVVQE
HLEASNDKADFVDILLSIEKDKNSGFQVQRNDIKFMILDMFIGGTSTTSTLLEWTMTELIRSPKSM
KKLQDEIRSTIRPHGSYIKEKEVENMKYLKAVIKEVLRLHPSLPMILPRLLSEDVKVKGYNIAAGT
EVIINAWAIQRDTAIWGPDAEEFKPERHLDSGLDYHGKNLNYIPFGSGRRICPGINLALGLAEVT
VANLVGRFDWRVEAGPNGDQPDLTEAIGIDVCRKFPLIAFPSSVV

**Patentansprüche**

1. Biokatalytisches Verfahren zur Herstellung von Nitrilen wobei man

   a) ein Oxim, ausgewählt unter Citraloxim, Neraloxim, Geranialoxim, Citronellaloxim und teil- oder vollhydrierten Analoga davon, wobei die Verbindung in stereoisomerenreiner Form oder als Gemisch von Stereoisomeren vorliegt,
   in Gegenwart einer aliphatischen Aldoxim Dehydratase (E.C. 4.99.1.5) oder einer Phenylacetaldoxim Dehydratase (PAOx) (EC 4.99.1.7) zum Nitril umsetzt, wobei das Nitril in stereoisomerenreiner Form oder als Gemisch von Stereoisomeren vorliegt, und
   b) gegebenenfalls das Reaktionsprodukt anschließend weiter reinigt.

2. Verfahren nach Anspruch 1, wobei die PAOx ein Enzym aus *Rhodococcus* sp., *Gibberella zeae* oder *Bacillus* sp. ist.

3. Verfahren nach einem der Ansprüche 1 und 2, wobei die PAOx eine Aminosäuresequenz gemäß SEQ ID NO: 1 oder eine dazu zu mindestens 60% identische Aminosäuresequenz aufweist.

4. Verfahren nach Anspruch 1, wobei man Citronellaloxim der Formel IIa

   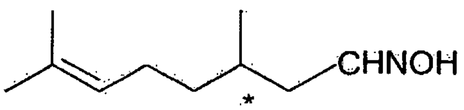 (IIa)

   zu Citronellylnitril der Formel Ia

   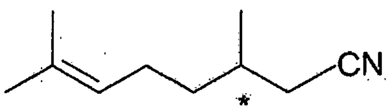 (Ia)

   umsetzt, wobei man die Verbindung der Formel IIa in stereoisomerenreiner Form oder als Stereoisomerengemisch einsetzt.

5. Verfahren nach einem der vorhergehenden Ansprüche, wobei die Umsetzung enzymatisch, in Gegenwart von PAOx,

eines PAOx enthaltenden Proteingemisches, oder in Gegenwart eines rekombinanten, PAOx funktional exprimierenden Mikroorganismus, eines davon abgeleiteten PAOx-haltigen Zellhomogenats oder einer PAOx-haltigen Fraktion davon erfolgt.

**6.** Verfahren nach einem der vorhergehenden Ansprüche, wobei PAOx oder der PAOx funktional exprimierende Mikroorganismus im Reaktionsgemisch frei oder in immobilisierter Form vorliegt.

**7.** Verfahren nach einem der Ansprüche 5 und 6, wobei der rekombinante Mikroorganismus ein PAOx funktional exprimierender Bakterienstamm ist.

**8.** Verfahren nach Anspruch 7, wobei der rekombinante Mikroorganismus ein Expressionskonstrukt trägt, das die kodierende PAOx Nukleinsäuresequenz, gegebenenfalls angepasst an die Codonnutzung des Mikroorganismus, aufweist.

**9.** Verfahren nach einem der Ansprüche 5 bis 8, wobei der Mikroorganismus zusätzlich wenigstens ein die funktionale Expression der PAOx unterstützendes Chaperon exprimiert.

**10.** Verfahren nach Anspruch 9, wobei der rekombinante Mikroorganismus ein Bakterienstamm ist, der zusätzlich zu PAOx ein oder mehrere Chaperone ausgewählt unter GroEL und GroES exprimiert.

**11.** Verfahren nach einem der vorhergehenden Ansprüche, wobei man die Umsetzung in Substrat als Reaktionsmedium durchführt.

## Claims

**1.** A biocatalytic process for producing nitriles wherein

a) an oxime, selected from citral oxime, neral oxime, geranial oxime, citronellal oxime and partially or fully hydrogenated analogs thereof, wherein the compound is in stereoisomerically pure form or as a mixture of stereoisomers,
is converted to the nitrile in the presence of an aliphatic aldoxime dehydratase (EC 4.99.1.5) or a phenylacetaldoxime dehydratase (PAOx) (EC 4.99.1.7), wherein the nitrile is in stereoisomerically pure form or as a mixture of stereoisomers, and
b) optionally the reaction product is then purified further.

**2.** The process according to claim 1, wherein the PAOx is an enzyme from *Rhodococcus* sp., *Gibberella zeae* or *Bacillus* sp.

**3.** The process according to any one of claims 1 and 2, wherein the PAOx has an amino acid sequence according to SEQ ID NO: 1 or an amino acid sequence identical to this to at least 60%.

**4.** The process according to claim 1, wherein citronellal oxime of formula IIa

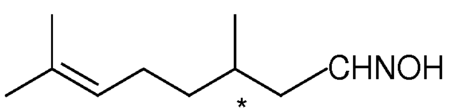 (IIa)

is converted to citronellyl nitrile of formula Ia

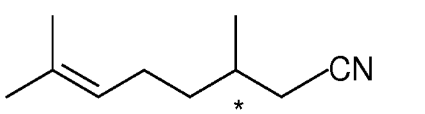 (Ia)

wherein the compound of formula IIa is used in stereoisomerically pure form or as stereoisomeric mixture.

5. The process according to any one of the preceding claims, wherein the reaction takes place enzymatically, in the presence of PAOx, a PAOx-containing protein mixture, or in the presence of a recombinant microorganism functionally expressing PAOx, a PAOx-containing cell homogenate derived therefrom or a PAOx-containing fraction thereof.

6. The process according to any one of the preceding claims, wherein PAOx or the microorganism functionally expressing PAOx is present in the reaction mixture in free or in immobilized form.

7. The process according to any one of claims 5 and 6, wherein the recombinant microorganism is a bacterial strain functionally expressing PAOx.

8. The process according to claim 7, wherein the recombinant microorganism carries an expression construct, which has the PAOx-coding nucleic acid sequence, optionally adapted to the codon usage of the microorganism.

9. The process according to any one of claims 5 to 8, wherein the microorganism additionally expresses at least one chaperone supporting the functional expression of the PAOx.

10. The process according to claim 9, wherein the recombinant microorganism is a bacterial strain, which in addition to PAOx expresses one or more chaperones selected from GroEL and GroES.

11. The process according to any one of the preceding claims, wherein the reaction is carried out in substrate as reaction medium.

## Revendications

1. Procédé biocatalytique pour la fabrication de nitriles, dans lequel

a) un oxime, choisi parmi le citraloxime, le néraloxime, le géranialoxime, le citronellaloxime et leurs analogues partiellement et entièrement hydrogénés, le composé se présentant sous forme stéréoisomériquement pure ou sous la forme d'un mélange de stéréoisomères,
est transformé en nitrile en présence d'une aldoxime déshydratase aliphatique (E.C. 4.99.1.5) ou d'une phénylacétaldoxime déshydratase (PAOx) (E.C. 4.99.1.7), le nitrile se présentant sous forme stéréoisomériquement pure ou sous la forme d'un mélange de stéréoisomères, puis
b) le produit de réaction est éventuellement davantage purifié.

2. Procédé selon la revendication 1, dans lequel la PAOx est une enzyme de *Rhodococcus sp., Gibberella zeae* ou *Bacillus sp.*

3. Procédé selon l'une quelconque des revendications 1 et 2, dans lequel la PAOx présente une séquence d'acides aminés selon SEQ ID NO : 1 ou une séquence d'acides aminés identique à au moins 60 % à celle-ci.

4. Procédé selon la revendication 1, dans lequel le citronellaloxime de formule IIa

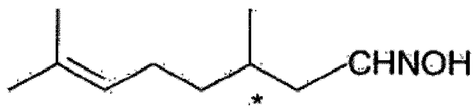

(IIa)

est transformé en citronellylnitrile de formule Ia

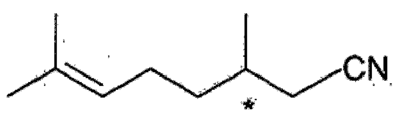

(Ia)

le composé de formule IIa étant utilisé sous forme stéréoisomériquement pure ou sous la forme d'un mélange de stéréoisomères.

**5.** Procédé selon l'une quelconque des revendications précédentes, dans lequel la réaction a lieu enzymatiquement, en présence de PAOx, d'un mélange de protéines contenant PAOx ou en présence d'un microorganisme recombinant exprimant fonctionnellement PAOx, d'un homogénat cellulaire contenant PAOx dérivé de celui-ci ou d'une fraction contenant PAOx de celui-ci.

**6.** Procédé selon l'une quelconque des revendications précédentes, dans lequel la PAOx ou le microorganisme exprimant fonctionnellement PAOx se présente sous forme libre ou immobilisée dans le mélange réactionnel.

**7.** Procédé selon l'une quelconque des revendications 5 et 6, dans lequel le microorganisme recombinant est une souche bactérienne exprimant fonctionnellement PAOx.

**8.** Procédé selon la revendication 7, dans lequel le microorganisme recombinant porte une construction d'expression, qui présence la séquence d'acides nucléiques PAOx codante, éventuellement adaptée à l'usage des codons du microorganisme.

**9.** Procédé selon l'une quelconque des revendications 5 à 8, dans lequel le microorganisme exprime en outre au moins un chaperon qui soutient l'expression fonctionnelle de la PAOx.

**10.** Procédé selon la revendication 9, dans lequel le microorganisme recombinant est une souche bactérienne, qui exprime en plus de PAOx un ou plusieurs chaperons choisis parmi GroEL et GroES.

**11.** Procédé selon l'une quelconque des revendications précédentes, dans lequel la réaction est réalisée dans un substrat en tant que milieu réactionnel.

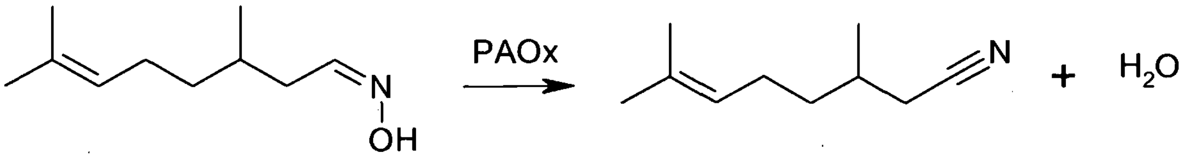
PAOx

Fig. 1

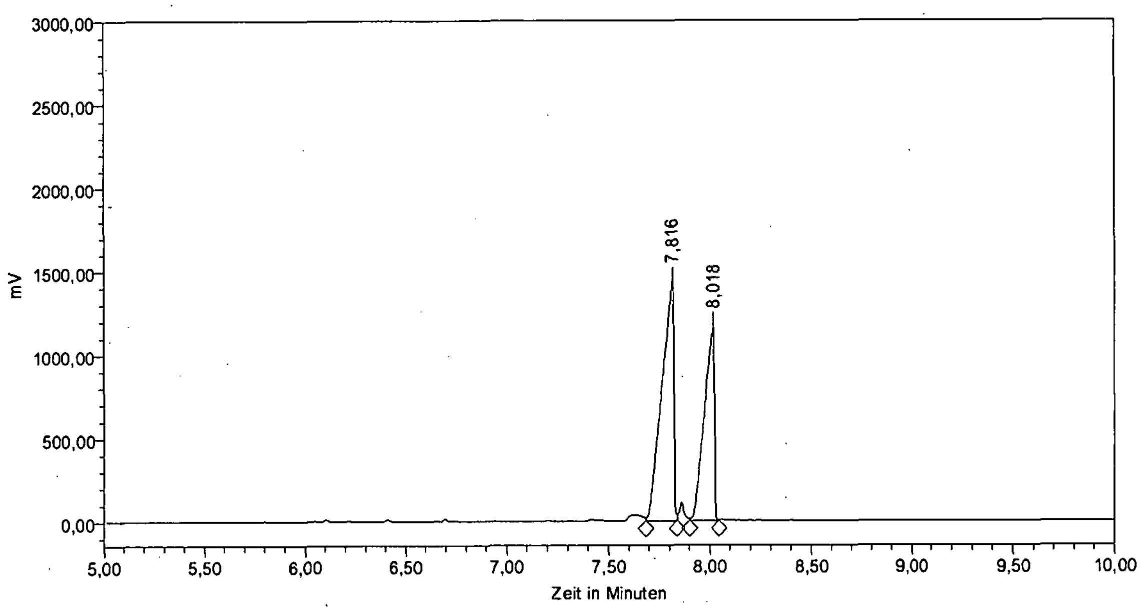
3000,00
2500,00
2000,00
1500,00
1000,00
500,00
0,00
mV
7,816
8,018
5,00
5,50
6,00
6,50
7,00
7,50
8,00
8,50
9,00
9,50
10,00
Zeit in Minuten

Fig. 2A

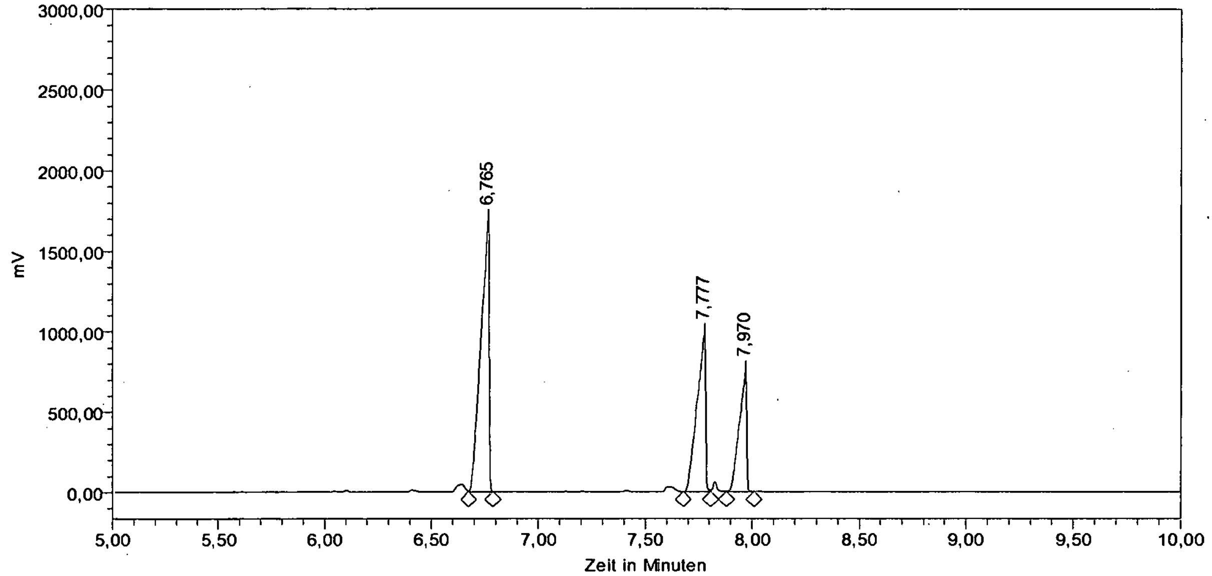
3000,00
2500,00
2000,00
1500,00
1000,00
500,00
0,00
mV
6,765
7,777
7,970
5,00
5,50
6,00
6,50
7,00
7,50
8,00
8,50
9,00
9,50
10,00
Zeit in Minuten

Fig. 2B

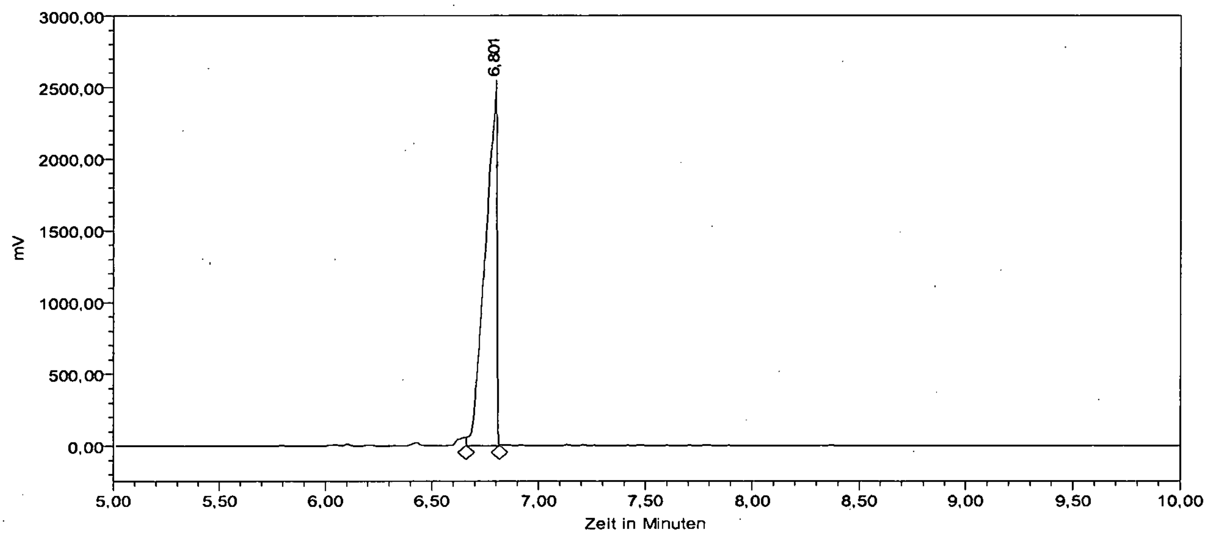
3000,00
2500,00
2000,00
1500,00
1000,00
500,00
0,00
mV
6,801
5,00
5,50
6,00
6,50
7,00
7,50
8,00
8,50
9,00
9,50
10,00
Zeit in Minuten

Fig. 2C

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006106141 A
- EP 0491127 A
- DE 3139358
- WO 2010044031 A1
- EP 1149849 A
- EP 1069183 A
- DE OS100193773 A
- EP 2010057696 W


### In der Beschreibung aufgeführte Nicht-Patentliteratur


- **KATO, Y. ; ASANO, Y.** *Bioscience, Biotechnology, and Biochemistry,* 2005, vol. 69 (11), 2254-2257
- **XIE, S.X. et al.** *Biochemistry,* 2003, vol. 42 (41), 12056-12066
- **PEDRAS M.S.C. et al.** *Phytochemistry,* 2010, vol. 71, 17-18, 1952-1962
- **KATO, Y. et al.** *Biochemistry,* 2000, vol. 39, 800-809
- **KATO et al.** *Biochemistry,* 2000, vol. 39, 800-809
- **PEARSON ; LIPMAN.** *Proc. Natl. Acad, Sci.,* 1988, vol. 85 (8), 2444-2448
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **SAMBROOK, J. ; FRITSCH, E.F. ; MANIATIS, T.** Molecular Cloning (A Laboratory Manual). Cold Spring Harbor Laboratory Press, 1989, 9.31-9.57
- Current Protocols in Molecular Biology. John Wiley & Sons, 1989
- **SAMBROOK ; RUSSELL.** Molecular Cloning. Cold Spring Harbor Laboratory Press, 2001
- **KEGLER-EBO DM ; DOCKTOR CM ; DIMAIO D.** *Nucleic Acids Res,* 1994, vol. 22, 1593
- **BARETTINO D ; FEIGENBUTZ M ; VALCÁREL R ; STUNNENBERG HG.** *Nucleic Acids Res,* 1994, vol. 22, 541
- **BARIK S.** *Mol Biotechnoi,* 1995, vol. 3, 1
- **ECKERT KA ; KUNKEL TA.** *Nucleic Acids Res,* 1990, vol. 18, 3739
- **SCHENK et al.** *Biospektrum,* 2006, vol. 3, 277-279
- **STEMMER WPC.** *Nature,* 1994, vol. 370, 389
- **STEMMER WPC.** *Proc Natl Acad Sci USA,* 1994, vol. 91, 10747
- **REETZ MT ; JAEGER K-E.** *Topics Curr Chem,* 1999, vol. 200 (31
- Methods for optimizing industrial enzymes by directed evolution. **ZHAO H ; MOORE JC ; VOLKOV AA ; ARNOLD FH.** Manual of industrial microbiology and biotechnology. American Society for Microbiology, 1999
- Goeddel, Gene Expression Technology: Methods in Enzymology. Academic Press, 1990, vol. 185
- Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984

- **AUSUBEL, F.M. et al.** Current Protocols in Molecular Biology. Greene Publishing Assoc. and Wiley Interscience, 1987
- Current Protocols in Molecular Biology. Wiley Interscience, 1997
- **SAMBROOK et al.** Molecular Cloning: A Laboratory Manual. Cold Spring Harbor Laboratory Press, 1989
- **CHMIEL.** Bioprozeßtechnik 1. Einführung in die Bioverfahrenstechnik. Gustav Fischer Verlag, 1991
- **STORHAS.** Bioreaktoren und periphere Einrichtungen. Vieweg Verlag, 1994
- Manual of Methods für General Bacteriology. American Society für Bacteriology, 1981
- Applied Microbiol. Physiology, A Practical Approach. IRL Press, 1997, 53-73
- **HARLOW, E. ; LANE, D.** Antibodies: A Laboratory Manual. Cold Spring Harbor (N.Y.) Press, 1988
- **J. LALONDE ; A. MARGOLIN.** *Immobilization of Enzymes*
- **K. DRAUZ ; H. WALDMANN.** Enzyme Catalysis in Organic Synthesis. Wiley-VCH, 2002, vol. III, 991-1032
- Biotechology. VCH, vol. 3
- **XIE, S.-X. et al.** *Biosci. Biotechnol. Biochem.,* 2001, vol. 65 (12), 2666-2672
- **SAMBROOK, J. ; FRITSCH, E.F. ; MANIATIS, T.** Molecular cloning: A Laboratory Manual. Cold Spring Harbor Laboratory Press, 1989